# EUROPEAN PATENT APPLICATION

(11) **EP 4 238 970 A1**
(43) Date of publication of application: **06.09.2023**
(21) Application number: 22159896.4
(22) Date of filing: 03.03.2022
(51) Int. Cl.: C07D 413/06, A61P 35/00

(54) **MODULATORS OF ACKR3 AND USES THEREOF**

(71) Applicant: InterAx Biotech AG, 5234 Villigen (CH); Stichting VU, 1081 HV Amsterdam (NL)
(72) Inventor: RIEMENS, Rick, 1081 HV Amsterdam (NL); BOSMA, Reggie, 1081 HV Amsterdam (NL); LEURS, Rob, 1081 HV Amsterdam (NL); DE ESCH, Iwan, 1081 HV Amsterdam (NL); WIJTMANS, Maikel, 1081 HV Amsterdam (NL); VISSCHER, Henry, 1081 HV Amsterdam (NL); NIKOLAEV, Yaroslav, 5234 Villigen (CH); KANEV, Georgi, 5234 Villigen (CH); ZIMMERMANN, Mirjam, 5234 Villigen (CH); RIZK, Aurélien, 5234 Villigen (CH); POTENZA, Alessandro, 5234 Villigen (CH); STEPNIEWSKI, Tomasz Maciej, 5234 Villigen (CH)
(74) Representative: Plasseraud IP

(57) **Abstract**

The present disclosure relates to compounds of formula (I) comprising a (4-piperidinylmethyl) carboxamide moiety, which can act as modulators of ACKR3. The present disclosure also relates to the use of these compounds as a drug.

## Description

### Technical field

The present disclosure relates to compounds of formula (I) comprising a (4-piperidinylmethyl) carboxamide moiety, which can act as modulators of ACKR3. The present disclosure also relates to the use of these compounds as a drug.

### Background art

Chemokines and G protein-coupled chemokine receptors (GPCRs) play an important role in the immune defense system by controlling the migration, activation, differentiation, and survival of leukocytes. Endogeneous chemokine proteins stabilize their cognant chemokine receptors in an active conformation that facilitates intracellular signal transduction by interactions with G proteins and/or arrestins. Because of their crucial role in the migration of immune cells, chemokine receptors are promising drug targets for various immune-related diseases, including chronic obstructive pulmonary disease, multiple sclerosis, rheumatoid arthritis, HIV-1 infection and cancer. Molecular pharmacology, medicinal chemistry, and molecular modeling studies have provided insights into molecular determinants of chemokine receptor modulation by proteins, peptides, and small-molecule ligands.

The chemokine receptor ACKR3, formerly called CXCR7, is activated by the chemokine peptides and majorly by chemokine CXCL12. The receptor is overexpressed in many cancer types indicating a vital role in the development of the disease. *In vitro* investigation of the ACKR3 receptor showed that overexpression and activation of the receptor by CXCL12 leads to oncogenic events such as angiogenesis, metastasis and trans endothelial cell-migration. ACKR3 knock-out models or ACKR3 inhibition by small-molecule ligands led to reduced oncogenic effects. Recently, the ACKR3 has been claimed to scavenge a broad range of opioid peptides, which might lead to a broadened application of a potential drug.

However, to date, no drug has been developed targeting the ACKR3 receptor. Thus, in this context, it is desirable to develop molecules that can act as modulators of ACKR3.

### Summary of the disclosure

The inventors surprisingly found that some compounds comprising a (4-piperidinylmethyl) carboxamide moiety can act as modulators of ACKR3.

Consequently, in a first aspect, the present disclosure relates to a compound formula (I): 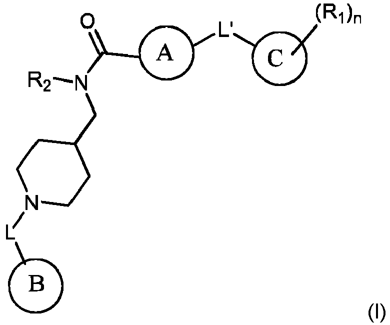 Wherein
Each R₁ is independently selected from halogen; n is an integer between 0 and 5, preferably between 1 and 5; Ring A is an heteroaryl having 5 ring atoms; L' is a bond or a divalent linker selected from the group consisting of C₁-C₆ alkylene, -O-, and C₁-C₆ heteroalkylene;
Ring C is a ring selected from the group consisting of C₃-C₁₂ cycloalkyl, heterocyclyl having 5 to 10 ring atoms, aryl having 6 to 10 ring atoms, and heteroaryl having 5 to 10 ring atoms; R₂ is selected from H, C₁-C₆ alkyl, C₃-C₆ cycloalkyl, heterocyclyl having 5 to 10 ring atoms, aryl having 6 to 10 ring atoms, and heteroaryl having 5 to 10 ring atoms; said alkyl, cycloalkyl, heterocyclyl, aryl and heteroaryl being optionally substituted with one or more substituents independently selected from oxo, halogen, -OH, -NO₂, -CN, and C₁-C₆ alkyl, preferably R2 is H;
L is a bond or a divalent linker selected from the group consisting of -(CR₃R₄)ₚ- p being an integer between 1 and 12, C₁-C₁₂ heteroalkylene, C₃-C₆ cycloalkylene, C₂-C₁₂ alkenylene, C₂-C₁₂ alkynylene, -O-, -NR'-, -S-, -SO-, and -SO2-,said heteroalkylene, alkenylene, and alkynylene being optionally interspersed with one or more groups selected from -(C₃-C₆ cycloalkyl)-, and said alkylene, heteroalkylene, cycloalkylene, alkenylene, and alkynylene being optionally substituted with one or more substituents independently selected from oxo, halogen, -OH, -NO₂, -CN, C₁-C₆ alkyl, C₃-C₆ cycloalkyl, heterocyclyl having 5 to 10 ring atoms, aryl having 6 to 10 ring atoms, heteroaryl having 5 to 10 ring atoms, C₁-C₆ alkoxy, C₁-C₆ haloalkyl, C₁-C₆ haloalkoxy, , -(CO)-R', -O-(CO)-R', -(CO)-OR', -(CO)-NR"R‴, -NR"-(CO)-R', and -NR"R‴;
Ring B is a ring selected from the group consisting of C₃-C₁₂ cycloalkyl, heterocyclyl having 5 to 10 ring atoms, aryl having 6 to 10 ring atoms, and heteroaryl having 5 to 10 ring atoms, said cycloalkyl, heterocyclyl, aryl and heteroaryl being optionally substituted with one or more substituents independently selected from oxo, halogen, -OH, -NO₂, -CN, C₁-C₆ alkyl, C₃-C₆ cycloalkyl, heterocyclyl having 5 to 10 ring atoms, aryl having 6 to 10 ring atoms, heteroaryl having 5 to 10 ring atoms, C₁-C₆ alkoxy, C₁-C₆ haloalkyl, C₁-C₆ haloalkoxy, -(CO)-R', -O-(CO)-R', -(CO)-O-R', -(CO)-NR"R‴, -NR"-(CO)-R', -NR"R‴, -S(O)₂-R', and S(O)₂-NR"R‴;
R₃ and R₄ are independently selected from H, C₁-C₆ alkyl, C₃-C₆ cycloalkyl, heterocyclyl having 5 to 10 ring atoms, aryl having 6 to 10 ring atoms, heteroaryl having 5 to 10 ring atoms; said alkyl, cycloalkyl, heterocyclyl, aryl and heteroaryl being optionally substituted with one or more substituents independently selected from oxo, halogen, -OH, -NO₂, -CN, and C₁-C₆ alkyl;
Or R₃ and R₄, together with the C atom to which they are bonded, form a C₃-C₁₂ cycloalkyl or a heterocyclyl having 5 to 10 ring atoms;
R' is selected from H, C₁-C₆ alkyl, C₃-C₆ cycloalkyl, heterocyclyl having 5 to 10 ring atoms, aryl having 6 to 10 ring atoms, and heteroaryl having 5 to 10 ring atoms;
R" and R‴ are independently selected from H, C₁-C₆ alkyl, C₃-C₆ cycloalkyl, heterocyclyl having 5 to 10 ring atoms, aryl having 6 to 10 ring atoms, heteroaryl having 5 to 10 ring atoms; said alkyl, cycloalkyl, heterocyclyl, aryl and heteroaryl being optionally substituted with one or more substituents independently selected from oxo, halogen, -OH, -NO₂, -CN, and C₁-C₆ alkyl;
Or R" and R‴, together with the N atom to which they are linked, form a heterocycle having 5 to 10 ring atoms, or a heteroaryl having 5 to 10 ring atoms, said heterocyclyl, and heteroaryl being optionally substituted with one or more substituents independently selected from oxo, halogen, -OH, -NO₂, -CN, and C₁-C₆ alkyl;
and pharmaceutically acceptable salts, solvates and esters thereof.

In a second aspect, the disclosure relates to a pharmaceutical composition comprising a compound of the present disclosure and a pharmaceutically acceptable carrier.

In a third aspect, the disclosure relates to a compound of the present disclosure for use as a drug.

In a fourth aspect, the disclosure relates to a compound of the present disclosure for use in treating cancer, autoimmune disorders, inflammatory diseases, transplant rejection, fibrosis, or pain.

### Detailed description

### Definitions

As used herein, the terms "C₁-Cₓ alkyl", by itself or as part of another substituent, refer to a linear or branched alkyl functional group having 1 to 12 carbon atoms, more preferably 1 to 6 carbon atoms. Suitable alkyl groups include methyl, ethyl, n-propyl, *i*-propyl, n-butyl, *i*-butyl, s-butyl and t-butyl, pentyl and its isomers (e.g. n-pentyl, *iso*-pentyl), and hexyl and its isomers (e.g. n-hexyl, *iso*hexyl).

As used herein, the terms "C₃-C₁₂ cycloalkyl" refer to a saturated or unsaturated cyclic group having 3 to 12 carbon atoms, preferably 3 to 6. The cycloalkyl can have a single ring or multiple rings fused together. The cycloalkyl can also include spirocyclic rings. Suitable cycloalkyl groups include cyclopropyl, cyclobutyl, cyclopentyl and cyclohexyl.

As used herein, the term "halogen" refers to a fluoro (-F), chloro (-CI), bromo (-Br), or iodo (-I) group.

As used herein, the terms "C₁-C₆ haloalkyl" refer to a C₁-C₆ alkyl as defined herein that is substituted by one or more halogen group as defined herein. Suitable C₁-C₆ haloalkyl groups include trifluoromethyl and dichloromethyl.

As used herein, the terms "C₁-Cₓ heteroalkyl", refer to a straight or branched hydrocarbon chain consisting of 1 to 12 carbon atoms, more preferably 1 to 6 carbon atoms, and from one to three, heteroatoms selected from the group consisting of O, N, Si and S, and wherein the nitrogen and sulfur atoms may optionally be oxidized (for example: a sulfoxide or a sulfone) and the nitrogen heteroatom may optionally be quaternized. The heteroatom(s) O, N and S may be placed at any interior position of the heteroalkyl group or at the position at which the alkyl group is attached to the remainder of the molecule.

As used herein, the terms "C₁-C₆ alkoxy" refer to a -O-alkyl group, wherein the alkyl group is a C₁-C₆ alkyl as defined herein. Suitable C₁-C₆ alkoxy groups include methoxy, ethoxy, propoxy.

As used herein, the terms "C₁-C₆ haloalkoxy" refer to a C₁-C₆ alkoxy group as defined herein, that is substituted by one or more halogen group as defined herein. Suitable haloalkoxy include trifluoromethoxy.

As used herein, the terms "C₁-Cₓ alkylene", used alone or as part of another substituent, refer to a divalent saturated, straight-chained or branched hydrocarbon group having 1 to 12 carbon atoms, preferably 1 to 6.

As used herein, the terms "C₁-Cₓ heteroalkylene", refer to a divalent heteroalkyl as defined above. For heteroalkylene groups, heteroatoms can also occupy either or both of the chain termini.

As used herein, the terms "aryl having 6 to 10 ring atoms" refer to a polyunsaturated, aromatic hydrocarbyl group having a single ring or multiple aromatic rings fused together, containing 6 to 10 ring atoms, wherein at least one ring is aromatic. The aromatic ring may optionally include one to two additional rings (cycloalkyl, heterocyclyl or heteroaryl as defined herein) fused thereto. Suitable aryl groups include phenyl, naphtyl and phenyl ring fused to a heterocyclyl, like benzopyranyl, benzodioxolyl, benzodioxanyl and the like.

As used herein, the terms "heteroaryl having 5 to 10 ring atoms" refer to a polyunsaturated, aromatic ring system having a single ring or multiple aromatic rings fused together or linked covalently, containing 5 to 10 atoms, wherein at least one ring is aromatic and at least one ring atom is a heteroatom selected from N, O and S. The nitrogen and sulfur heteroatoms may optionally be oxidized and the nitrogen heteroatoms may optionally be quaternized. Such rings may be fused to an aryl, cycloalkyl or heterocyclyl ring. Non-limiting examples of such heteroaryl, include: furanyl, thiophenyl, pyrrolyl, pyrazolyl, imidazolyl, oxazolyl, isoxazolyl, thiazolyl, isothiazolyl, triazolyl, oxadiazolyl, thiadiazolyl, tetrazolyl, oxatriazolyl, thiatriazolyl, pyridinyl, pyrimidyl, pyrazinyl, pyridazinyl, oxazinyl, dioxinyl, thiazinyl, triazinyl, indolyl, isoindolyl, benzofuranyl, isobenzofuranyl, benzothiophenyl, isobenzothiophenyl, indazolyl, benzimidazolyl, benzoxazolyl, purinyl, benzothiadiazolyl, quinolinyl, isoquinolinyl, cinnolinyl, quinazolinyl and quinoxalinyl.

As used herein, the terms "heterocyclyl having 5 to 10 ring atoms" refer to a saturated or unsaturated cyclic group having 5 to 10 ring atoms, wherein at least one ring atom is a heteroatom selected from N, O and S. The nitrogen and sulfur heteroatoms may optionally be oxidized and the nitrogen heteroatoms may optionally be quaternized. The heterocycle can include fused or bridged rings as well as spirocyclic rings. Examples of heterocycle include, but are not limited to, tetrahydropyridyl, piperidinyl, morpholinyl, tetrahydrofuranyl, tetrahydrothienyl, piperazinyl, 1-azepanyl, imidazolinyl, 1,4-dioxanyl and the like.

As used herein, the terms "organyl group" refer to any organic substituent group, regardless of functional type, having one free valence at a carbon atom.

Various embodiments of the disclosure are described herein. It will be recognized that features specified in each embodiment may be combined with other specified features to provide further embodiments.

The present disclosure encompasses the compounds of the present disclosure, their tautomers, enantiomers, diastereomers, racemates or mixtures thereof, and their hydrates, esters, solvates or pharmaceutically acceptable salts.

The terms "pharmaceutically acceptable salts" refers to salts that retain the biological effectiveness and properties of the compounds of this disclosure and, which typically are not biologically or otherwise undesirable. In many cases, the compounds of the disclosure are capable of forming acid and/or base salts by virtue of the presence of amino and/or carboxyl groups or groups similar thereto. Pharmaceutically acceptable acid addition salts can be formed with organic acids and/or inorganic acids. Pharmaceutically acceptable base addition salts can be formed with organic bases and/or inorganic bases.

In many cases, the compounds of the disclosure are capable of forming esters by virtue of the presence of carboxyl groups. Esters include C₁-C₆ alkyl esters.

Any formula given herein is also intended to represent unlabeled as well as isotopically forms of the compounds, like deuterium labeled compounds or ¹⁴C-labeled compounds.

### Compound of formula I

The present disclosure first relates to a compound of formula (I): 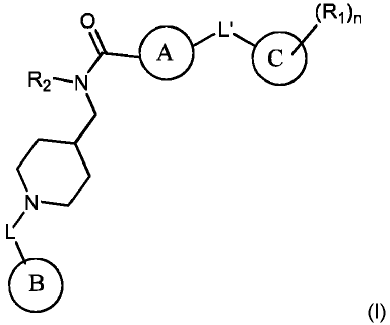 Wherein
Each R₁ is independently selected from halogen;
n is an integer between 0 and 5, preferably between 1 and 5;
Ring A is an heteroaryl having 5 ring atoms;
L' is a bond or a divalent linker selected from the group consisting of C₁-C₆ alkylene, -O-, and C₁-C₆ heteroalkylene;
Ring C is a ring selected from the group consisting of C₃-C₁₂ cycloalkyl, heterocyclyl having 5 to 10 ring atoms, aryl having 6 to 10 ring atoms, and heteroaryl having 5 to 10 ring atoms;
R₂ is selected from H, C₁-C₆ alkyl, C₃-C₆ cycloalkyl, heterocyclyl having 5 to 10 ring atoms, aryl having 6 to 10 ring atoms, and heteroaryl having 5 to 10 ring atoms; said alkyl, cycloalkyl, heterocyclyl, aryl and heteroaryl being optionally substituted with one or more substituents independently selected from oxo, halogen, -OH, -NO₂, -CN, and C₁-C₆ alkyl, preferably R2 is H;
L is a bond or a divalent linker selected from the group consisting of -(CR₃R₄)ₚ- p being an integer between 1 and 12, C₁-C₁₂ heteroalkylene, C₃-C₆ cycloalkylene, C₂-C₁₂ alkenylene, C₂-C₁₂ alkynylene, -O-, -NR'-, -S-, -SO-, and -SO2-, said heteroalkylene, alkenylene, and alkynylene being optionally interspersed with one or more groups selected from -(C₃-C₆ cycloalkyl)-, and said alkylene, heteroalkylene, cycloalkylene, alkenylene, and alkynylene being optionally substituted with one or more substituents independently selected from oxo, halogen, -OH, -NO₂, -CN, C₁-C₆ alkyl, C₃-C₆ cycloalkyl, heterocyclyl having 5 to 10 ring atoms, aryl having 6 to 10 ring atoms, heteroaryl having 5 to 10 ring atoms, C₁-C₆ alkoxy, C₁-C₆ haloalkyl, C₁-C₆ haloalkoxy, -(CO)-R', -O-(CO)-R', -(CO)-OR', -(CO)-NR"R"', -NR"-(CO)-R', and -NR"R"';
Ring B is a ring selected from the group consisting of C₃-C₁₂ cycloalkyl, heterocyclyl having 5 to 10 ring atoms, aryl having 6 to 10 ring atoms, and heteroaryl having 5 to 10 ring atoms, said cycloalkyl, heterocyclyl, aryl and heteroaryl being optionally substituted with one or more substituents independently selected from oxo, halogen, -OH, -NO₂, -CN, C₁-C₆ alkyl, C₃-C₆ cycloalkyl, heterocyclyl having 5 to 10 ring atoms, aryl having 6 to 10 ring atoms, heteroaryl having 5 to 10 ring atoms, C₁-C₆ alkoxy, C₁-C₆ haloalkyl, C₁-C₆ haloalkoxy, -(CO)-R', -O-(CO)-R', -(CO)-O-R', -(CO)-NR"R"', -NR"-(CO)-R', -NR"R"', -S(O)₂-R', and S(O)₂-NR"R"';
R₃ and R₄ are independently selected from H, C₁-C₆ alkyl, C₃-C₆ cycloalkyl, heterocyclyl having 5 to 10 ring atoms, aryl having 6 to 10 ring atoms, heteroaryl having 5 to 10 ring atoms; said alkyl, cycloalkyl, heterocyclyl, aryl and heteroaryl being optionally substituted with one or more substituents independently selected from oxo, halogen, -OH, -NO₂, -CN, and C₁-C₆ alkyl;
Or R₃ and R₄, together with the C atom to which they are bonded, form a C₃-C₁₂ cycloalkyl or a heterocyclyl having 5 to 10 ring atoms;
R' is selected from H, C₁-C₆ alkyl, C₃-C₆ cycloalkyl, heterocyclyl having 5 to 10 ring atoms, aryl having 6 to 10 ring atoms, and heteroaryl having 5 to 10 ring atoms;
R" and R‴ are independently selected from H, C₁-C₆ alkyl, C₃-C₆ cycloalkyl, heterocyclyl having 5 to 10 ring atoms, aryl having 6 to 10 ring atoms, heteroaryl having 5 to 10 ring atoms; said alkyl, cycloalkyl, heterocyclyl, aryl and heteroaryl being optionally substituted with one or more substituents independently selected from oxo, halogen, -OH, -NO₂, -CN, and C₁-C₆ alkyl;
Or R" and R‴, together with the N atom to which they are linked, form a heterocycle having 5 to 10 ring atoms, or a heteroaryl having 5 to 10 ring atoms, said heterocyclyl, and heteroaryl being optionally substituted with one or more substituents independently selected from oxo, halogen, -OH, -NO₂, -CN, and C₁-C₆ alkyl;
and pharmaceutically acceptable salts, solvates and esters thereof.

According to a preferred embodiment, the compound of formula (I) is a modulator of ACKR3. As used herein, the term "ACKR3 modulator" refers to a compound which directly binds to ACKR3, and which competitively inhibits the binding of reference ACKR3 binding compound. For example, an ACKR3 modulator of the present disclosure is a compound which directly binds to ACKR3 and is capable of displacing the binding of a fluorescently labelled chemokine CXCL12, as measured in vitro, typically as measured with the displacement assay using a chemokine CXCL12 fluorescently labelled with the dye AF647^{®} (Alexa Fluor 647). The dye Alexa Fluor 647 is a far-red-fluorescent cyanine dye. More details of such displacement assay are described in the Examples. In a more specific embodiment, said ACKR3 modulator of the present disclosure has preferably a pKᵢ greater than or equal to 5.0, typically comprised between 5.0 and 9.0, and for example comprised between 5.0 and 8.4, as measured by the displacement assay with a fluorescently labelled chemokine CXCL12. More preferably, said ACKR3 modulator of the present disclosure has a pKᵢ greater than or equal to 5.0, or at least 6.0, or at least 7.0.

In a more specific embodiment, said ACKR3 modulator of the present disclosure has preferably a pKᵢ greater than or equal to 5.0, typically comprised between 5.0 and 9.0, and for example comprised between 6.0 and 8.5, as measured by the displacement assay with labelled ACKR3 binding reference compound:a fluorescently labelled chemokine CXCL12. More preferably, said ACKR3 modulator of the present disclosure has a pKᵢ greater than or equal to 5.0, or at least 6.0, or at least 7.0.

According to an embodiment, ring A is selected from the group consisting of 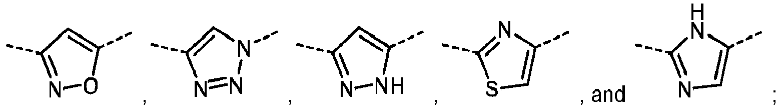 preferably ring A is

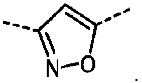

According to an embodiment, L is a divalent linker selected from C₃-C₁₂ alkylene, C₃-C₆ cycloalkylene, C₃-C₁₂ alkenylene, and C₃-C₁₂ alkynylene.

According to an embodiment, L' is a bond or a divalent linker selected from the group consisting of C₁-C₆ alkylene.

According to an embodiment, ring B is an aryl having 6 to 10 ring atoms, said aryl being optionally substituted with one or more substituents independently selected from halogen, -OH, C₁-C₆ alkyl, C₁-C₆ alkoxy, -(CO)-O-R', -NR"-(CO)-R', and -NR"R"'.

According to an embodiment, the compound of formula (I) is a compound of formula (II)

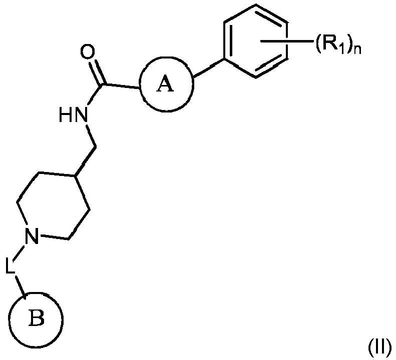

According to an embodiment, the compound of formula (I) is a compound of formula (III) 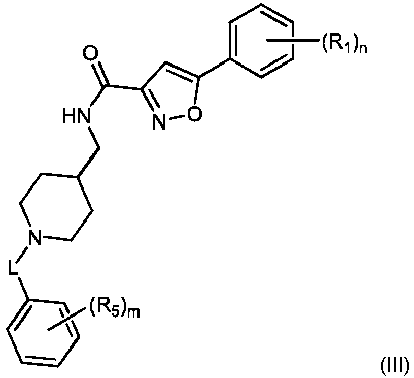 Wherein
Each R₁ is independently selected from halogen, provided that at least one R₁ is -F;
n is an integer between 1 and 5,
m is an integer from 0 to 5; and
each R5 is selected from halogen, -OH, -NO₂, -CN, C₁-C₆ alkyl, C₃-C₆ cycloalkyl, heterocyclyl having 5 to 10 ring atoms, aryl having 6 to 10 ring atoms, heteroaryl having 5 to 10 ring atoms, C₁-C₆ alkoxy, C₁-C₆ haloalkyl, C₁-C₆ haloalkoxy, -(CO)-R', -O-(CO)-R', -(CO)-O-R', -(CO)-NR"R"', -NR"-(CO)-R', - NR"R"', -S(O)₂-R', and S(O)₂-NR"R‴.

According to an embodiment, the compound of formula (I) is a compound of formula (IV) 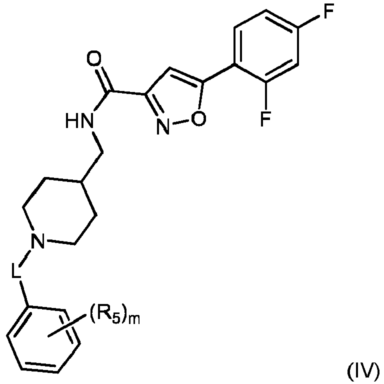 Wherein
m is an integer from 0 to 3; and
each R5 is selected from halogen, -OH, -NO₂, -CN, C₁-C₆ alkyl, C₃-C₆ cycloalkyl, heterocyclyl having 5 to 10 ring atoms, aryl having 6 to 10 ring atoms, heteroaryl having 5 to 10 ring atoms, C₁-C₆ alkoxy, C₁-C₆ haloalkyl, C₁-C₆ haloalkoxy, -(CO)-R', -O-(CO)-R', -(CO)-O-R', -(CO)-NR"R‴, -NR"-(CO)-R', - NR"R"', -S(O)₂-R', and S(O)₂-NR"R‴.

According to an embodiment, the compound of formula (I) is a compound of formula (V) 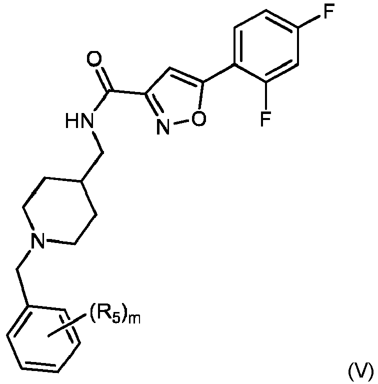 Wherein
m is an integer from 0 to 3; and
each R5 is selected from oxo, halogen, -OH, -NO₂, -CN, C₁-C₆ alkyl, C₃-C₆ cycloalkyl, heterocyclyl having 5 to 10 ring atoms, aryl having 6 to 10 ring atoms, heteroaryl having 5 to 10 ring atoms, C₁-C₆ alkoxy, C₁-C₆ haloalkyl, C₁-C₆ haloalkoxy, -(CO)-R', -O-(CO)-R', -(CO)-O-R', -(CO)-NR"R"', -NR"-(CO)-R', -NR"R"', -S(O)₂-R', and S(O)₂-NR"R‴, preferably from halogen, -OH, -NO₂, -CN, C₁-C₆ alkyl, C₃-C₆ cycloalkyl, heterocyclyl having 5 to 10 ring atoms, aryl having 6 to 10 ring atoms, heteroaryl having 5 to 10 ring atoms, C₁-C₆ alkoxy, C₁-C₆ haloalkyl, C₁-C₆ haloalkoxy, -(CO)-R', -O-(CO)-R', -(CO)-O-R', -(CO)-NR"R'", -NR"-(CO)-R', -NR"R"', -S(O)₂-R', and S(O)₂-NR"R‴.

According to an embodiment, the compound of formula (I) is selected from 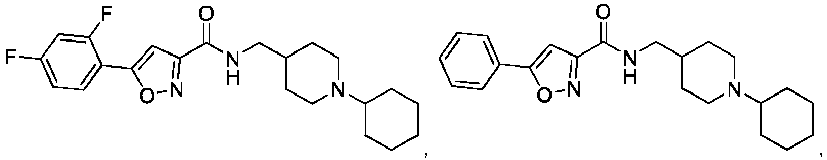 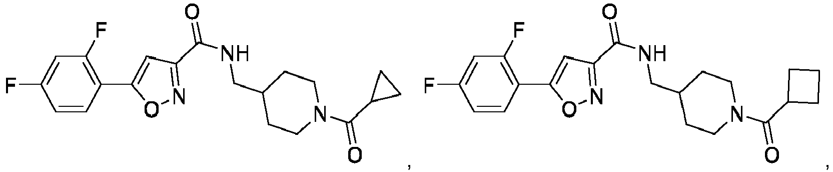 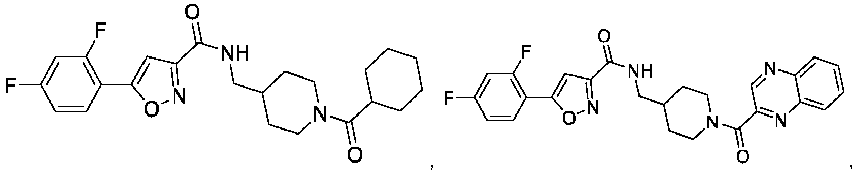 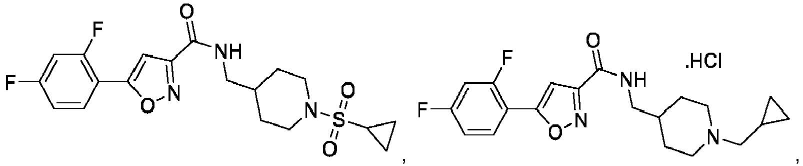 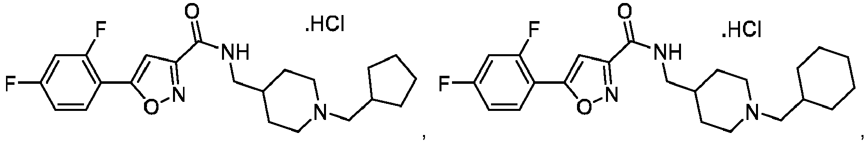 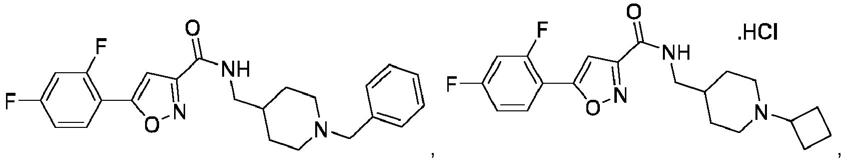 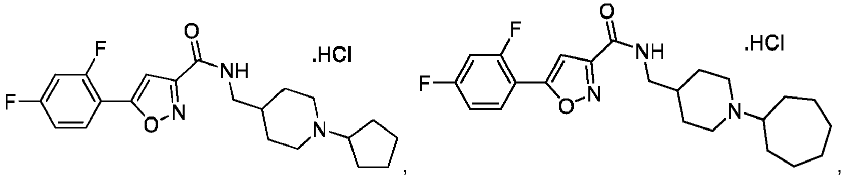 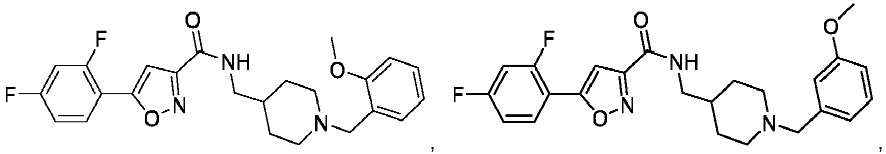 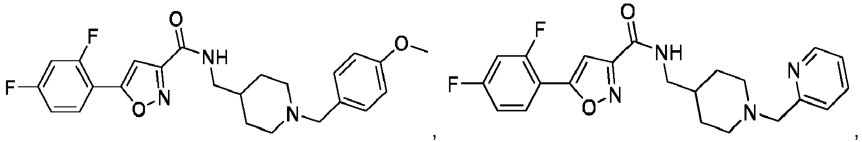 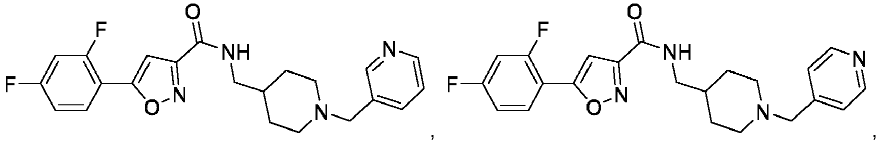 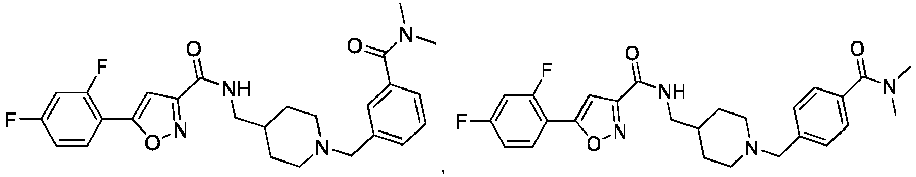 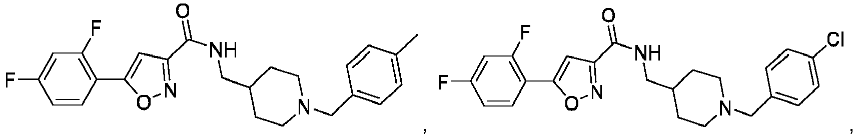 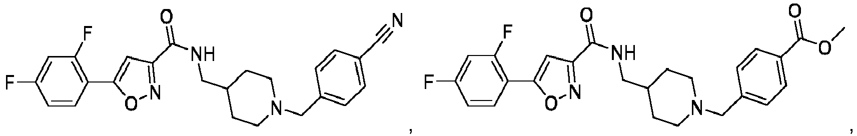 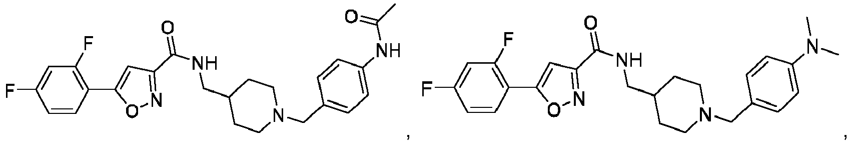 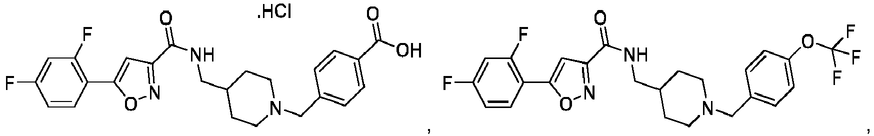 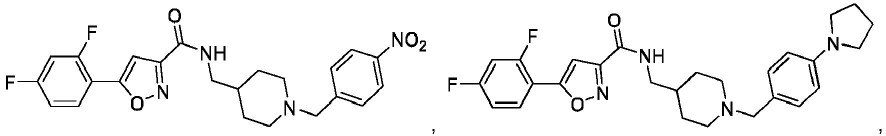 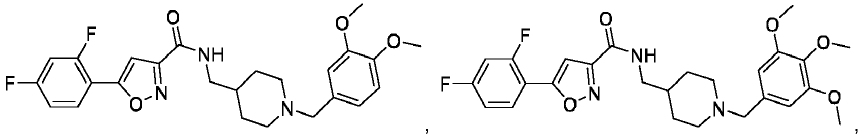 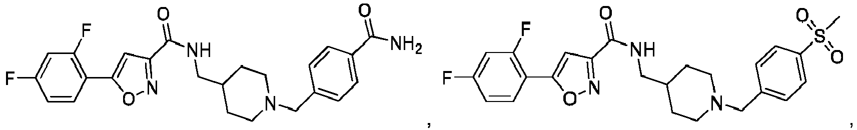 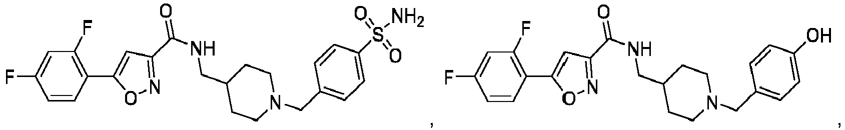 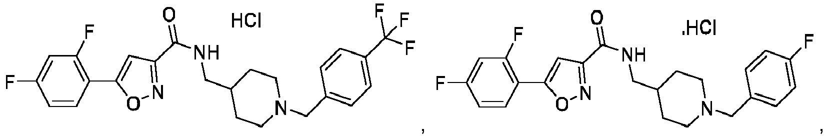 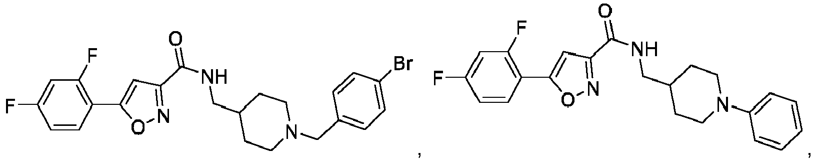 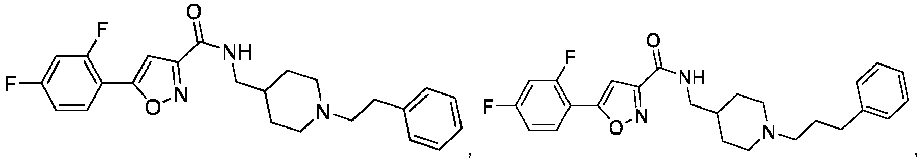 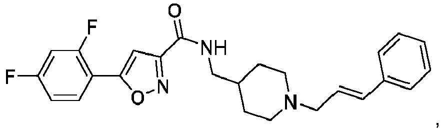 and 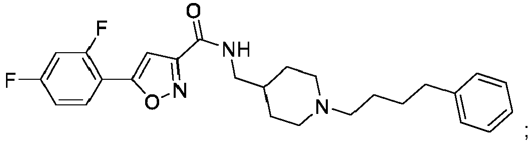 preferably, the compound is selected from 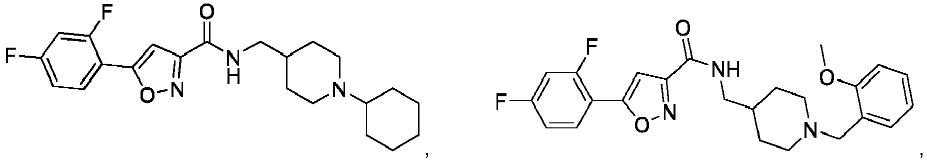 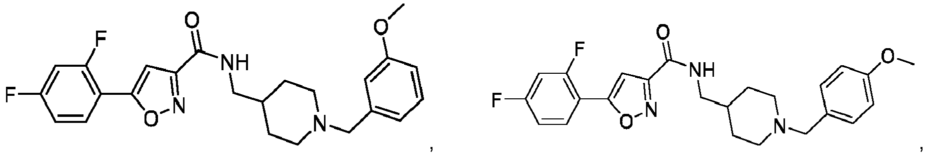 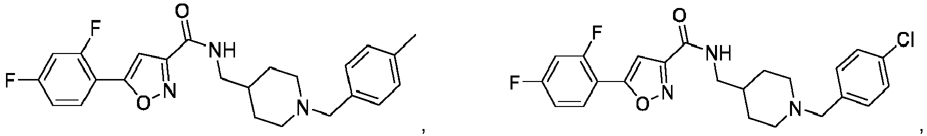 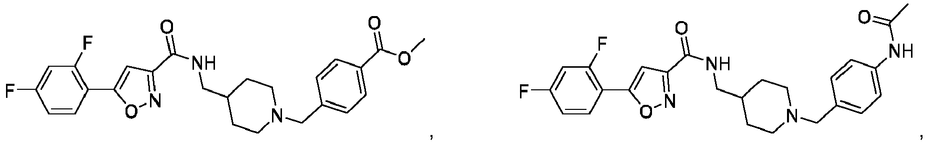 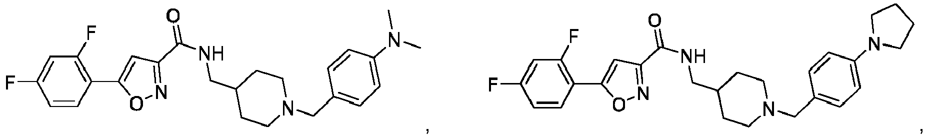 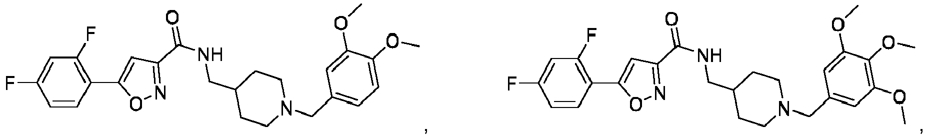 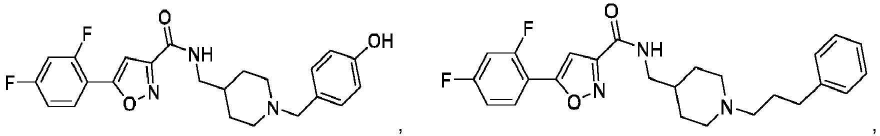 and

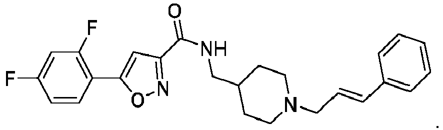

### Pharmaceutical composition

The disclosure also relates to a pharmaceutical composition comprising a compound of the disclosure and at least one pharmaceutically acceptable carrier.

The form of the pharmaceutical compositions, the route of administration, the dosage and the regimen naturally depend upon the condition to be treated, the severity of the illness, the age, weight, and sex of the patient, etc.

The pharmaceutical compositions of the disclosure can be formulated for a topical, oral, intranasal, intraocular, intravenous, intramuscular or subcutaneous administration and the like.

The pharmaceutical compositions can take the form of tablets, pills, capsules, semisolids, powders, sustained release formulations, solutions, suspensions, emulsions, syrups, elixirs, aerosols, or any other appropriate compositions; and comprise at least one compound according this disclosure.

Because of their ease in administration, tablets and capsules represent the most advantageous oral dosage unit form, in which case solid pharmaceutical carriers are obviously employed. If desired, tablets may be sugar coated or enteric coated by standard techniques. The tablets or pills can be coated to provide a dosage form affording the advantage of prolonged action. For example, the tablet or pills can comprise an inner dosage and an outer dosage component, the latter being in the form of an envelope over the former. The two components can be separated by an enteric layer, which serves to resist disintegration in the stomach and permits the inner component to pass intact into the duodenum or to be delayed in release. A variety of material can be used for such enteric layers or coatings, such materials including a number of polymeric acids with such materials as shellac, cetyl alcohol and cellulose acetate.

The compound of the disclosure and the further agent may also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

According to an embodiment, the pharmaceutical compositions contain vehicles which are pharmaceutically acceptable for oral formulation.

According to an embodiment, the pharmaceutical compositions contain vehicles which are pharmaceutically acceptable for a formulation capable of being injected or infused. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions or solutions for infusion.

The pharmaceutical forms suitable for injection or infusion include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Solutions of the active compounds as free base or pharmacologically acceptable salts can be prepared in water suitably mixed with a surfactant, such as hydroxypropylcellulose. Dispersions can also be prepared in glycerol, liquid polyethylene glycols, mixtures thereof and in oils. Under ordinary conditions of storage and use, these preparations contain a preservative to prevent the growth of microorganisms.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions and solutions for infusion are prepared by incorporating the active compounds in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions or slutions for infusion, the preferred methods of preparation are vacuum-drying and freeze-drying techniques which yield a powder of the active ingredient plus any additional desired ingredient from a previously sterile-filtered solution thereof. Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution may be suitably buffered and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject.

For aerosol administration, the compound of the disclosure and the further agent are preferably supplied in finely divided from along with a surfactant and propellant. The surfactant must, of course, be nontoxic, and preferably soluble in the propellant. Representative of such agents are the esters or partial esters of fatty acids containing from 6 to 22 carbon atoms, such as caproic, octanoic lauric, palmitic, stearic, linoleic, linolenic, olesteric and oleic acids with an aliphatic polyhydric alcohol or its cyclic anhydride. Mixed esters, such as mixed or natural glycerides may be employed. A carrier can also be included, as desired, as with, e.g., lecithin for intranasal delivery. An example includes a solution in which each milliliter included 7.5 mg NaCl, 1.7 mg citric acid monohydrate, 3 mg disodium phosphate dihydrate and 0.2 mg benzalkonium chloride solution (50%) (Gozes et al., J Mol Neurosci. 19(1-2):167-70 (2002)).

Suitable compositions for topical application include aqueous solutions, suspensions, ointments, creams, gels or sprayable formulations, e.g. aerosol administration.

The doses used for the administration can be adapted as a function of various parameters, and in particular as a function of the mode of administration used, of the relevant pathology, or alternatively of the desired duration of treatment. It will be appreciated that appropriate dosages of the compounds, and compositions comprising the compounds, can vary from patient to patient. Determining the optimal dosage will generally involve the balancing of the level of therapeutic benefit against any risk or deleterious side effects of the treatments described herein. The selected dosage level will depend on a variety of factors including, but not limited to, the activity of the particular compound, the route of administration, the time of administration, the rate of excretion of the compound, the duration of the treatment, other drugs, compounds, and/or materials used in combination, and the age, sex, weight, condition, general health, and prior medical history of the patient. The amount of compound and route of administration will ultimately be at the discretion of the physician, although generally the dosage will be to achieve local concentrations at the site of action which achieve the desired effect without causing substantial harmful or deleterious sideeffects. For example, the dose used for the administration can be of about 1-1000 mg of the compound of the disclosure for a subject of about 50-70 kg.

### Method of use

The compounds of the disclosure exhibit valuable pharmaceutical properties as indicated in the in vitro tests provided in the examples and are therefore indicated for therapy. In particular, the compounds of the disclosure are useful in the prevention or treatment of disorders relating to the ACKR3 receptor.

The disclosure also relates to a compound of the disclosure for use as a medicament.

The disclosure also relates to a compound of the disclosure for use in the treatment of cancer, autoimmune disorders, inflammatory diseases, transplant rejection, fibrosis, or pain.

The disclosure also relates to a compound of the disclosure for use in the treatment of disorders relating to the ACKR3 receptor.

In addition, further diseases or disorders relating to the ACKR3 receptor or its ligands are diseases involving ACKR3 and / or CXCL12 and / or CXCL11 mediated metastasis, chemotaxis, cell adhesion, trans-endothelial migration, cell proliferation and/or survival.

In addition, further particular diseases or disorders relating to the ACKR3 receptor or its ligands are proliferative diabetic retinopathy; West Nile virus encephalitis; pulmonary vascular diseases, acute renal failure, ischemia including cerebral ischemia, acute coronary syndrome, injured central nervous system, hypertension, pulmonary hypertension, Shiga-toxin-associated heomolytic uremic syndrome, preeclampsia, vascular injury, HIV / AIDS, angiogenesis, and brain and neuronal dysfunctions (such as inflammatory components of Alzheimer's disease), stress-related disorders (such as anxiety, depression, and posttraumatic stress disorder), and diseases involving opioid receptors. In a sub-embodiment, such a further particular disease or disorder relating to the ACKR3 receptor or its ligands is especially pulmonary hypertension.

As used herein, the term "cancer" has its general meaning in the art and includes an abnormal state or condition characterized by rapidly proliferating cell growth. The term is meant to include all types of cancerous growths or oncogenic processes, metastatic tissues or malignantly transformed cells, tissues or organs, irrespective of histopathologic type or stage of invasiveness. The term cancer includes malignancies of the various organ systems, such as affecting skin, lung, breast, thyroid, lymphoid, gastrointestinal, and genito-urinary tract, as well as adenocarcinomas which include malignancies such as most colon cancers, renal-cell carcinoma, prostate cancer and/or testicular tumors, non-small cell carcinoma of the lung, cancer of the small intestine and cancer of the oesophages.

Examples of cancer include, but are not limited, to hematological malignancies such as B-cell lymphoid neoplasm, T-cell lymphoid neoplasm, non-hodgkin lymphoma (NHL), B-NHL, T-NHL, chronic lymphocytic leukemia (CLL), small lymphocytic lymphoma (SLL), mantle cell lymphoma (MCL), NK-cell lymphoid neoplasm, and myeloid cell lineage neoplasm. Examples of nonhematological cancers include, but are not limited to, skin cancer, colon cancer, breast cancer, lung cancer, brain cancer, prostate cancer, head and neck cancer, pancreatic cancer, bladder cancer, colorectal cancer, bone cancer, cervical cancer, liver cancer, oral cancer, esophageal cancer, thyroid cancer, kidney cancer, stomach cancer and testicular cancer.

Examples of autoimmune disorders include (inflammatory) demyelinating diseases; multiple sclerosis (MS); Guillain Barre syndrome; rheumatoid arthritis (RA); inflammatory bowel diseases (IBD, especially comprising Crohn's disease and ulcerative colitis); systemic lupus erythematosus (SLE); lupus nephritis; interstitial cystitis; celiac disease; autoimmune encephalomyelitis; osteoarthritis; and type I diabetes.

Examples of inflammatory diseases include chronic rhinosinusitis, asthma, chronic obstructive pulmonary disorder, atherosclerosis, myocarditis, and sarcoidosis.

Examples of transplant rejection include renal allograft rejection, cardiac allograft rejection, and graft-versus-host diseases brought about by hematopoietic stem cell transplantation.

Examples of fibrosis include liver fibrosis, liver cirrhosis, lung fibrosis, especially idiopathic pulmonary fibrosis.

The disclosure relates to a method for treating cancer, autoimmune disorders, inflammatory diseases, transplant rejection, fibrosis, or pain, said method comprising administering to a subject in need thereof, preferably a human, a therapeutically efficient amount of
(i) a compound of the disclosure, or
(ii) a pharmaceutical composition as described herein.

The disclosure relates to a method for treating disorders relating to the ACKR3 receptor said method comprising administering to a subject in need thereof, preferably a human, a therapeutically efficient amount of
(i) a compound of the disclosure, or
(ii) a pharmaceutical composition as described herein.

As used herein, the term "treating" includes reversing, alleviating, inhibiting the progression of, preventing or reducing the likelihood of the disease, disorder, or condition to which such term applies, or one or more symptoms or manifestations of such disease, disorder or condition. Preventing refers to causing a disease, disorder, condition, or symptom or manifestation of such, or worsening of the severity of such, not to occur. Accordingly, the presently disclosed compounds can be administered prophylactically to prevent or reduce the incidence or recurrence of the disease, disorder, or condition.

As used herein, the terms "therapeutically efficient amount" of a compound refer to an amount of the compound that will elicit the biological or medical response of a subject, for example, ameliorate the symptoms, alleviate conditions, slow or delay disease progression, or prevent a disease.

The disclosure also relates to the use of a compound of the disclosure, for the manufacture of a medicament for the treatment of cancer, autoimmune disorders, inflammatory diseases, transplant rejection, fibrosis, or pain.

The disclosure also relates to the use of a compound of the disclosure, for the manufacture of a medicament for the treatment of disorders relating to the ACKR3 receptor.

### Examples

### Experimentals

### Pharmacology

### Binding studies - membrane production

Membranes of HEK293T cells that transiently express the NanoLuc-hACKR3 were produced. 2 million HEK293T cells were seeded per 10 cm² dish and were transfected the next day using a DNA/PEI-mix containing 0.25 µg plasmid DNA encoding for the ACKR3, 4.75 µg pcDEF3 plasmid DNA and 30µg linear PEI. Two days after transfection, cells were collected in PBS and pelleted by centrifugation. Cell pellets were resuspended in ice-cold membrane buffer (15 mM Tris, 0.3 mM EDTA, 2 mM MgCl2, pH 7.4 at 4°C) and were dounce-homogenized on ice by plunging the pestle 10 times with 1500 rpm. Cell homogenates were subsequently subjected to two freeze-thaw cycles using liquid nitrogen. Next, the cell homogenates were centrifuged at 40,000 g and pellets were resuspended in Tris-sucrose buffer (20 mM Tris, 250 mM Sucrose, pH 7.4 at 4°C). Finally, the membrane samples were homogenized using a 23-gauge needle, snap-frozen with liquid nitrogen and stored until further experimentation at -80 °C.

### Binding studies - NanoBRET binding

Binding reactions were measured in triplicate on white low-volume 384-well plates and started by combining 0.3 nM fluorescently labelled CXCL12 (CXCL12 labelled with a AF647^{®} dye in a molar ratio of dye:peptide of 1:1, purchased from ALMAC, h-SDF-1α (AF647^{®}), cat. no. CAF-11), increasing concentrations of test compound (10⁻⁵ M - 10⁻¹¹ M) with 35 ng membranes (protein content) per well of HEK293T cells expressing the NanoLuc-ACKR3. All dilutions were prepared in HBSS supplemented with 0.2% BSA. After assembling the binding reaction, the plate was flashcentrifuged at 500 g. Binding reactions were then incubated for 1 hour after which NanoGlo substrate was added (310 times diluted from stock concentration) to a final volume of 13.5 µL. The reaction plate was again pulse-centrifuged to 500 g and the total light intensity was measured for 460 nm wavelength with 80 nm bandwidth and separately for wavelengths ≥610 nm using the PHERAstar-FSX (BMG Labtech, Ortenberg, Germany) with a dual emission filter. The ratio of light intensities (>610 nm over 460 nm) is a measure for the relative binding of CXCL12-A647 to the NanoLuc-ACKR3. The pharmacological data are presented in Appendix 1.

### Chemistry

### General

Unless mentioned otherwise, all reactions were performed under an N₂ atmosphere. All chemicals and solvents were obtained from commercial suppliers (primarily Sigma-Aldrich, Acros Organics, Fluorochem and Combi-Blocks) and used without purification. DCM, DMF, THF and Et₂O were dried by passing through a PureSolv solvent purification system. IUPAC names were adapted from ChemBioDraw Ultra 20.0 (PerkinElmer). Reactions were monitored by thin layer chromatography (Merck Silicagel 60 F254) by visualization under 254 nm lamp. Flash column chromatography was performed with SNAP KP-Sil 50 µm (Biotage) or GraceResolv (Büchi) cartridges on Isolera One with UV-Vis detection (Biotage). Nuclear magnetic resonance (NMR) spectra were determined with a Brücker Avance 500 Ultrashield or a Brücker Avance 600 Ultrashield plus spectrometer. Chemical shifts are reported in parts per million (ppm) against the reference compound using the signal of the residual non-deuterated solvent (e.g. CDCl₃ δ = 7.26 ppm (¹H), δ = 77.16 ppm (¹³C)). NMR spectra were processed using MestreNova 14.1.0 software. The peak multiplicities are defined as follows: s, singlet; d, doublet; t, triplet; q, quartet; dd, doublet of doublets; ddd, doublet of doublets of doublets; dt, doublet of triplets; dq, doublet of quartets; td, triplet of doublets; tt, triplet of triplets; br, broad signal; m, multiplet; app, apparent. Purity determination was performed with Liquid Chromatography using a Shimadzu LC-20AD liquid chromatography pump system with a Shimadzu SPDM20A photodiode array detector and MS detection with a Shimadzu LCMS-2010EV mass spectrometer operating in both positive and negative ionization mode. A Waters XBridge C18 column 5 µm 4.6×50 mm was used at 40°C. The mobile phase used was a mixture of A = Water + 0.1% HCO₂H and B = acetonitrile (MeCN) + 0.1% HCO₂H. The eluent program 'acidic mode' used is as follows: flow rate: 1.0 mL/min, start 95% A in a linear gradient to 10% A over 4.5 min, hold 1.5 min at 10% A, in 0.5 min in a linear gradient to 95% A, hold 1.5 min at 95% A, total runtime: 8.0 min. Compound purities were calculated as the percentage peak area of the analyzed compound by UV detection at 254 nm. Highresolution mass spectra (HRMS) were recorded on a Bruker micrOTOF mass spectrometer using ESI in positive ion mode (HRMS).

### List of abbriviations

- ACN: Acetonitrile
- DCM: Dichloromethane
- DIPEA: Diisopropyl ethyl amine
- DMF: Dimethylformamide
- DMSO: Dimethyl Sulfoxide
- FA: Formic acid
- HATU: (1-[Bis(dimethylamino)methylene]-1H-1,2,3-triazolo[4,5-b]pyridinium 3-oxide hexafluorophosphate
- NMR: Nuclear magnetic resonance
- NP: Normal phase
- RP: Reverse phase
- RT: Room temperature
- STAB-H: Sodium triacetoxyhydroborate
- TEA: Triethylamine
- THF: Tetrahydrofuran

### General procedure A

5-(2,4-difluorophenyl)-N-(piperidin-4-ylmethyl)isoxazole-3-carboxamide hydrochloric acid was suspended in DMF (0.2 M) with AcOH (1.0 eq.) and the respective carbonyl compound (1.4 eq.). After stirring at RT for 15 min, STAB-H (1.4 eq.) was added and the reaction mixture was stirred overnight at RT. In cases where starting material was present additional carbonyl compound and STAB-H were added. The reaction mixture was concentrated *in vacuo.* The resulting oil was mixed with an aqueous solution of NaOH (2M). The solids were collected by vacuum filtration. In case of the preparation of HCI salt, the solids were dissolved in Et₂O and precipitated with 4M HCI in dioxane. The precipitated HCI salt was collected by vacuum filtration.

### General procedure B

5-(2,4-difluorophenyl)-N-(piperidin-4-ylmethyl)isoxazole-3-carboxamide hydrochloride was suspended in DCM (0.3 M) and DIPEA (2.5 eq.) was added. The respective acyl chloride or sulfonyl chloride (1.5 eq.) was added and the resulting mixture was stirred overnight at RT. The reaction mixture was washed with an aqueous solution of NaOH (3M) and the aqueous layer was extracted with DCM (twice). The combined organic layers were dried over anhydrous Na₂SO₄, filtered and concentrated *in vacuo.* The resulting residue was purified using NP column chromatography.

### General procedure C

5-(2,4-difluorophenyl)-N-(piperidin-4-ylmethyl)isoxazole-3-carboxamide hydrochloride was suspended in DMF (0.3 M) and K₂CO₃ (2.0 eq.) was added. The respective benzyl halide (1.1 eq.) was added and the reaction mixture was stirred overnight at RT. The reaction mixture was poured into an ice-cold aqueous solution of NaOH (2M). Extracted with DCM was performed 3 times. The combined organic layers were dried over anhydrous Na₂SO₄, filtered and concentrated *in vacuo.* The resulting residue was purified using NP column chromatography.

### General procedure D

5-(2,4-difluorophenyl)-N-(piperidin-4-ylmethyl)isoxazole-3-carboxamide hydrochloride was suspended in THF (0.3 M) and TEA (1.0 eq.) was added (in some cases AcOH was added). The respective benzaldehyde (1.2 eq.) was added and after stirring for 10 min at RT, STAB-H (2.5 eq.) was added. The resulting mixture was stirred overnight at RT and, if necessary, additional benzaldehyde and STAB-H was added to complete the reaction. MeOH was added to terminate the reaction and the volatiles were removed by *in vacuo.* The resulting solids were mixed with an aqueous solution of NaOH (2M) and extracted with DCM (3 times). The combined organic layers were dried over anhydrous Na₂SO₄, filtered and concentrated *in vacuo.* The resulting residue was purified using NP column chromatography.

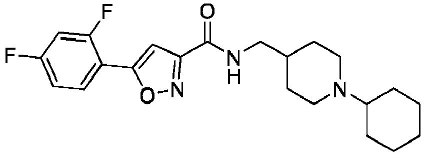

### N-[(1-cyclohexylpiperidin-4-yl)methyl]-5-(2,4-difluorophenyl)-1,2-oxazole-3-carboxamide HCI (1)

This compound was synthesised according to general procedure A. For the first step, 5-(2,4-difluorophenyl)-N-(piperidin-4-ylmethyl)isoxazole-3-carboxamide (200 mg, 0.623 mmol, 1.0 eq.), AcOH (36 µL, 0. 62 mmol, 1.0 eq), cyclohexanone (90 µL, 0.872 mmol, 1.4 eq.) and STAB-H (185 mg, 0. 872 mmol, 1.4 eq.) were used. The formed HCI salt was triturated with MeOH to obtain the title compound as an off-white solid (26 mg, 9%). **LCMS** (acidic mode) Rt 3.71 min, purity 97 %, [M+H]⁺ calculated 404.21, found 404.20. **¹H NMR** (500 MHz, Methanol-*d*₄) δ 8.0 (ddd, *J* = 8.6, 6.2 Hz, 1H), 7.2 (ddd, *J* = 11.3, 8.9, 2.5 Hz, 1H), 7.2 (ddd, *J* = 8.9, 2.5, 1.0 Hz, 1H), 7.1 (d, *J* = 3.5 Hz, 1H), 3.3 (d, *J* = 6.9 Hz, 2H), 3.0 - 2.9 (m, 2H), 2.3 - 2.2 (m, 3H), 1.9 - 1.9 (m, 2H), 1.9 - 1.8 (m, 4H), 1.7 - 1.6 (m, 2H), 1.4 - 1.2 (m, 6H), 1.2 - 1.1 (m, 1H). **¹³C NMR** (126 MHz, MeOD) δ 166.0, 165.8 (dd, *J* = 256.0, 12.0 Hz), 161.2, 161.0 (dd, J = 256.5, 12.0 Hz), 160.8, 130.3 (dd, *J* = 10.3, 3.4 Hz), 113.7 (dd, *J* = 22.4, 3.6 Hz), 113.1 (dd, *J* = 12.5, 3.9 Hz), 106.1 (dd, *J* = 26.0 Hz), 103.1 (d, *J* = 10.2 Hz), 65.4, 49.9, 45.9, 37.5, 30.9, 29.4, 27.4, 27.1. **HRMS** m/z [M+H]⁺ C₁₅H₁₆F₂N₃O₂⁺ calculated 404.2144, found 404.2151.

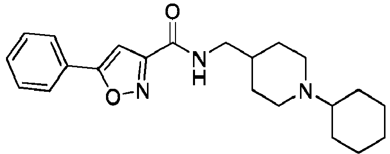

### N-[(1-cyclohexylpiperidin-4-yl)methyl]-5-phenyl-1,2-oxazole-3-carboxamide (2)

This compound was synthesised according to general procedure A. For the first step, 5-phenyl-N-(piperidin-4-ylmethyl)isoxazole-3-carboxamide (177mg, 0.623 mmol, 1.0 eq.), AcOH (36 µL, 0. 62 mmol, 1.0 eq), cyclohexanone (90 µL, 0.872 mmol, 1.4 eq.) and STAB-H (185 mg, 0. 872 mmol, 1.4 eq.) were used. The crude product was purified by NP column chromatography (using a linear gradient of 10% - 50% EtOAc:MeOH:TEA 90:5:5 in cyclohexane) to obtain the title compound as an off-white solid (150 mg, 66%). **LCMS** (acidic mode) Rt min 3.57, purity >99 %, [M+H]⁺ calculated 368.23, found 368.25. **¹H NMR** (500 MHz, Methanol-*d*₄) δ 7.89 - 7.86 (m, 2H), 7.55 - 7.50 (m, 3H), 7.08 (s, 1H), 3.29 (d, *J* = 6.7 Hz, 2H), 3.00 (dt, *J* = 12.2, 3.5 Hz, 2H), 2.41 - 2.31 (m, 3H), 1.97 - 1.90 (m, 2H), 1.85 - 1.78 (m, 4H), 1.72 - 1.62 (m, 2H), 1.39 - 1.23 (m, 6H), 1.19 - 1.08 (m, 1H). **¹³C NMR** (126 MHz, MeOD) δ 172.8, 161.5, 160.7, 131.9, 130.3, 128.2, 126.9, 99.9, 65.5, 49.9, 45.8, 37.4, 30.7, 29.3, 27.3, 27.1. **HRMS** m/z [M+H]⁺C₂₂H₃₀N₃O₂⁺calculated 368.2333, found 368.2348.

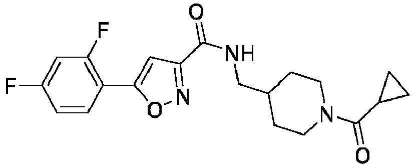

### N-((1-(cyclopropanecarbonyl)piperidin-4-yl)methyl)-5-(2,4-difluorophenyl)isoxazole-3-carboxamide (7)

This compound was synthesised according to general procedure B. 5-(2,4-difluorophenyl)-N-(piperidin-4-ylmethyl)isoxazole-3-carboxamide hydrochloride (200 mg, 0.559 mmol, 1.0 eq.), DIPEA (243 µL, 1.40 mmol, 2.5 eq) and cyclopropylcarbonyl chloride (76 µL, 0.84 mmol, 1.5 eq.) were used. The crude product was purified by NP column chromatography (using a linear gradient of 50% EtOAc/cyclohexane to 100 % EtOAc) to obtain the title compound as an off-white solid (167 mg, 77%). **LC/MS** (acidic mode) Rt 4.19 min, purity >99%, [M+H]⁺ calculated 390.16, found 390.20. **¹H NMR** (500 MHz, DMSO-d6) δ 8.96 (t, J = 6.0 Hz, 1H), 8.07 (ddd, J = 8.7, 8.7, 6.4 Hz, 1H), 7.59 (ddd, *J* = 11.6, 9.2, 2.6 Hz, 1H), 7.34 (ddd, *J* = 8.6, 8.6, 2.5 Hz, 1H), 7.17 (d, J = 3.0 Hz, 1H), 4.41 - 4.20 (m, 2H), 3.26 - 3.13 (m, 2H), 3.13 - 3.01 (m, 1H), 2.63 - 2.54 (m, 1H), 2.00 - 1.93 (m, 1H), 1.93 - 1.82 (m, 1H), 1.82 - 1.63 (m, 2H), 1.21 - 0.97 (m, 2H), 0.77 - 0.63 (m, 4H). ¹³C NMR (126 MHz, DMSO-d6) δ 170.7, 164.0 (d, J = 3.5 Hz), 163.7 (dd, J = 251.7, 13.3 Hz), 159.6, 159.0 (dd, J = 255.2, 12.8 Hz), 158.3, 129.6 (d, J = 10.4 Hz), 113.0 (dd, J = 22.1, 3.5 Hz), 111.5 (d, J = 12.4 Hz), 105.5 (dd, J = 26.0, 26.0 Hz), 102.5 (d, J = 8.3 Hz), 44.8, 44.2, 41.5, 35.9, 30.4, 29.4, 10.3, 6.8. *Rotamers are observed.* **HRMS** m/z [M+Na]⁺ C₂₀H₂₁F₂N₃NaO₃⁺ calculated 412.1443, found 412.1432.

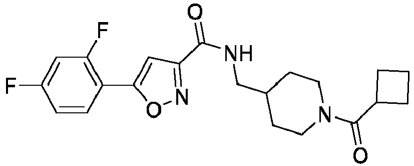

### N-((1-(cyclobutanecarbonyl)piperidin-4-yl)methyl)-5-(2,4-difluorophenyl)isoxazole-3-carboxamide (8)

This compound was synthesised according to general procedure B. 5-(2,4-difluorophenyl)-N-(piperidin-4-ylmethyl)isoxazole-3-carboxamide hydrochloride (200 mg, 0.559 mmol, 1.0 eq.), DIPEA (243 µL, 1.40 mmol, 2.5 eq) and cyclobutylcarbonyl chloride (96 µL, 0.84 mmol, 1.5 eq.) were used. The crude product was purified by NP column chromatography (using a linear gradient of 50% EtOAc/cyclohexane to 100 % EtOAc) to obtain the title compound as an off-white solid (124 mg, 55%). **LC/MS** (acidic mode) Rt 4.42 min, purity >99%, [M+H]⁺ calculated 404.18, found 404.20. **¹H NMR** (500 MHz, DMSO-d6) δ 8.92 (t, J = 6.0 Hz, 1H), 8.05 (ddd, J = 8.7, 8.7, 6.4 Hz, 1H), 7.57 (ddd, J = 11.5, 9.3, 2.6 Hz, 1H), 7.33 (ddd, *J* = 8.6, 8.6, 2.5 Hz, 1H), 7.15 (d, J = 2.9 Hz, 1H), 4.38 - 4.29 (m, 1H), 3.71 - 3.63 (m, 1H), 3.32 - 3.26 (m, 1H), 3.21 - 3.10 (m, 2H), 2.92 - 2.83 (m, 1 H), 2.55 - 2.46 (m, 1H), 2.19 - 2.01 (m, 4H), 1.93 -1.75 (m, 2H), 1.75 - 1.60 (m, 3H), 1.07 - 0.92 (m, 2H). ¹³C **NMR** (126 MHz, DMSO-d6) δ 171.7, 164.0 (d, J = 2.1 Hz), 163.7 (dd, J = 251.9, 12.4 Hz), 159.6, 159.0 (dd, J = 255.3, 12.7 Hz), 158.3, 129.6 (dd, *J* = 10.4, 3.2 Hz), 113.0 (dd, *J* = 21.9, 3.2 Hz), 111.5 (dd, J = 12.4, 3.8 Hz), 105.5 (dd, J = 26.0, 26.0 Hz), 102.5 (d, J = 8.4 Hz), 44.2 (d, J = 3.5 Hz), 41.0, 36.4, 35.8, 30.3, 29.4, 24.7, 24.5, 17.4. *Rotamers are observed.* **HRMS** m/z [M+Na]⁺ C₂₁H₂₃F₂N₃NaO₃⁺ calculated 426.1600, found 426.1600.

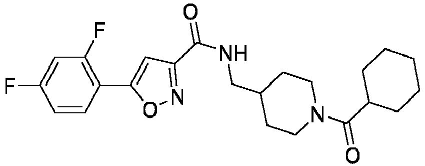

### N-((1-(cyclohexanecarbonyl)piperidin-4-yl)methyl)-5-(2,4-difluorophenyl)isoxazole-3-carboxamide (9)

This compound was synthesised according to general procedure B. 5-(2,4-difluorophenyl)-N-(piperidin-4-ylmethyl)isoxazole-3-carboxamide hydrochloride (200 mg, 0.559 mmol, 1.0 eq.), DIPEA (243 µL, 1.40 mmol, 2.5 eq) and isobutyryl chloride (112 µL, 0.838 mmol, 1.5 eq.) were used. The crude product was purified by NP column chromatography (using a linear gradient of 50% EtOAc/cyclohexane to 100 % EtOAc) to obtain the title compound as an off-white solid (124 mg, 55%). **LC/MS** (acidic mode) Rt 4.42 min, purity >99%, [M+H]⁺ calculated 404.18, found 404.20. **¹H NMR** (500 MHz, DMSO-d6) δ 8.93 (t, J = 6.0 Hz, 1H), 8.04 (ddd, J = 8.7, 8.6, 6.3 Hz, 1H), 7.59 - 7.53 (m, 1H), 7.32 (ddd, *J* = 8.6, 8.5, 2.5 Hz, 1H), 7.14 (d, J = 2.8 Hz, 1 H), 4.41 - 4.31 (m, 1H), 3.96 - 3.87 (m, 1H), 3.23 - 3.10 (m, 2H), 3.01 - 2.90 (m, 1H), 2.58 - 2.52 (m, 1H), 2.48 - 2.44 (m, 1H), 1.87 - 1.76 (m, 1H), 1.76 - 1.56 (m, 7H), 1.36 - 1.20 (m, 4H), 1.20 - 1.02 (m, 2H), 1.02 - 0.89 (m, 1H). **¹³C NMR** (126 MHz, DMSO-d6) δ 173.7, 164.5, 164.1 (dd, J = 252.0, 12.5 Hz), 160.0, 159.5 (dd, J = 255.3, 12.9 Hz), 158.8, 130.1 (dd, J = 10.4, 3.4 Hz), 113.5 (dd, J = 22.1, 3.5 Hz), 111.9 (dd, J = 12.5, 3.9 Hz), 105.9 (dd, J = 26.0, 26.0 Hz), 102.9 (d, J = 8.5 Hz), 45.1, 44.6, 41.3, 39.6, 36.3, 31.0, 29.8, 29.6, 26.1, 25.7. *Rotamers are observed.* **HRMS** m/z [M+Na]⁺ C₂₃H₂₇F₂N₃NaO₃⁺ calculated 454.1913, found 454.1922.

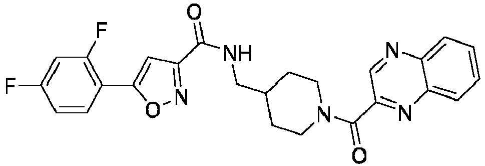

### 5-(2,4-difluorophenyl)-N-((1-(quinoxaline-2-carbonyl)piperidin-4-yl)methyl)isoxazole-3-carboxamide (12)

This compound was synthesised according to general procedure B. 5-(2,4-difluorophenyl)-N-(piperidin-4-ylmethyl)isoxazole-3-carboxamide hydrochloride (200 mg, 0.559 mmol, 1.0 eq.), DIPEA (243 µL, 1.40 mmol, 2.5 eq) and quinoxaline-2-carbonyl chloride (162 mg, 0.838 mmol, 1.5 eq.) were used. The crude product was purified by NP column chromatography (using a linear gradient of 50% EtOAc/cyclohexane to 100 % EtOAc) to obtain the title compound as an off-white solid (165 mg, 62%). **LC/MS** (acidic mode) Rt 4.42 min, purity >99%, [M+H]⁺ calculated 478.17, found 478.25. **¹H NMR** (500 MHz, DMSO-d6) δ 9.09 (s, 1H), 8.98 (t, J = 6.0 Hz, 1H), 8.17 - 8.13 (m, 1H), 8.13 - 8.08 (m, 1H), 8.05 (ddd, J = 8.7, 8.7, 6.3 Hz, 1H), 7.96 - 7.91 (m, 2H), 7.57 (ddd, J = 11.5, 9.2, 2.5 Hz, 1H), 7.32 (ddd, J = 8.5, 8.4, 2.5 Hz, 1H), 7.15 (d, J = 2.9 Hz, 1H), 4.59 - 4.52 (m, 1H), 3.85 - 3.79 (m, 1H), 3.27 - 3.20 (m, 2H), 3.19 - 3.10 (m, 1H), 2.95 - 2.87 (m, 1H), 1.98 - 1.89 (m, 1H), 1.89 - 1.82 (m, 1H), 1.70 - 1.63 (m, 1H), 1.35 - 1.19 (m, 2H). **¹³C NMR** (126 MHz, DMSO-d6) δ 164.7, 164.0 (d, J = 2.4 Hz), 163.3 (dd, J = 252.2, 12.7 Hz), 159.6, 159.0 (dd, J = 255.3, 12.7 Hz), 158.3, 149.2, 144.7, 141.6, 139.9, 131.2, 131.0, 129.6 (dd, J = 10.4, 3.3 Hz), 129.3, 129.0, 113.0 (dd, J = 22.1, 3.5 Hz), 111.5 (dd, J = 12.5, 3.9 Hz), 105.5 (dd, J = 25.9, 25.9 Hz), 102.5 (d, J = 8.5 Hz), 46.5, 44.1, 41.5, 35.6, 30.1, 29.3. *Rotamers are observed.* **HRMS** m/z [M+Na]⁺ C₂₃H₂₁F₂N₃NaO₃⁺ calculated 500.15050, found 500.1480.

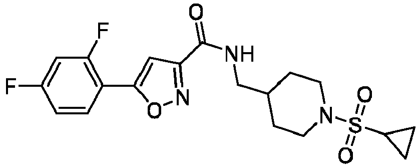

### N-((1-(cyclopropylsulfonyl)piperidin-4-yl)methyl)-5-(2,4-difluorophenyl)isoxazole-3-carboxamide (14)

This compound was synthesised according to general procedure B. 5-(2,4-difluorophenyl)-N-(piperidin-4-ylmethyl)isoxazole-3-carboxamide hydrochloride (200 mg, 0.559 mmol, 1.0 eq.), DIPEA (243 µL, 1.40 mmol, 2.5 eq) and cyclopropanesulfonyl chloride (85 µL, 0.84 mmol, 1.5 eq.) were used. The crude product was purified by NP column chromatography (using a linear gradient of 50% EtOAc/cyclohexane to 100 % EtOAc) to obtain the title compound as an off-white solid (200 mg, 84%). **LC/MS** (acidic mode) Rt 4.39 min, purity 99%, [M+H]⁺ calculated 426.13, found 426.20. ¹H **NMR** (600 MHz, DMSO-d6) δ 8.93 (t, J = 6.0 Hz, 1H), 8.05 (ddd, J = 8.6, 8.6, 6.2 Hz, 1H), 7.56 (ddd, J = 11.5, 9.1, 2.5 Hz, 1H), 7.32 (ddd, J = 8.6, 8.5, 2.6 Hz, 1H), 7.15 (d, J = 2.9 Hz, 1H), 3.64 - 3.55(m, 2H), 3.20 (dd, J = 6.3, 6.3 Hz, 2H), 2.83 - 2.74 (m, 2H), 2.58 - 2.52 (m, 1H), 1.81 - 1.68 (m, 3H), 1.26 - 1.15 (m, 2H), 1.00 - 0.84 (m, 4H). **¹³C NMR** (151 MHz, DMSO-d6) δ 164.0 (d, J = 3.1 Hz), 163.7 (dd, J = 252.1, 12.4 Hz), 159.6, 159.0 (dd, J = 255.3, 12.8 Hz), 158.3, 129.6 (dd, J = 10.3, 3.3 Hz), 113.0 (dd, J = 22.1, 3.4 Hz), 111.5 (dd, J = 12.4, 4.1 Hz), 105.5 (dd, J = 26.0, 26.0 Hz), 102.5 (d, J = 8.4 Hz), 45.6, 44.0, 34.9, 29.1, 25.2, 3.9. **HRMS** m/z [M+Na]⁺ C₁₉H₂₁F₂N₃NaO₄S⁺ calculated 448.1113, found 448.1094.

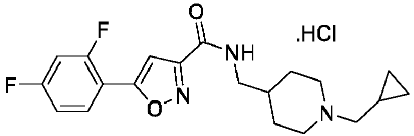

### N-((1-(cyclopropylmethyl)piperidin-4-yl)methyl)-5-(2,4-difluorophenyl)isoxazole-3-carboxamide hydrochloride (15)

This compound was synthesised according to general procedure A. 5-(2,4-difluorophenyl)-N-(piperidin-4-ylmethyl)isoxazole-3-carboxamide (200 mg, 0.623 mmol, 1.0 eq.), AcOH (36 µL, 0. 62 mmol, 1.0 eq), cyclopropanecarboxaldehyde (65 µL, 0.87 mmol, 1.4 eq.) and STAB-H (185 mg, 0. 872 mmol, 1.4 eq.) were used. The formed HCI salt was triturated with MeOH to obtain the title compound as an off-white solid (140 mg, 55%). **LC/MS** (acidic mode) Rt 3.44 min, purity >98%, [M+H]⁺ calculated 376.18, found 376.20. **¹H NMR** (500 MHz, DMSO-d6) δ 10.97 (s, 1H), 9.20 - 9.06 (m, 1H), 8.05 (ddd, J = 8.7, 8.7, 6.3 Hz, 1H), 7.58 (ddd, J = 11.6, 9.2, 2.6 Hz, 1H), 7.33 (ddd, J = 8.5, 8.5, 2.2 Hz, 1H), 7.25 - 7.19 (m, 1H), 3.54 - 3.44 (m, 2H), 3.17 (dd, J = 6.3, 6.3 Hz, 2H), 2.92 - 2.81 (m, 4H), 1.89 - 1.77 (m, 3H), 1.68 - 1.58 (m, 2H), 1.19 - 1.09 (m, 1H), 0.65 - 0.55 (m, 2H), 0.42 - 0.34 (m, 2H). **¹³C NMR** (126 MHz, DMSO-d6) δ 164.0 (d, J = 2.6 Hz), 163.6 (dd, J = 252.2, 12.2 Hz), 159.5, 159.0 (dd, J = 255.2, 12.7 Hz), 158.3, 129.6 (dd, J = 10.4, 3.3 Hz), 113.0 (dd, J = 21.9, 3.5 Hz), 111.5 (dd, J = 12.5, 3.8 Hz), 105.5 (dd, J = 26.0, 26.0 Hz), 102.5 (d, J = 8.2 Hz), 59.9, 50.8, 43.8, 33.7, 26.6, 5.2, 4.2. **HRMS** m/z [M+H]⁺ C₂₀H₂₃F₂N₃O₂⁺ calculated 376.1831, found 376.1827.

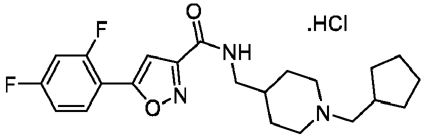

### N-((1-(cyclopentylmethyl)piperidin-4-yl)methyl)-5-(2,4-difluorophenyl)isoxazole-3-carboxamide (16)

This compound was synthesised according to general procedure A. 5-(2,4-difluorophenyl)-N-(piperidin-4-ylmethyl)isoxazole-3-carboxamide (200 mg, 0.623 mmol, 1.0 eq.), AcOH (36 µL, 0. 62 mmol, 1.0 eq), cyclopentanecarboxaldehyde (93 µL, 0.87 mmol, 1.4 eq.) and STAB-H (185 mg, 0. 872 mmol, 1.4 eq.) were used. The formed HCI salt was triturated with MeOH to obtain the title compound as an off-white solid (222 mg, 81%). **LC/MS** (acidic mode) Rt 3.82 min, purity >98%, [M+H]⁺ calculated 404.21, found 404.20. **¹H NMR** (500 MHz, DMSO-d6) δ 10.44 (d, J = 71.9 Hz, 1H), 9.26 - 9.00 (m, 1H), 8.06 (ddd, J = 8.7, 8.7, 6.4 Hz, 1H), 7.59 (ddd, J = 11.5, 9.2, 2.5 Hz, 1H), 7.38 - 7.31 (m, 1H), 7.24 (d, J = 2.9 Hz, 1H), 3.46 (s, 2H), 3.19 (dd, J = 6.4, 6.4 Hz, 2H), 3.00 (d, J = 7.0 Hz, 2H), 2.90 (d, J = 10.6 Hz, 2H), 2.30-2.26 (m, 1H), 2.00 - 1.77 (m, 5H), 1.77 - 1.64 (m, 2H), 1.64 - 1.55 (m, 2H), 1.55 - 1.43 (m, 2H), 1.33 - 1.20 (m, 2H). **¹³C NMR** (126 MHz, DMSO-d6) δ 164.1 (d, J = 2.7 Hz), 163.7 (dd, J = 252.0, 12.3 Hz), 159.6, 159.0 (dd, J = 255.3, 12.8 Hz), 158.4, 129.7 (dd, J = 10.4, 3.3 Hz), 113.1 (dd, J = 22.1, 3.5 Hz), 111.5 (dd, J = 12.3, 4.1 Hz), 105.5 (dd, J = 25.9, 25.9 Hz), 102.5 (d, J = 8.5 Hz), 61.1, 51.7, 43.8, 34.4, 34.0, 31.2, 26.6, 24.7. **HRMS** m/z [M+H]⁺ C₂₂H₂₈F₂N₃O₂⁺ calculated 404.2144, found 404.2136.

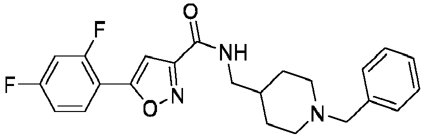

### N-((1-benzylpiperidin-4-yl)methyl)-5-(2,4-difluorophenyl)isoxazole-3-carboxamide (17)

5-(2,4-difluorophenyl)-N-(piperidin-4-ylmethyl)isoxazole-3-carboxamide (200 mg, 0.623 mmol, 1.0 eq.) was suspended in DMF (0.2 M) and AcOH (36 µL, 0. 62 mmol, 1.0 eq) and benzaldehyde (88 µL, 0.87 mmol, 1.4 eq.) were added. The reaction was stirred for 15 min followed by the addition of STAB-H (185 mg, 0. 872 mmol, 1.4 eq.). The resulting suspension was stirred overnight at RT. The reaction mixture was concentrated *in vacuo* and the resulting oil was mixed with an aqueous solution of NaOH (2M) . The formed solids were collected by vacuum filtration and triturated with MeOH to obtain the title compound as an off-white solid (17 mg, 6%). **LC/MS** (acidic mode) Rt 3.79 min, purity 94%, [M+H]⁺ calculated 418.23, found 418.25. **¹H NMR** (500 MHz, DMSO-d6) δ 8.92 - 8.83 (m, 1H), 8.09 - 7.99 (m, 1H), 7.62 - 7.51 (m, 1H), 7.35 - 7.25 (m, 5H), 7.25 - 7.20 (m, 1H), 7.16 - 7.12 (m, 1H), 3.50 - 3.39 (m, 2H), 3.16 (dd, J = 6.5, 6.5 Hz, 2H), 2.84 - 2.71 (m, 2H), 1.94 - 1.81 (m, 2H), 1.69 - 1.59 (m, 2H), 1.59 - 1.49 (m, 1H), 1.26 - 1.09 (m, 2H).**¹³C NMR** (126 MHz, DMSO-d6) δ 164.0, 163.6 (dd, J = 252.3, 12.7 Hz), 159.6, 159.0 (dd, J = 255.8, 13.7 Hz), 158.2, 138.7, 129.6 (dd, J = 10.0, 3.1 Hz), 128.7, 128.1, 126.8, 113.0 (d, J = 23.5 Hz), 111.9 - 111.2 (m), 105.5 (dd, J = 26.0, 26.0 Hz), 102.5 (d, J = 8.5 Hz), 62.4, 52.9, 44.4, 35.6, 29.7. **HRMS** m/z [M+H]⁺ C₂₃H₂₄F₂N₃O₂⁺ calculated 412.1831, found 412.1819.

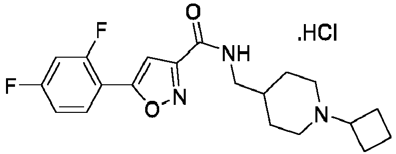

### N-((1-cyclobutylpiperidin-4-yl)methyl)-5-(2,4-difluorophenyl)isoxazole-3-carboxamide hydrochloride (18)

This compound was synthesised according to general procedure A. 5-(2,4-difluorophenyl)-N-(piperidin-4-ylmethyl)isoxazole-3-carboxamide (200 mg, 0.623 mmol, 1.0 eq.), AcOH (36 µL, 0. 62 mmol, 1.0 eq), cyclobutanone (65 µL, 0.87 mmol, 1.4 eq.) and STAB-H (185 mg, 0. 872 mmol, 1.4 eq.) were used. The formed HCI salt was triturated with MeOH to obtain the title compound as an off-white solid (169 mg, 66%). **LC/MS** (acidic mode) Rt 3.48 min, purity >98%, [M+H]⁺ calculated 376.18, found 376.20. **¹H NMR (500** MHz, DMSO-d6) δ 11.49 - 11.22 (m, 1H), 9.12 (t, J = 6.1 Hz, 1H), 8.05 (ddd, J = 8.7, 8.7, 6.3 Hz, 1H), 7.58 (ddd, J = 11.6, 9.3, 2.6 Hz, 1H), 7.33 (ddd, J = 8.6, 8.5, 2.2 Hz, 1H), 7.22 (d, J = 2.9 Hz, 1H), 3.58 - 3.47 (m, 1H), 3.30 - 3.23 (m, 2H), 3.16 (dd, J = 6.4, 6.4 Hz, 2H), 2.71 - 2.61 (m, 2H), 2.48 - 2.38 (m, 2H), 2.15 - 2.06 (m, 2H), 1.84 - 1.77 (m, 3H), 1.75 - 1.55 (m, 4H). **¹³C NMR** (126 MHz, DMSO-d6) δ 164.1, 163.7 (dd, J = 252.3, 12.4 Hz), 159.5, 159.0 (dd, J = 255.4, 12.8 Hz), 158.3, 129.7 (dd, J = 10.4, 3.5 Hz), 113.0 (dd, J = 22.2, 3.5 Hz), 111.5 (dd, J = 12.3, 3.5 Hz), 105.5 (dd, J = 25.9, 25.9 Hz), 102.5 (d, J = 8.2 Hz), 58.2, 48.0, 43.8, 33.6, 26.2, 24.7, 13.3. **HRMS** m/z [M+H]⁺ C₂₀H₂₄F₂N₃O₂⁺ calculated 376.1831, found 376.1829.

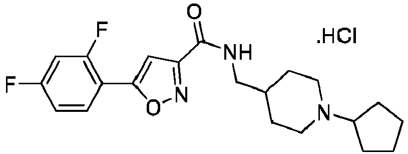

### N-((1-cyclopentylpiperidin-4-yl)methyl)-5-(2,4-difluorophenyl)isoxazole-3-carboxamide hydrochloride (19)

This compound was synthesised according to general procedure A. 5-(2,4-difluorophenyl)-N-(piperidin-4-ylmethyl)isoxazole-3-carboxamide (200 mg, 0.623 mmol, 1.0 eq.), AcOH (36 µL, 0. 62 mmol, 1.0 eq), cyclopentanone (77 µL, 0.87 mmol, 1.4 eq.) and STAB-H (185 mg, 0. 872 mmol, 1.4 eq.) were used. Additional cyclopentanone (77 µL, 0.87 mmol, 1.4 eq.) and STAB-H (185 mg, 0. 872 mmol, 1.4 eq.) were added two times over 2 days to complete the reaction. The formed HCI salt was triturated with MeOH to obtain the title compound as an off-white solid (114 mg, 43%). **LC/MS** (acidic mode) Rt 3.88 min, purity 95%, [M+H]⁺ calculated 390.20, found 390.15. **¹H NMR** (500 MHz, DMSO-d6) δ 10.84 (s, 1H), 9.09 (t, J = 6.0 Hz, 1H), 8.05 (ddd, J = 8.7, 8.6, 6.3 Hz, 1H), 7.58 (ddd, J = 11.6, 9.2, 2.6 Hz, 1H), 7.33 (ddd, J = 8.5, 8.3, 2.1 Hz, 1H), 7.20 (d, J = 2.9 Hz, 1H), 3.48 - 3.42 (m, 2H), 3.36 - 3.31 (m, 2H), 3.16 (dd, J = 6.3, 6.3 Hz, 2H), 2.89 - 2.79 (m, 2H), 2.00 - 1.93 (m, 2H), 1.88 - 1.76 (m, 4H), 1.74 - 1.56 (m, 4H), 1.56 - 1.46 (m, 2H). **¹³C NMR** (126 MHz, DMSO-d6) δ 164.1 (d, J = 2.6 Hz), 163.7 (dd, J = 252.2, 12.4 Hz), 159.6, 159.0 (dd, J = 255.5, 12.9 Hz), 158.4, 129.7, 113.0 (dd, J = 22.1, 3.5 Hz), 111.5 (dd, J = 12.3, 3.7 Hz), 105.5 (dd, J = 26.0, 26.0 Hz), 102.5 (d, J = 7.7 Hz), 66.6, 50.6, 43.7, 33.6, 27.5, 26.8, 23.3. **HRMS** m/z [M+H]⁺ C₂₁H₂₆F₂N₃O₂⁺ calculated 390.1988, found 390.2003.

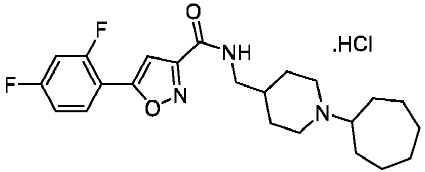

### N-((1-cycloheptylpiperidin-4-yl)methyl)-5-(2,4-difluorophenyl)isoxazole-3-carboxamide hydrochloride (20)

This compound was synthesised according to general procedure A. For the first step, 5-(2,4-difluorophenyl)-N-(piperidin-4-ylmethyl)isoxazole-3-carboxamide (200 mg, 0.623 mmol, 1.0 eq.), AcOH (36 µL, 0. 62 mmol, 1.0 eq), cycloheptanone (103 µL, 0.872 mmol, 1.4 eq.) and STAB-H (185 mg, 0. 872 mmol, 1.4 eq.) were used. The formed HCI salt was triturated with MeOH to obtain the title compound as an off-white solid (26 mg, 9%). **LC/MS** (acidic mode) Rt 3.92 min, purity 99%, [M+H]⁺ calculated 418.23, found 418.25. **¹H NMR** (500 MHz, DMSO-d6) δ 10.51 - 10.23 (m, 1H), 9.07 (t, J = 6.0 Hz, 1H), 8.05 (ddd, J = 8.7, 8.7, 6.4 Hz, 1H), 7.58 (ddd, J = 11.5, 9.2, 2.5 Hz, 1H), 7.33 (ddd, J = 8.6, 8.4, 2.2 Hz, 1H), 7.19 (d, J = 2.9 Hz, 1H), 3.32 - 3.20 (m, 3H), 3.16 (dd, J = 6.4, 6.4 Hz, 2H), 3.02 - 2.90 (m, 2H), 2.15 - 2.06 (m, 2H), 1.92 - 1.84 (m, 1H), 1.85 - 1.78 (m, 2H), 1.72 - 1.62 (m, 4H), 1.62 - 1.55 (m, 2H), 1.53 - 1.36 (m, 6H). ¹³C **NMR** (126 MHz, DMSO-d6) δ 164.0 (d, J = 2.7 Hz), 163.6 (dd, J = 252.2, 12.5 Hz), 159.6, 159.0 (dd, J = 255.4, 12.8 Hz), 158.3, 129.6 (dd, J = 10.3, 3.3 Hz), 113.0 (dd, J = 22.0, 3.5 Hz), 111.5 (dd, J = 12.4, 3.8 Hz), 105.5 (dd, J = 26.0, 26.0 Hz), 102.5 (d, J = 8.4 Hz), 65.5, 47.5, 43.8, 33.7, 27.5, 26.9, 26.7, 24.1. **HRMS** m/z [M+H]⁺ C₂₃H₃₀F₂N₃O₂⁺ calculated 418.2301, found 418.2302.

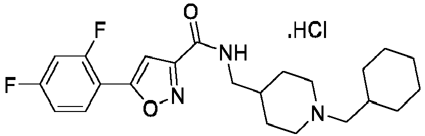

### N-((1-(cyclohexylmethyl)piperidin-4-yl)methyl)-5-(2,4-difluorophenyl)isoxazole-3-carboxamide hydrochloride (22)

This compound was synthesised according to general procedure A. 5-(2,4-difluorophenyl)-N-(piperidin-4-ylmethyl)isoxazole-3-carboxamide (200 mg, 0.623 mmol, 1.0 eq.), AcOH (36 µL, 0. 62 mmol, 1.0 eq), cyclohexanecarboxaldehyde (106 µL, 0.872 mmol, 1.4 eq.) and STAB-H (185 mg, 0. 872 mmol, 1.4 eq.) was used. The HCI salt was purified by RP column chromatography (using a linear gradient of 30-50% ACN/H₂O with 0.1% FA). The product fractions were combined, basified with an aqueous solution of NaOH (2M) and extracted with DCM (3 times). The organic layer was dried over anhydrous Na₂SO₄, filtered and an excess of 4M HCI in dioxane was added. The resulting mixture was concentrated *in vacuo* to obtain the title compound as an off-white solid (187 mg, 72%). **LC/MS** (acidic mode) Rt 3.91 min, purity >99%, [M+H]⁺ calculated 418.23, found 418.25. **¹H NMR** (500 MHz, DMSO-d6) δ 10.53 - 10.24 (m, 1H), 9.12 (t, J = 6.0 Hz, 1H), 8.04 (ddd, J = 8.7, 8.6, 6.3 Hz, 1H), 7.57 (ddd, J = 11.5, 9.1, 2.6 Hz, 1H), 7.33 (ddd, J = 8.5, 8.5, 2.5 Hz, 1H), 7.22 (d, J = 2.9 Hz, 1H), 3.49 - 3.43 (m, 2H), 3.19 - 3.10 (m, 2H), 2.98 - 2.77 (m, 4H), 1.98 - 1.69 (m, 8H), 1.69 - 1.54 (m, 3H), 1.27 - 1.16 (m, 2H), 1.16 - 1.05 (m, 1H), 0.99 - 0.86 (m, 2H). **¹³C NMR** (126 MHz, DMSO-d6) δ 164.0 (d, J = 2.8 Hz), 163.7 (dd, J = 252.0, 12.3 Hz), 159.6, 159.0 (dd, J = 255.4, 12.9 Hz), 158.4, 129.6 (dd, J = 10.4, 3.2 Hz), 113.0 (dd, J = 22.1, 3.5 Hz), 111.5 (dd, J = 12.5, 3.9 Hz), 105.5 (dd, J = 25.9, 25.9 Hz), 102.5 (d, J = 8.2 Hz), 62.2, 52.0, 43.8, 33.5, 32.1, 31.0, 26.4, 25.5, 25.1. **HRMS** m/z [M+H]⁺ C₂₃H₃₀F₂N₃O₂⁺ calculated 418.2301, found 418.2307.

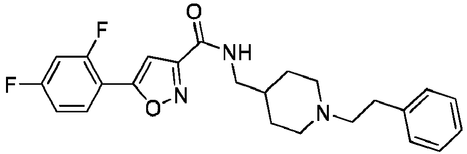

### 5-(2,4-difluorophenyl)-N-((1-phenethylpiperidin-4-yl)methyl)isoxazole-3-carboxamide (23)

5-(2,4-difluorophenyl)-N-(piperidin-4-ylmethyl)isoxazole-3-carboxamide (200 mg, 0.623 mmol, 1.0 eq.) was suspended in DMF (2.8 mL) that contained AcOH (36 µL, 0.62 mmol, 1.0 eq.) and 2-phenylacetaldehyde (97 µL, 0.87 mmol, 1.4 eq.). The mixture was stirred at RT for 10 min. STAB-H (185 mg, 0.871 mmol, 1.4 eq.) were added and the reaction mixture was stirred overnight. The DMF was removed *in vacuo* and the resulting crude product was suspended in an aqueous solution of NaOH (2M) . The formed solids were collected by vacuum filtration. The crude product was dissolved in dioxane and converted to the HCI salt by addition of 4M HCI in dioxane. The resulting HCI salt was purified by RP column chromatography (using a linear gradient of 10% ACN/H₂O with 0.1% FA to 100% ACN). The product fractions were combined, basified with an aqueous solution of NaOH (2M) and extracted three times with DCM. The combined DCM layers were dried over anhydrous Na₂SO₄, filtered and concentrated *in vacuo,* yielding the title compound as an off-white solid (178 mg, 67%). **LC/MS** (acidic mode) Rt 3.90 min, purity >99%, [M+H]⁺ calculated 426.20, found 426.25. **¹H NMR** (500 MHz, DMSO-d6) δ 11.33 - 11.14 (m, 1H), 9.13 (t, J = 5.9 Hz, 1H), 8.08 - 8.01 (m, 1H), 7.61 - 7.53 (m, 1H), 7.32 (dd, J = 9.8, 5.0 Hz, 3H), 7.26 (d, J = 7.7 Hz, 2H), 7.24 - 7.21 (m, 2H), 3.57 - 3.49 (m, 2H), 3.24 - 3.14 (m, 4H), 3.14 - 3.07 (m, 2H), 3.00 - 2.85 (m, 2H), 1.92 - 1.81 (m, 3H), 1.71 - 1.59 (m, 2H). **¹³C NMR** 126 MHz, DMSO-d6) δ 164.0 (d, J = 2.7 Hz), 163.6 (dd, J = 252.2, 12.5 Hz), 159.5, 159.0 (dd, J = 255.2, 12.8 Hz), 158.3, 137.4, 129.6 (dd, J = 10.3, 3.3 Hz), 128.7, 128.6, 126.7, 113.0 (dd, J = 22.2, 3.5 Hz), 111.5 (dd, J = 12.3, 3.6 Hz), 105.5 (dd, J = 25.8, 25.8 Hz), 102.5 (d, J = 8.4 Hz), 56.6, 51.3, 43.7, 33.6, 29.3, 26.8. **HRMS** m/z [M+H]⁺ C₂₄H₂₆F₂N₃O₂⁺ calculated 426.1988, found 426.1995.

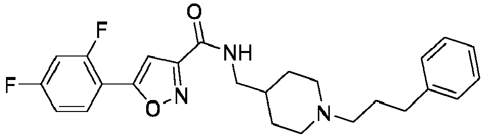

### 5-(2,4-difluorophenyl)-N-((1-(3-phenylpropyl)piperidin-4-yl)methyl)isoxazole-3-carboxamide (24)

5-(2,4-difluorophenyl)-N-(piperidin-4-ylmethyl)isoxazole-3-carboxamide hydrochloride (100 mg, 0.279 mmol, 1.0 eq.) was suspended in DMF (2.8 mL) and TEA (39 µL, 0.28 mmol, 1.0 eq.), AcOH (16 µL, 0.28 mmol, 1.0 eq.) and hydrocinnamaldehyde (44 µL, 0.34 mmol, 1.2 eq.) were added. After stirring for 10 min at RT, STAB-H (71 mg, 0.34 mmol, 1.2 eq.) was added and the reaction mixture was stirred overnight at RT. Additional hydrocinnamaldehyde (44 µL, 0.34 mmol, 1.2 eq.) and STAB-H (71 mg, 0.34 mmol, 1.2 eq.) were added to complete the reaction. MeOH was added to terminate the reaction and the volatiles were removed by *in vacuo.* The resulting solids were suspended in an aqueous solution of NaOH (2M) and extracted with DCM (3 times). The combined organic layers were dried over anhydrous Na₂SO₄, filtered and concentrated *in vacuo.* The crude product was purified by NP column chromatography (using a linear gradient of 10-60% (EtOAc:MeOH:TEA, 90:5:5, v:v:v%)/cyclohexane) to obtain the title compound as an off-white solid (66 mg, 54%). **LC/MS** (acidic mode) Rt 4.33 min, purity 99%, [M+H]⁺ calculated 440.21, found 440.25. **¹H NMR** (500 MHz, DMSO-d6) δ 8.87 (t, J = 6.0 Hz, 1H), 8.05 (ddd, J = 8.7, 8.7, 6.3 Hz, 1H), 7.58 (ddd, J = 11.5, 9.2, 2.5 Hz, 1H), 7.36 - 7.30 (m, 1H), 7.26 (t, J = 7.5 Hz, 2H), 7.20 - 7.17 (m, 2H), 7.16 - 7.12 (m, 2H), 3.15 (dd, J = 6.4, 6.4 Hz, 2H), 2.86 - 2.77 (m, 2H), 2.60 - 2.53 (m, 2H), 2.26 - 2.20 (m, 2H), 1.83 - 1.75 (m, 2H), 1.75 -1.66 (m, 2H), 1.66 -1.59 (m, 2H), 1.59 - 1.49 (m, 1H), 1.23 - 1.10 (m, 2H). **¹³C NMR** (126 MHz, DMSO-d6) δ 164.2 - 163.9 (m), 163.6 (dd, *J* = 252.0, 12.3 Hz), 159.6, 159.0 (dd, J = 255.2, 12.7 Hz), 158.2, 142.2, 129.6 (dd, *J* = 10.4, 3.3 Hz), 129.0, 128.6 - 127.9 (m), 125.6, 113.0 (dd, J = 22.2, 3.4 Hz), 111.5 (dd, J = 12.4, 3.9 Hz), 105.5 (dd, J = 26.0, 26.0 Hz), 102.5 (d, *J* = 8.3 Hz), 57.5, 53.1, 44.5, 35.7, 33.0, 29.8, 28.5. **HRMS** m/z [M+H]⁺ C₂₅H₂₈F₂N₃O₂⁺ calculated 440.2144, found 440.2150.

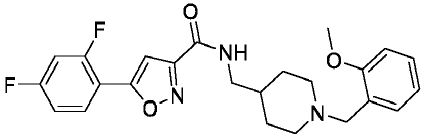

### 5-(2,4-difluorophenyl)-N-((1-(2-methoxybenzyl)piperidin-4-yl)methyl)isoxazole-3-carboxamide (25)

This compound was synthesised according to the general procedure D. 5-(2,4-difluorophenyl)-N-(piperidin-4-ylmethyl)isoxazole-3-carboxamide hydrochloride (100 mg, 0.279 mmol, 1.0 eq.), TEA (39 µL, 0.28 mmol, 1.0 eq.), AcOH (16 µL, 0.28 mmol, 1.0 eq), 2-methoxybenzaldehyde (46 mg, 0.34 mmol, 1.2 eq.) and STAB-H (71 mg, 0.34 mmol, 1.2 eq.) were used. The crude product was purified by NP column chromatography (10-50% (EtOAc:MeOH:TEA, 90:5:5, v:v:v%)/cyclohexane ) to obtain the title compound as an off-white solid (62 mg, 50%). **LC/MS** (acidic mode) Rt 4.18 min, purity >97%, [M+H]⁺ calculated 442.19, found 442.25. **¹H NMR** (500 MHz, DMSO-d6) δ 8.88 (t, J = 6.0 Hz, 1H), 8.05 (ddd, J = 8.7, 8.7, 6.3 Hz, 1H), 7.57 (ddd, J = 11.6, 9.2, 2.6 Hz, 1H), 7.35 - 7.29 (m, 1H), 7.30 - 7.28 (m, 1H), 7.23 - 7.18 (m, 1H), 7.15 (d, J = 2.9 Hz, 1H), 6.95 (dd, J = 8.3, 1.1 Hz, 1H), 6.90 (ddd, J = 7.4, 7.4, 1.1 Hz, 1H), 3.75 (s, 3H), 3.43 (s, 2H), 3.16 (dd, J = 6.4, 6.4 Hz, 2H), 2.85 - 2.78 (m, 2H), 1.97 - 1.89 (m, 2H), 1.66 - 1.60 (m, 2H), 1.60 - 1.51 (m, 1H), 1.24 - 1.18 (m, 2H). **¹³C NMR** (126 MHz, DMSO-d6) δ 164.0 (d, J = 2.2 Hz), 163.6 (dd, J = 252.0, 12.7 Hz), 159.6, 159.0 (dd, J = 255.2, 13.2 Hz), 158.2, 157.3, 129.6 (dd, J = 10.9, 3.7 Hz), 129.6, 127.8, 126.2 *, 120.1, 113.0 (dd, J = 22.0, 3.5 Hz), 111.5 (dd, J = 12.5, 3.9 Hz), 110.7, 105.5 (dd, J = 26.0, 26.0 Hz), 102.5 (d, J = 8.5 Hz), 55.7, 55.3, 53.1, 44.5, 35.5, 29.8. *Identified using HMBC **HRMS** m/z [M+H]⁺ C₂₄H₂₆F₂N₃O₃⁺ calculated 442.1937, found 442.1950.

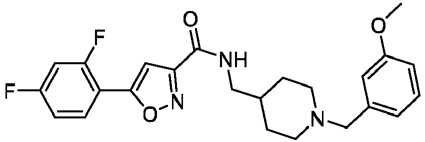

### 5-(2,4-difluorophenyl)-N-((1-(3-methoxybenzyl)piperidin-4-yl)methyl)isoxazole-3-carboxamide (26)

This compound was synthesised according to the general procedure D. 5-(2,4-difluorophenyl)-N-(piperidin-4-ylmethyl)isoxazole-3-carboxamide hydrochloride (100 mg, 0.279 mmol, 1.0 eq.), TEA (39 µL, 0.28 mmol, 1.0 eq.), AcOH (16 µL, 0.28 mmol, 1.0 eq), 3-methoxybenzaldehyde (41 µL, 0.34 mmol, 1.2 eq.) and STAB-H (71 mg, 0.34 mmol, 1.2 eq.) were used. The crude product was purified by NP column chromatography (using a linear gradient of 10-60% (EtOAc:MeOH:TEA, 90:5:5, v:v:v%)/cyclohexane) to obtain the title compound as an off-white solid (67 mg, 54%). **LC/MS** (acidic mode) Rt 4.04 min, purity >98%, [M+H]⁺ calculated 442.19, found 442.20. **¹H NMR** (500 MHz, CDCl₃) δ 7.93 (ddd, J = 8.5, 8.5, 6.2 Hz, 1H), 7.22 (dd, J = 8.0, 8.0 Hz, 1H), 7.09 (d, J = 3.7 Hz, 1H), 7.07 - 7.01 (m, 1H), 7.01 - 6.95 (m, 1H), 6.95 - 6.91 (m, 1H), 6.92 - 6.87 (m, 1H), 6.89 - 6.88 (m, 1H), 6.82 - 6.76 (m, 1H), 3.80 (s, 3H), 3.48 (s, 2H), 3.37 (dd, J = 6.5, 6.5 Hz, 2H), 2.96 - 2.88 (m, 2H), 2.02 - 1.93 (m, 2H), 1.77 - 1.70 (m, 2H), 1.69 - 1.59(m, 1H), 1.44 - 1.32 (m, 2H). **¹³C NMR** (126 MHz, CDCl₃)δ 164.8 (dd, J = 2.7, 1.1 Hz), 164.3 (dd, J = 254.8, 12.0 Hz), 159.8 (dd, J = 257.4, 12.2 Hz), 159.7, 159.5, 158.9, 140.0 *, 129.3, 128.9 (dd, J = 10.2, 3.4 Hz), 121.6, 114.7, 112.6, 112.5 (dd, J = 21.8, 3.6 Hz), 112.1 (dd, J = 12.5, 3.9 Hz), 105.2 (dd, J = 25.4, 25.4 Hz), 102.8 (d, J = 11.4 Hz), 63.3, 55.3, 53.4, 45.1, 36.2, 30.0. *Identified using HMBC **HRMS** m/z [M+H]⁺ C₂₄H₂₆F₂N₃O₃⁺ calculated 442.1937, found 442.1937.

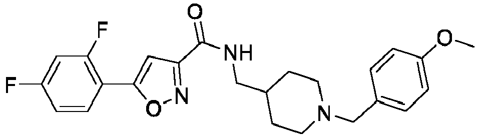

### 5-(2,4-difluorophenyl)-N-((1-(4-methoxybenzyl)piperidin-4-yl)methyl)isoxazole-3-carboxamide (27)

This compound was synthesised according to the general procedure D. 5-(2,4-difluorophenyl)-N-(piperidin-4-ylmethyl)isoxazole-3-carboxamide hydrochloride (100 mg, 0.279 mmol, 1.0 eq.), TEA (39 µL, 0.28 mmol, 1.0 eq.), AcOH (16 µL, 0.28 mmol, 1.0 eq), 4-methoxybenzaldehyde (41 µL, 0.34 mmol, 1.2 eq.) and STAB-H (71 mg, 0.34 mmol, 1.2 eq.) were used. Additional 4-methoxybenzaldehyde (41 µL, 0.34 mmol, 1.2 eq.) and STAB-H (71 mg, 0.34 mmol, 1.2 eq.) were added to drive the reaction to completion. The crude product was purified by NP column chromatography (using a linear gradient of 10-50% (EtOAc:MeOH:TEA, 90:5:5, v:v:v%)/cyclohexane) to obtain the title compound as an off-white solid (84 mg, 68%). **LC/MS** (acidic mode) Rt 4.08 min, purity 99%, [M+H]⁺ calculated 442.19, found 442.20. **¹H NMR** (500 MHz, CDCl₃) δ 7.92 (ddd, J = 8.5, 8.5, 6.2 Hz, 1H), 7.23 - 7.18 (m, 2H), 7.08 (d, J = 3.7 Hz, 1H), 7.05 - 7.00 (m, 1H), 7.00 - 6.91 (m, 2H), 6.86 - 6.81 (m, 2H), 3.79 (s, 3H), 3.43 (s, 2H), 3.36 (dd, J = 6.5, 6.5 Hz, 2H), 2.93 - 2.86 (m, 2H), 1.97 - 1.89 (m, 2H), 1.76 - 1.68 (m, 2H), 1.68 - 1.58 (m, 1H), 1.40 - 1.30 (m, 2H). **¹³C NMR** (126 MHz, CDCl₃)δ 164.7 (d, J = 2.7 Hz), 164.3 (dd, J = 254.8, 11.9 Hz), 159.8 (dd, J = 257.4, 12.2 Hz), 159.5, 158.9, 158.8, 130.5, 130.4, 128.9 (dd, J = 10.1, 3.4 Hz), 113.6, 112.5 (dd, J = 22.0, 3.6 Hz), 112.0 (dd, J = 12.5, 3.9 Hz), 105.2 (dd, J = 25.4, 25.4 Hz), 102.8 (d, J = 10.6 Hz), 62.8, 55.3, 53.2, 45.1, 36.2, 30.0. **HRMS** m/z [M+H]⁺ C₂₄H₂₆F₂N₃O₃⁺ calculated 442.1937, found 442.1931.

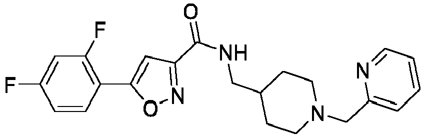

### 5-(2,4-difluorophenyl)-N-((1-(pyridin-2-ylmethyl)piperidin-4-yl)methyl)isoxazole-3-carboxamide (28)

This compound was synthesised according to the general procedure D. 5-(2,4-difluorophenyl)-N-(piperidin-4-ylmethyl)isoxazole-3-carboxamide hydrochloride (100 mg, 0.279 mmol, 1.0 eq.), TEA (39 µL, 0.28 mmol, 1.0 eq.), AcOH (16 µL, 0.28 mmol, 1.0 eq), 2-pyridinecarboxaldehyde (32 µL, 0.34 mmol, 1.2 eq.) and STAB-H (71 mg, 0.34 mmol, 1.2 eq.) were used. Additional 2-pyridinecarboxaldehyde (32 µL, 0.34 mmol, 1.2 eq.) and STAB-H (71 mg, 0.34 mmol, 1.2 eq.) were added to drive the reaction to completion. The crude product was purified by NP column chromatography (using a linear gradient of 50-100% (EtOAc:MeOH:TEA, 90:5:5, v:v:v%)/cyclohexane) to obtain the title compound as an off-white solid (82 mg, 71%). **LC/MS** (acidic mode) Rt 3.82 min, purity 99%, [M+H]⁺ calculated 413.18, found 413.20. **¹H NMR** (500 MHz, CDCl₃) δ 8.57 - 8.52 (m, 1H), 7.93 (ddd, J = 8.5, 8.5, 6.2 Hz, 1H), 7.64 (ddd, J = 7.7, 7.7, 1.8 Hz, 1H), 7.48 - 7.40 (m, 1H), 7.20 - 7.13 (m, 1H), 7.08 (d, J = 3.7 Hz, 1H), 7.06 - 7.00 (m, 1H), 7.00 - 6.90 (m, 2H), 3.68 (s, 2H), 3.37 (dd, J = 6.5, 6.5 Hz, 2H), 3.00 - 2.90 (m, 2H), 2.19 - 2.05 (m, 2H), 1.80 - 1.72 (m, 2H), 1.72 - 1.57 (m, 1H), 1.57 - 1.40 (m, 2H). **¹³C NMR** (126 MHz, CDCl₃)δ 164.9 - 164.7 (m), 164.3 (dd, J = 255.0, 12.0 Hz), 159.8 (dd, J = 257.3, 12.0 Hz), 159.5, 158.9, 158.3*, 149.4, 136.6, 128.9 (dd, J = 10.0, 3.5 Hz), 123.5, 122.2, 112.5 (dd, J = 21.8, 3.6 Hz), 112.0 (dd, J = 12.3, 4.1 Hz), 105.2 (dd, J = 25.4, 25.4 Hz), 102.8 (d, J = 10.6 Hz), 64.8, 53.6, 45.0, 36.0, 29.8. *Identified using HMBC **HRMS** m/z [M+H]⁺ C₂₂H₂₃F₂N₄O₂⁺ calculated 413.1784, found 413.1785.

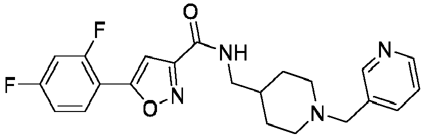

### 5-(2,4-difluorophenyl)-N-((1-(pyridin-3-ylmethyl)piperidin-4-yl)methyl)isoxazole-3-carboxamide (29)

This compound was synthesised according to the general procedure D. 5-(2,4-difluorophenyl)-N-(piperidin-4-ylmethyl)isoxazole-3-carboxamide hydrochloride (100 mg, 0.279 mmol, 1.0 eq.), TEA (39 µL, 0.28 mmol, 1.0 eq.), AcOH (16 µL, 0.28 mmol, 1.0 eq), 3-pyridinecarboxaldehyde (31 µL, 0.34 mmol, 1.2 eq.) and STAB-H (71 mg, 0.34 mmol, 1.2 eq.) were used. Additional 3-pyridinecarboxaldehyde (31 µL, 0.34 mmol, 1.2 eq.) and STAB-H (71 mg, 0.34 mmol, 1.2 eq.) were added to drive the reaction to completion. The crude product was purified by NP column chromatography (using a linear gradient of 50-100% (EtOAc:MeOH:TEA, 90:5:5, v:v:v%)/cyclohexane) to obtain the title compound as an off-white solid (83 mg, 72%). **LC/MS** (acidic mode) Rt 3.55 min, purity >95%, [M+H]⁺ calculated 413.18, found 413.15. **¹H NMR** (500 MHz, DMSO-d6) δ 8.88 (t, J = 6.0 Hz, 1H), 8.48 - 8.47 (m, 1H), 8.46 - 8.44 (m, 1H), 8.05 (ddd, J = 8.7, 8.7, 6.3 Hz, 1H), 7.72 - 7.66 (m, 1H), 7.58 (ddd, J = 11.6, 9.3, 2.6 Hz, 1H), 7.37 - 7.29 (m, 2H), 7.15 (d, J = 3.0 Hz, 1H), 3.48 (s, 2H), 3.16 (dd, J = 6.4, 6.4 Hz, 2H), 2.82 - 2.72 (m, 2H), 1.98 - 1.88 (m, 2H), 1.74 - 1.60 (m, 2H), 1.67 - 1.47 (m, 1H), 1.24 - 1.12 (m, 2H). ¹³C **NMR** (126 MHz, DMSO-d6) δ 164.3 - 163.8 (m), 163.6 (dd, J = 252.0, 12.3 Hz), 159.6, 159.0 (dd, J = 255.2, 12.7 Hz), 158.2, 150.0, 148.2, 136.5, 133.9 *, 129.6 (dd, J = 10.4, 3.2 Hz), 123.4, 113.0 (dd, J = 22.1, 3.4 Hz), 111.5 (dd, J = 12.6, 3.8 Hz), 105.5 (dd, J = 25.9, 25.9 Hz), 102.5 (d, J = 8.4 Hz), 59.4, 52.8, 44.4, 35.4, 29.6. *Identified using HMBC **HRMS** m/z [M+H]⁺ C₂₂H₂₃F₂N₄O₂⁺ calculated 413.1784, found 413.1793.

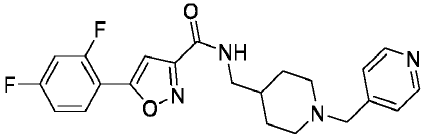

### 5-(2,4-difluorophenyl)-N-((1-(pyridin-4-ylmethyl)piperidin-4-yl)methyl)isoxazole-3-carboxamide (30)

This compound was synthesised according to the general procedure D. 5-(2,4-difluorophenyl)-N-(piperidin-4-ylmethyl)isoxazole-3-carboxamide hydrochloride (100 mg, 0.279 mmol, 1.0 eq.), TEA (39 µL, 0.28 mmol, 1.0 eq.), AcOH (16 µL, 0.28 mmol, 1.0 eq), 4-pyridinecarboxaldehyde (32 µL, 0.34 mmol, 1.2 eq.) and STAB-H (71 mg, 0.34 mmol, 1.2 eq.) were used. Additional 4-pyridinecarboxaldehyde (32 µL, 0.34 mmol, 1.2 eq.) and STAB-H (71 mg, 0.34 mmol, 1.2 eq.) were added to drive the reaction to completion. The crude product was purified by NP column chromatography (using a linear gradient of 50-100% (EtOAc:MeOH:TEA, 90:5:5, v:v:v%)/cyclohexane) to obtain the title compound as an off-white solid (86 mg, 75%). **LC/MS** (acidic mode) Rt 3.45 min, purity >99%, [M+H]⁺ calculated 413.18, found 413.15. **¹H NMR** (500 MHz, DMSO-d6) δ 8.89 (t, J = 6.0 Hz, 1H), 8.49 (d, J = 5.9 Hz, 2H), 8.05 (ddd, J = 8.7, 8.6, 6.3 Hz, 1H), 7.57 (ddd, J = 11.5, 9.2, 2.6 Hz, 1H), 7.35- 7.28 (m, 3H), 7.15 (d, J = 3.0 Hz, 1H), 3.48 (s, 2H), 3.17 (dd, J = 6.4, 6.4 Hz, 2H), 2.84 - 2.70 (m, 2H), 2.02 - 1.86 (m, 2H), 1.70 - 1.61 (m, 2H), 1.61 - 1.50 (m, 1H), 1.27 - 1.13 (m, 2H). **¹³C NMR** (126 MHz, DMSO-d6) δ 164.4 - 163.8 (m), 163.6 (dd, J = 252.3, 12.5 Hz), 159.6, 159.0 (dd, J = 255.2, 13.2 Hz), 158.2, 149.5, 148.9 *, 129.6 (dd, J = 10.3, 3.3 Hz), 123.7, 113.7 - 111.5 (m), 111.4 (d, J = 3.6 Hz), 105.5 (dd, J = 26.0, 26.0 Hz), 102.5 (d, J = 8.5 Hz), 60.9, 52.9, 44.4, 35.4, 29.6. *Identified using HMBC **HRMS** m/z [M+H]⁺ C₂₂H₂₃F₂N₄O₂⁺ calculated 413.1784, found 413.1791.

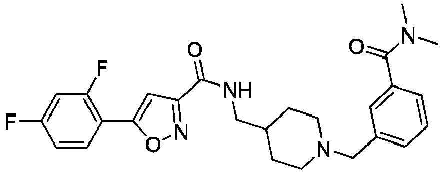

### 5-(2,4-difluorophenyl)-N-((1-(3-(dimethylcarbamoyl)benzyl)piperidin-4-yl)methyl)isoxazole-3-carboxamide(31)

This compound was synthesised according to the general procedure D. 5-(2,4-difluorophenyl)-N-(piperidin-4-ylmethyl)isoxazole-3-carboxamide hydrochloride (100 mg, 0.279 mmol, 1.0 eq.), TEA (39 µL, 0.28 mmol, 1.0 eq.), 3-formyl-N,N-dimethylbenzamide (59 mg, 0.34 mmol, 1.2 eq.) and STAB-H (71 mg, 0.34 mmol, 1.2 eq.) were used. Additional 3-formyl-N,N-dimethylbenzamide (59 mg, 0.34 mmol, 1.2 eq.) and STAB-H (142 mg, 0.671 mmol, 2.4 eq.) were added to drive the reaction to completion. The crude product was purified by NP column chromatography (using a linear gradient of 10-60% (EtOAc:MeOH:TEA, 90:5:5, v:v:v%)/cyclohexane) to obtain the title compound as an off-white solid (32 mg, 24%). **LC/MS** (acidic mode) Rt 3.78 min, purity 98%, [M+H]⁺ calculated 483.22, found 483.15. **¹H NMR** (500 MHz, DMSO-d6) δ 8.98 - 8.84 (m, 1H), 8.12 - 8.00 (m, 1H), 7.61 - 7.53 (m, 1H), 7.49 - 7.18 (m, 4H), 7.27 - 7.19 (m, 1H), 7.20 - 7.11 (m, 1H), 3.47 (s, 2H), 3.22 - 3.11 (m, 2H), 2.97 (s, 3H), 2.88 (s, 3H), 2.82 - 2.75 (m, 2H), 1.94 - 1.85 (m, 2H), 1.77 - 1.50 (m, 3H), 1.26 - 1.12 (m, 2H). **¹³C NMR** (126 MHz, DMSO-d6) δ 170.2, 164.0, 163.6 (dd, J = 252.0, 12.3 Hz), 159.6, 159.0 (dd, J = 255.2, 12.7 Hz), 158.2, 138.9, 136.3, 129.7 (d, J = 3.1 Hz), 129.6, 128.1, 127.0, 125.4, 113.0 (dd, J = 22.2, 3.4 Hz), 111.5 (dd, J = 12.5, 3.9 Hz), 105.5 (dd, J = 26.0, 26.0 Hz), 102.5 (d, J = 8.2 Hz), 61.9, 52.8, 44.4, 35.5, 34.7, 29.7. Rotamers are observed. **HRMS** m/z [M+H]⁺ C₂₆H₂₉F₂N₄O₃⁺ calculated 483.2202, found 483.2220.

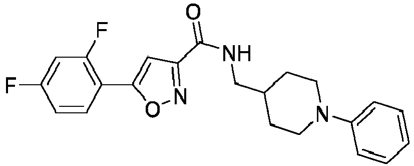

### 5-(2,4-difluorophenyl)-N-((1-phenylpiperidin-4-yl)methyl)isoxazole-3-carboxamide (32)

A suspension of 5-(2,4-difluorophenyl)-N-(piperidin-4-ylmethyl)isoxazole-3-carboxamide hydrochloride (98 mg, 0.274 mmol, 1.0 eq.), bromobenzene (58 µL, 0.55 mmol, 2.0 eq.), NaO*t*Bu (53 mg, 0.55 mmol, 2.0 eq.)) and S-Phos (11 mg, 0.027 mmol, 0.1 eq.) in dry PhMe (1.4 mL). Pd₂dba₃·CHCl₃ (14 mg, 0.14 mmol, 0.05 eq.) was added and the reaction mixture was heated to reflux by microwave irradiation for 19 h. Additional S-Phos (11 mg, 0.027 mmol, 0.1 eq.) and Pd₂dba₃·CHCl₃ (14 mg, 0.14 mmol, 0.05 eq.) were added and the reaction mixture was heated for an additional 14 h. The reaction mixture was cooled, filtered over celite and the residue washed with EtOAc. The filtrate was concentrated *in vacuo.* The resulting crude was purified by NP column chromatography (using a linear gradient of 10-50% (EtOAc:MeOH:TEA, 90:5:5, v:v:v%)/cyclohexane) to obtain a yellow solid. The solid was triturated with MeOH to obtain the title compound as a white solid (15 mg, 14%). LC/MS (acidic mode) Rt 4.40 min, purity 99%, [M+H]⁺ calculated 398.17, found 398.10. **¹H NMR** (500 MHz, DMSO-d6) δ 8.95 (t, J = 6.0 Hz, 1H), 8.06 (ddd, J = 8.6, 8.6, 6.3 Hz, 1H), 7.58 (ddd, J = 11.6, 9.3, 2.5 Hz, 1H), 7.33 (ddd, J = 8.5, 8.5, 2.6 Hz, 1H), 7.21 - 7.15 (m, 3H), 6.92 (d, J = 8.1 Hz, 2H), 6.73 (t, J = 7.2 Hz, 1H), 3.72 - 3.64 (m, 2H), 3.25 - 3.18 (m, 2H), 2.67 - 2.58 (m, 2H), 1.81 - 1.69 (m, 3H), 1.33 - 1.23 (m, 2H). **¹³C NMR** (126 MHz, DMSO-d6) δ 164.0 (d, J = 1.7 Hz), 163.6 (dd, J = 251.6, 12.2 Hz), 159.6, 159.0 (dd, J = 255.3, 12.7 Hz), 158.3, 151.3, 129.6 (dd, J = 10.1, 3.3 Hz), 128.9, 118.4, 115.8, 113.0 (dd, J = 22.2, 3.5 Hz), 111.5 (dd, J = 12.6, 4.0 Hz), 105.5 (dd, J = 26.0, 26.0 Hz), 102.5 (d, J = 9.1 Hz), 48.5, 44.3, 35.5, 29.3. **HRMS** m/z [M+H]⁺ C₂₂H₂₂F₂N₃O₂⁺ calculated 398.1675, found 398.1680.

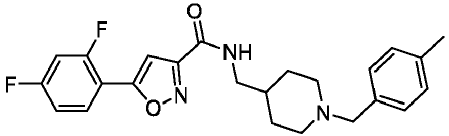

### 5-(2,4-difluorophenyl)-N-((1-(4-methylbenzyl)piperidin-4-yl)methyl)isoxazole-3-carboxamide (33)

This compound was synthesised according to general procedure C. 5-(2,4-difluorophenyl)-N-(piperidin-4-ylmethyl)isoxazole-3-carboxamide hydrochloride (100 mg, 0.279 mmol, 1.0 eq.), K₂CO₃ (77 mg, 0.56 mmol, 2.0 eq.) and 4-methylbenzyl bromide (57 mg, 0.31 mmol, 1.1 eq.) were used. The crude product was purified by NP column chromatography (using a linear gradient of 10-50% (EtOAc:MeOH:TEA, 90:5:5, v:v:v%)/cyclohexane) to obtain the title compound as a white solid (28 mg, 24%). **LC/MS** (acidic mode) Rt 4.50 min, purity 99%, [M+H]⁺ calculated 426.20, found 426.20. **¹H NMR** (500 MHz, DMSO-d6) δ 8.89 (t, J = 6.0 Hz, 1H), 8.05 (ddd, J = 8.7, 8.7, 6.3 Hz, 1H), 7.57 (ddd, J = 11.5, 9.2, 2.6 Hz, 1H), 7.32 (ddd, J = 8.5, 8.5, 2.6 Hz, 1H), 7.23 - 7.08 (m, 5H), 3.48 - 3.41 (m, 2H), 3.16 (dd, J = 6.3, 6.3 Hz, 2H), 2.86 - 2.74 (m, 2H), 2.27 (s, 3H), 2.02 - 1.84 (m, 2H), 1.69 - 1.61 (m, 2H), 1.61 - 1.52 (m, 1H), 1.26 - 1.12 (m, 2H). **¹³C NMR** (126 MHz, DMSO-d6) δ 164.0, 164.0, 163.7 (dd, J = 251.8, 12.0 Hz), 159.6, 159.0 (dd, J = 255.2, 12.7 Hz), 158.2, 136.5 *130.6 - 129.4 (m), 128.9, 128.8, 113.0 (dd, J = 22.1, 3.4 Hz), 111.5 (dd, J = 12.5, 3.9 Hz), 105.5 (dd, J = 26.0, 26.0 Hz), 102.5 (d, J = 8.4 Hz), 61.9, 52.7, 44.4, 35.4, 29.5, 20.7. *Identified using HMBC **HRMS** m/z [M+H]⁺ C₂₄H₂₆F₂N₃O₂⁺ calculated 426.1988, found 426.1994.

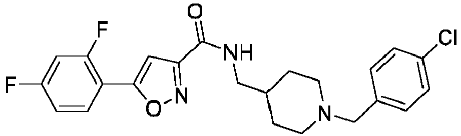

### N-((1-(4-chlorobenzyl)piperidin-4-yl)methyl)-5-(2,4-difluorophenyl)isoxazole-3-carboxamide (34)

This compound was synthesised according to general procedure C. 5-(2,4-difluorophenyl)-N-(piperidin-4-ylmethyl)isoxazole-3-carboxamide hydrochloride (100 mg, 0.279 mmol, 1.0 eq.), K₂CO₃ (77 mg, 0.56 mmol, 2.0 eq.) and 4-chlorobenzyl bromide (63 mg, 0.31 mmol, 1.1 eq.) were used. The crude product was purified by NP column chromatography (using a linear gradient of 10-50% (EtOAc:MeOH:TEA, 90:5:5, v:v:v%)/cyclohexane) to obtain the title compound as a white solid (71 mg, 57%). **LC/MS** (acidic mode) Rt 4.52 min, purity 98%, [M+H]⁺ calculated 446.14, found 446.10. **¹H NMR** (500 MHz, DMSO-d6) δ 8.88 (t, J = 6.0 Hz, 1H), 8.05 (ddd, J = 8.7, 8.7, 6.3 Hz, 1H), 7.57 (ddd, J = 11.6, 9.2, 2.6 Hz, 1H), 7.37 - 7.28 (m, 5H), 7.14 (d, J = 3.0 Hz, 1H), 3.42 (s, 2H), 3.15 (dd, J = 6.4, 6.4 Hz, 2H), 2.81 - 2.71 (m, 2H), 1.93 - 1.85 (m, 2H), 1.67 - 1.60 (m, 2H), 1.60 - 1.51 (m, 1H), 1.23 - 1.13 (m, 2H). **¹³C NMR** (126 MHz, DMSO-d6) δ 164.0, 163.6 (dd, J = 251.5, 12.2 Hz), 159.6, 159.0 (dd, J = 255.4, 12.9 Hz), 158.2, 137.8 (d, J = 9.7 Hz), 131.3, 130.5, 129.6 (dd, J = 10.3, 3.2 Hz), 128.1, 113.0 (dd, J = 22.2, 3.5 Hz), 111.5 (dd, J = 12.7, 4.1 Hz), 105.5 (dd, J = 26.0, 26.0 Hz), 102.5 (d, J = 8.5 Hz), 61.4, 52.8, 44.4, 35.5, 29.7. **HRMS** m/z [M+H]⁺ C₂₃H₂₃CIF₂N₃O₂⁺ calculated 446.1441, found 446.1461.

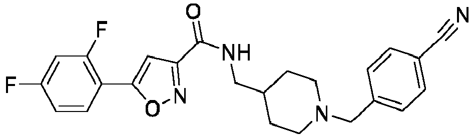

### N-((1-(4-cyanobenzyl)piperidin-4-yl)methyl)-5-(2,4-difluorophenyl)isoxazole-3-carboxamide (35)

This compound was synthesised according to general procedure C. 5-(2,4-difluorophenyl)-N-(piperidin-4-ylmethyl)isoxazole-3-carboxamide hydrochloride (100 mg, 0.279 mmol, 1.0 eq.), K₂CO₃ (77 mg, 0.56 mmol, 2.0 eq.) and 4-cyanobenzyl bromide (60 mg, 0.31 mmol, 1.1 eq.) were used. The crude product was purified by NP column chromatography (using a linear gradient of 10-50% (EtOAc:MeOH:TEA, 90:5:5, v:v:v%)/cyclohexane) to obtain the title compound as a white solid (66 mg, 54%). **LC/MS** (acidic mode) Rt 4.05 min, purity >97%, [M+H]⁺ calculated 437.18, found 437.15. **¹H NMR** (500 MHz, DMSO-d6) δ 8.88 (t, J = 6.0 Hz, 1H), 8.09 - 8.01 (m, 1H), 7.77 (d, J = 7.8 Hz, 2H), 7.61 - 7.53 (m, 1H), 7.49 (d, J = 7.9 Hz, 2H), 7.36 - 7.29 (m, 1H), 7.14 (d, J = 2.9 Hz, 1H), 3.52 (s, 2H), 3.16 (dd, J = 6.4, 6.4 Hz, 2H), 2.80 - 2.71 (m, 2H), 1.97 - 1.88 (m, 2H), 1.68 - 1.61 (m, 2H), 1.61 - 1.53 (m, 1H), 1.24 - 1.15 (m, 2H). **¹³C NMR** (126 MHz, DMSO-d6) δ 164.0, 163.6 (dd, J = 252.0, 12.7 Hz), 159.6, 159.0 (dd, J = 255.2, 12.7 Hz), 158.2, 145.0, 132.1, 129.6 (dd, J = 10.4, 3.4 Hz), 129.4, 119.0, 113.0 (d, J = 22.1 Hz), 111.5 (dd, J = 12.5, 3.9 Hz), 109.6, 105.5 (dd, J = 26.0, 26.0 Hz), 102.5 (d, J = 8.6 Hz), 61.7, 52.9, 44.4, 35.4, 29.7. **HRMS** m/z [M+H]⁺ C₂₄H₂₃F₂N₄O₂⁺ calculated 437.1784, found 437.1792.

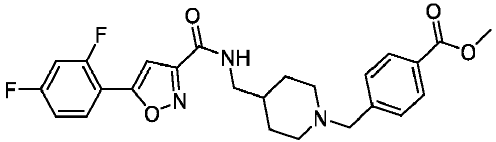

### Methyl 4-((4-((5-(2,4-difluorophenyl)isoxazole-3-carboxamido) methyl)piperidin-1-yl) methyl) benzoate (36)

This compound was synthesised according to general procedure C. 5-(2,4-difluorophenyl)-N-(piperidin-4-ylmethyl)isoxazole-3-carboxamide hydrochloride (100 mg, 0.279 mmol, 1.0 eq.), K₂CO₃ (77 mg, 0.56 mmol, 2.0 eq.) and methyl 4-(bromomethyl)benzoate (70 mg, 0.31 mmol, 1.1 eq.) were used. The crude product was purified by NP column chromatography (using a linear gradient of 10-50% (EtOAc:MeOH:TEA, 90:5:5, v:v:v%)/cyclohexane) to obtain the title compound as a white solid (92 mg, 70%). **LC/MS** (acidic mode) Rt 4.15 min, purity 98%, [M+H]⁺ calculated 470.19, found 470.15. **¹H NMR** (500 MHz, DMSO-d6) δ 8.88 (t, J = 6.0 Hz, 1H), 8.04 (ddd, J = 8.7, 8.7, 6.4 Hz, 1H), 7.93 - 7.88 (m, 2H), 7.57 (ddd, J = 11.6, 9.2, 2.6 Hz, 1H), 7.46 - 7.40 (m, 2H), 7.36 - 7.29 (m, 1H), 7.14 (d, J = 2.9 Hz, 1H), 3.83 (s, 3H), 3.51 (s, 2H), 3.16 (dd, J = 6.4 Hz, 6.4 Hz,2H), 2.81 - 2.72 (m, 2H), 1.96 - 1.87 (m, 2H), 1.67 - 1.61 (m, 2H), 1.61 - 1.52 (m, 1H), 1.25 - 1.14 (m, 2H). **¹³C NMR** (126 MHz, DMSO-d6) δ 166.2, 164.0 (d, J = 2.7 Hz), 163.6 (dd, J = 251.8, 12.5 Hz), 159.6, 159.0 (dd, J = 255.2, 12.7 Hz), 158.2, 144.7, 129.6 (dd, J = 10.3, 3.4 Hz), 129.1, 128.8, 128.2, 113.0 (dd, J = 22.1, 3.5 Hz), 111.5 (dd, J = 12.3, 3.6 Hz), 105.5 (dd, J = 25.9, 25.9 Hz), 102.5 (d, J = 8.5 Hz), 61.9, 53.0, 52.0, 44.4, 35.5, 29.7. **HRMS** m/z [M+H]⁺ C₂₅H₂₆F₂N₃O₄⁺ calculated 470.1886, found 470.1900.

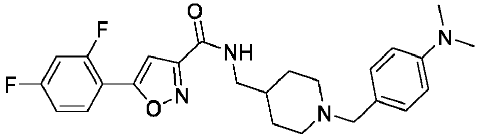

### 5-(2,4-difluorophenyl)-N-((1-(4-(dimethylamino)benzyl)piperidin-4-yl)methyl)isoxazole-3-carboxamide (37)

This compound was synthesised according to general procedure D. 5-(2,4-difluorophenyl)-N-(piperidin-4-ylmethyl)isoxazole-3-carboxamide hydrochloride (100 mg, 0.279 mmol, 1.0 eq.), TEA (39 µL, 0.28 mmol, 1.0 eq.), 4-(dimethylamino)benzaldehyde (50 mg, 0.34 mmol, 1.2 eq.) and STAB-H (148 mg, 0.699 mmol, 2.5 eq.) were used. Additional 4-(dimethylamino)benzaldehyde (25 mg, 0.17 mmol, 0.6 eq.) and STAB-H (36 mg, 0.17 mmol, 0.6 eq.) were added to drive the reaction to completion. The crude product was purified by NP column chromatography (using a linear gradient of 30-70% (EtOAc:MeOH:TEA, 90:5:5, v:v:v%)/cyclohexane) to obtain the title compound as a white solid (73 mg, 58%). **LC/MS** (acidic mode) Rt 4.27 min, purity 98%, [M+H]⁺ calculated 455.23, found 455.20. **¹H NMR** (500 MHz, DMSO-d6) δ 8.87 (t, J = 5.9 Hz, 1H), 8.05 (ddd, J = 8.7, 8.7, 6.4 Hz, 1H), 7.58 (ddd, J = 11.5, 9.2, 2.5 Hz, 1H), 7.32 (ddd, J = 8.6, 8.3, 2.1 Hz, 1H), 7.15 (d, J = 3.0 Hz, 1H), 7.07 (dd, J = 9.3, 2.6 Hz, 2H), 6.67 - 6.64 (m, 2H), 3.32 - 3.29 (m, 2H), 3.15 (dd, J = 6.4, 6.4 Hz, 2H), 2.85 (s, 6H), 2.80 - 2.73 (m, 2H), 1.86 - 1.77 (m, 2H), 1.65 - 1.58 (m, 2H), 1.58 - 1.49 (m, 1H), 1.20 - 1.09 (m, 2H). **¹³C NMR** (126 MHz, DMSO-d6) δ 164.0, 163.6 (dd, J = 251.6, 12.4 Hz), 159.6, 159.0 (dd, J = 255.1, 12.7 Hz), 158.2, 149.5, 129.6, 129.2, 125.9, 113.0 (dd, J = 22.1, 3.5 Hz), 112.2, 112.8 - 110.3 (m), 105.5 (dd, J = 26.0, 26.0 Hz), 102.5 (d, J = 8.2 Hz), 62.1, 52.7, 44.5, 40.3, 35.6, 29.7. **HRMS** m/z [M+H-*p*-(NMe₂)Bn]⁺ C₁₆H₁₈F₂N₃O₂⁺ calculated 322.1362, found 322.1382.

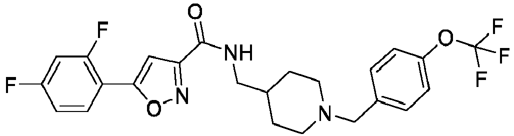

### 5-(2,4-difluorophenyl)-N-((1-(4-(trifluoromethoxy)benzyl)piperidin-4-yl)methyl)isoxazole-3-carboxamide(38)

This compound was synthesised according to general procedure D. 5-(2,4-difluorophenyl)-N-(piperidin-4-ylmethyl)isoxazole-3-carboxamide hydrochloride (100 mg, 0.279 mmol, 1.0 eq.), TEA (39 µL, 0.28 mmol, 1.0 eq.), 4-(trifluoromethoxy)benzaldehyde (64 mg, 0.34 mmol, 1.2 eq.) and STAB-H (148 mg, 0.699 mmol, 2.5 eq.) were used. For this compound no column chromatography was necessary. The crude product was purified by trituration with MeOH to obtain the title compound as a white solid (66 mg, 48%). **LC/MS** (acidic mode) Rt 4.60 min, purity 98%, [M+H]⁺ calculated 496.17, found 496.20. **¹H NMR** (500 MHz, CDCl₃)δ 7.94 (ddd, J = 8.5, 8.5, 6.1 Hz, 1H), 7.40 - 7.34 (m, 2H), 7.18 - 7.14 (m, 2H), 7.09 (d, J = 3.8 Hz, 1H), 7.07 - 7.01 (m, 1H), 7.01 - 6.95 (m, 1H), 6.95 - 6.89 (m, 1H), 3.54 (s, 2H), 3.38 (dd, J = 6.5, 6.5 Hz, 2H), 3.00 - 2.87 (m, 2H), 2.04 (s, 2H), 1.82 - 1.73 (m, 2H), 1.73 - 1.62 (m, 1H), 1.50 - 1.38 (m, 2H). **¹³C NMR** (126 MHz, CDCl₃) δ 164.9, 164.3 (dd, J = 255.5, 12.3 Hz), 159.5, 159.0, 130.7, 128.9 (dd, J = 10.0, 3.6 Hz), 120.9, 112.6 (dd, J = 22.0, 3.4 Hz), 112.1 (dd, J = 12.8, 4.2 Hz), 105.2 (dd, J = 25.2, 25.2 Hz), 102.7, 62.3, 53.2, 45.0, 35.9, 29.7. *Four quaternary carbon signals are missing in this spectrum.* **HRMS** m/z [M+H]⁺ C₂₄H₂₃F₃N₃O₃⁺ calculated 496.1654, found 496.1678.

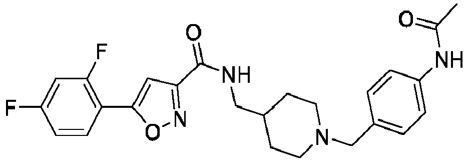

### N-((1-(4-acetamidobenzyl)piperidin-4-yl)methyl)-5-(2,4-difluorophenyl)isoxazole-3-carboxamide (39)

This compound was synthesised according to general procedure D. 5-(2,4-difluorophenyl)-N-(piperidin-4-ylmethyl)isoxazole-3-carboxamide hydrochloride (100 mg, 0.279 mmol, 1.0 eq.), TEA (39 µL, 0.28 mmol, 1.0 eq.), 4-acetamidobenzaldehyde (55 mg, 0.34 mmol, 1.2 eq.) and STAB-H (148 mg, 0.699 mmol, 2.5 eq.) were used. Additional 4-acetamidobenzaldehyde (55 mg, 0.34 mmol, 1.2 eq.) and STAB-H (148 mg, 0.699 mmol, 2.5 eq.) were added to drive the reaction to completion. The crude product was purified by trituration in MeOH to obtain the title compound as a white solid (39 mg, 30%). **LC/MS** (acidic mode) Rt 3.53 min, purity 98%, [M+H]⁺ calculated 469.20, found 469.25. **¹H NMR** (500 MHz, DMSO-d6) δ 9.88 (s, 1H), 8.87 (t, J = 5.9 Hz, 1H), 8.09 - 8.00 (m, 1H), 7.62 - 7.54 (m, 1H), 7.50 (d, J = 8.0 Hz, 2H), 7.37 - 7.28 (m, 1H), 7.21- 7.12 (m, 3H), 3.35 (s, 2H), 3.15 (dd, J = 6.4, 6.4 Hz, 2H), 2.81 - 2.72 (m, 2H), 2.02 (s, 3H), 1.90 - 1.80 (m, 2H), 1.67 - 1.59 (m, 2H), 1.59 - 1.50 (m, 1H), 1.23 - 1.11 (m, 2H). **¹³C NMR** (126 MHz, DMSO-d6) δ 168.1, 164.0, 163.6 (dd, J = 251.9, 12.5 Hz), 159.6, 159.0 (dd, J = 255.2, 12.8 Hz), 158.2, 138.0, 133.1, 129.6 (dd, J = 10.3, 3.4 Hz), 129.0, 118.7, 113.0 (dd, J = 22.1, 3.3 Hz), 111.5 (dd, J = 12.3, 3.8 Hz), 105.5 (dd, J = 25.9, 25.9 Hz), 102.5 (d, J = 8.5 Hz), 62.0, 52.8, 44.4, 35.6, 29.7, 23.9. **HRMS** m/z [M+H]⁺ C₂₅H₂₇F₂N₄O₃⁺ calculated 469.2046, found 469.2066.

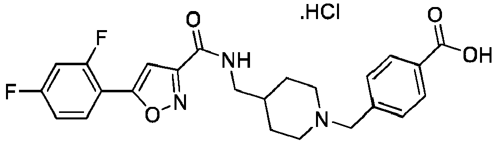

### 4-((4-((5-(2,4-difluorophenyl)isoxazole-3-carboxamido)methyl)piperidin-1-yl)methyl)benzoic acid hydrochloride (40)

5-(2,4-difluorophenyl)-N-(piperidin-4-ylmethyl)isoxazole-3-carboxamide hydrochloride (100 mg, 0.279 mmol, 1.0 eq.) was suspended in THF (2.8 mL) that contained TEA (39 µL, 0.28 mmol, 1.0 eq.) and 4-formylbenzaldehyde (50 mg, 0.34 mmol, 1.2 eq.). The mixture was stirred at RT for 10 min. Subsequently, STAB-H (148 mg, 0.699 mmol, 2.5 eq.) was added and the reaction mixture was stirred overnight at RT. Additional 4-formylbenzoic acid (55 mg, 0.34 mmol, 1.2 eq.) and STAB-H (148 mg, 0.699 mmol, 2.5 eq.) were added to drive the reaction to completion. MeOH was added to terminate the reaction and the volatiles were removed by *in vacuo.* The resulting solids were mixed with an aqueous solution of NaOH (2M) and extracted with DCM (3 times). The combined organic layers were dried over anhydrous Na₂SO₄, filtered and concentrated *in vacuo.* The resulting residue was purified using RP column chromatography (using a linear gradient of 10-70% ACN/H₂O with 0.1% FA). The product fractions were combined and an excess of an aqueous solution of HCl (3M) was added. The resulting mixture was lyophilised to obtain the title compound as a white solid (20 mg, 16%). **LC/MS** (acidic mode) Rt 3.16 min, purity 97%, [M+H]⁺ calculated 456.17, found 456.20. **¹H NMR** (500 MHz, DMSO-d6) δ 13.15 (s, 1H), 10.79 - 10.49 (m, 1H), 9.09 - 8.99 (m, 1H), 8.08 - 8.02 (m, 1H), 7.99 (d, J = 8.0 Hz, 2H), 7.72 (d, J = 8.0 Hz, 2H), 7.58 (ddd, J = 11.4, 9.1, 2.5 Hz, 1H), 7.33 (ddd, J = 8.5, 8.5, 2.5 Hz, 1H), 7.16 (d, J = 3.0 Hz, 1H), 4.33 (d, J = 4.6 Hz, 2H), 3.33 - 3.29 (m, 2H), 3.17 (dd, J = 6.3, 6.3 Hz, 2H), 2.96 - 2.85 (m, 2H), 1.88 - 1.77 (m, 3H), 1.60 - 1.48 (m, 2H). **¹³C NMR** (126 MHz, DMSO-d6) δ 166.9, 164.1 (d, J = 2.5 Hz), 163.7 (dd, J = 252.3, 12.4 Hz), 159.5, 159.0 (dd, J = 255.2, 12.8 Hz), 158.4, 134.5, 131.7, 129.9 - 129.4 (m), 129.5, 113.0 (dd, J = 22.2, 3.4 Hz), 111.5 (dd, J = 12.4, 3.8 Hz), 105.5 (dd, J = 25.9, 25.9 Hz), 102.5 (d, J = 8.5 Hz), 58.5, 51.3, 43.6, 33.5, 26.6. One carbon signal could not be detected even after 2D NMR analysis. **HRMS** m/z [M+H]⁺ C₂₄H₂₄F₂N₃O₄⁺ calculated 456.1729, found 456.1735.

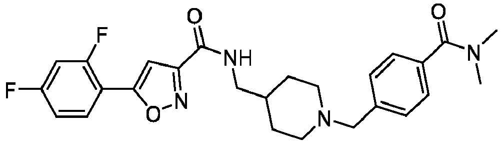

### 5-(2,4-difluorophenyl)-N-((1-(4-(dimethylcarbamoyl)benzyl)piperidin-4-yl)methyl)isoxazole-3-carboxamide (41)

This compound was synthesised according to the general procedure D. 5-(2,4-difluorophenyl)-N-(piperidin-4-ylmethyl)isoxazole-3-carboxamide hydrochloride (100 mg, 0.279 mmol, 1.0 eq.), TEA (39 µL, 0.28 mmol, 1.0 eq.), 4-formyl-N,N-dimethylbenzamide (59 mg, 0.34 mmol, 1.2 eq.) and STAB-H (71 mg, 0.34 mmol, 1.2 eq.) were used. Additional 4-formyl-N,N-dimethylbenzamide (59 mg, 0.34 mmol, 1.2 eq.) and STAB-H (142 mg, 0.671 mmol, 2.4 eq.) were added to drive the reaction to completion. The crude product was purified by NP column chromatography (using a linear gradient of 10-45% (EtOAc:MeOH:TEA, 90:5:5, v:v:v%)/cyclohexane) to obtain the title compound as an off-white solid (28 mg, 21%). **LC/MS** (acidic mode) Rt 3.13 min, purity 95%, [M+H]⁺ calculated 483.22, found 483.30. **¹H NMR** (500 MHz, DMSO-d6) δ 8.89 (t, J = 5.9 Hz, 1H), 8.10 - 8.00 (m, 1H), 7.61 - 7.54 (m, 1H), 7.39 - 7.29 (m, 5H), 7.15 (d, J = 2.9 Hz, 1H), 3.46 (s, 2H), 3.16 (dd, J = 6.4, 6.4 Hz, 2H), 2.96 (s, 3H), 2.90 (s, 3H), 2.79 (d, J = 11.1 Hz, 2H), 1.95 - 1.85 (m, 2H), 1.69 - 1.60 (m, 2H), 1.60 - 1.52 (m, 1H), 1.25 - 1.14 (m, 2H). **¹³C NMR** (126 MHz, DMSO-d6) δ 170.1, 164.0, 163.6 (dd, J = 251.8, 12.5 Hz), 159.6, 159.0 (dd, J = 255.2, 12.4 Hz), 158.2, 140.1, 134.9, 129.6 (dd, J = 10.3, 3.3 Hz), 128.4, 126.9, 113.0 (dd, J = 21.9, 3.5 Hz), 112.3 - 110.7 (m), 105.5 (dd, J = 25.9, 25.9 Hz), 102.5 (d, J = 8.7 Hz), 62.0, 52.9, 44.4, 38.5, 35.5, 29.7. Rotamers are observed. **HRMS** m/z [M+H]⁺ C₂₆H₂₉F₂N₄O₃⁺ calculated 483.2202, found 483.2221.

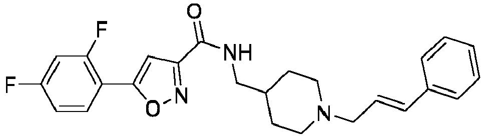

### N-((1-cinnamylpiperidin-4-yl)methyl)-5-(2,4-difluorophenyl)isoxazole-3-carboxamide (42)

5-(2,4-difluorophenyl)-N-(piperidin-4-ylmethyl)isoxazole-3-carboxamide hydrochloride (100 mg, 0.279 mmol, 1.0 eq.) was suspended in THF (2.8 mL) that contained TEA (39 µL, 0.28 mmol, 1.0 eq.), and cinnamaldehyde (42 µL, 0.34 mmol, 1.2 eq.). After stirring at RT for 10 min, STAB-H (148 mg, 0.699 mmol, 2.5 eq.) was added and the reaction mixture was stirred overnight. MeOH was added to terminate the reaction and the volatiles were removed by *in vacuo.* The resulting solids were suspended in an aqueous solution of NaOH (2M) and extracted with DCM (3 times). The combined organic layers were dried over anhydrous Na₂SO₄, filtered and concentrated *in vacuo.* The crude product was purified by NP column chromatography (using a linear gradient of 10-60% (EtOAc:MeOH:TEA, 90:5:5, v:v:v%)/cyclohexane) to obtain the title compound as an off-white solid (68 mg, 56%). LC/MS (acidic mode) Rt 3.94 min, purity >96%, [M+H]⁺ calculated 438.20, found 438.20. **¹H** NMR (500 MHz, DMSO-d6) δ 8.89 (t, J = 5.9 Hz, 1H), 8.05 (ddd, J = 8.7, 8.7, 6.4 Hz, 1H), 7.57 (ddd, J = 11.5, 9.2, 2.5 Hz, 1H), 7.47 - 7.39 (m, 2H), 7.36 - 7.28 (m, 3H), 7.26 - 7.20 (m, 1H), 7.15 (d, J = 3.0 Hz, 1H), 6.50 (d, J = 15.9 Hz, 1H), 6.29 (dt, J = 15.9, 6.5 Hz, 1H), 3.16 (dd, J = 6.4, 6.4 Hz, 2H), 3.09 - 3.02 (m, 2H), 2.90 - 2.82 (m, 2H), 1.93 - 1.84 (m, 2H), 1.81 - 1.57 (m, 2H), 1.60 - 1.51 (m, 1H), 1.25 - 1.13 (m, 2H). **¹³C** NMR (126 MHz, DMSO-d6) δ 164.0, 163.6 (dd, J = 251.9, 12.5 Hz), 159.6, 159.0 (dd, *J* = 255.2, 12.7 Hz), 158.2, 136.7, 131.6, 129.6 (dd, *J* = 10.3, 3.3 Hz), 128.6, 127.7, 127.3, 126.2, 113.0 (dd, *J* = 22.0, 3.5 Hz), 112.0 - 110.9 (m), 105.5 (dd, *J* = 26.0, 26.0 Hz), 102.5 (d, *J* = 8.3 Hz), 60.5, 53.0, 44.4, 35.6, 29.8. HRMS m/z [M+H]⁺ C₂₅H₂₆F₂N₃O₂⁺ calculated 438.1988, found 438.2007.

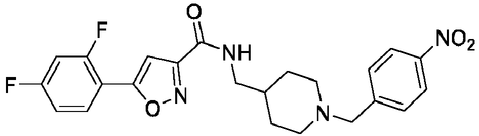

### 5-(2,4-difluorophenyl)-N-((1-(4-nitrobenzyl)piperidin-4-yl)methyl)isoxazole-3-carboxamide (43)

This compound was synthesised according to general procedure D. 5-(2,4-difluorophenyl)-N-(piperidin-4-ylmethyl)isoxazole-3-carboxamide hydrochloride (100 mg, 0.279 mmol, 1.0 eq.), TEA (39 µL, 0.28 mmol, 1.0 eq.), 4-nitrobenzaldehyde (51 mg, 0.34 mmol, 1.2 eq.) and STAB-H (148 mg, 0.699 mmol, 2.5 eq.) were used. Additional 4-nitrobenzaldehyde (25 mg, 0.17 mmol, 0.6 eq.) and STAB-H (71 mg, 0.34 mmol, 1.2 eq. each) were added drive the reaction to completion. The crude product was purified by NP column chromatography (using a linear gradient of 10-50% (EtOAc:MeOH:TEA, 90:5:5, v:v:v%)/cyclohexane). The obtained solid was triturated with MeOH to afford the title compound as a white solid (32 mg, 25%). LC/MS (acidic mode) Rt 3.41 min, purity 98%, [M+H]⁺ calculated 457.17, found 457.20. **¹H** NMR (500 MHz, DMSO-d6) δ 8.89 (t, J = 5.9 Hz, 1H), 8.21 - 8.16 (m, 2H), 8.05 (ddd, J = 8.7, 8.7, 6.4 Hz, 1H), 7.62 - 7.55 (m, 3H), 7.32 (ddd, J = 8.4, 8.4, 2.4 Hz, 1H), 7.15 (d, J = 2.9 Hz, 1H), 3.58 (s, 2H), 3.17 (dd, J = 6.4, 6.4 Hz, 2H), 2.82 - 2.74 (m, 2H), 2.00 - 1.91 (m, 2H), 1.68 - 1.61 (m, 2H), 1.61 - 1.53 (m, 1H), 1.26 - 1.15 (m, 2H). **¹³C** NMR (126 MHz, DMSO-d6) δ 164.5, 164.1 (dd, J = 251.9, 12.3 Hz), 160.1, 159.5 (dd, J = 255.3, 12.9 Hz), 158.7, 147.8, 147.0, 130.1 (dd, J = 10.4, 3.3 Hz), 130.0, 123.8, 113.5 (dd, J = 21.9, 3.5 Hz), 112.4 - 111.5 (m), 106.0 (dd, J = 25.9, 25.9 Hz), 102.9 (d, J = 8.5 Hz), 61.8, 53.4, 44.9, 35.9, 30.2. HRMS m/z [M+H]⁺ C₂₃H₂₃F₂N₄O₄⁺ calculated 457.1682, found 457.1702.

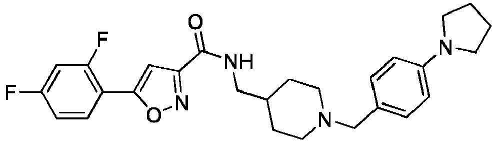

### 5-(2,4-difluorophenyl)-N-((1-(4-(pyrrolidin-1-yl)benzyl)piperidin-4-yl)methyl)isoxazole-3-carboxamide (44)

This compound was synthesised according to general procedure D. 5-(2,4-difluorophenyl)-N-(piperidin-4-ylmethyl)isoxazole-3-carboxamide hydrochloride (100 mg, 0.279 mmol, 1.0 eq.), TEA (39 µL, 0.28 mmol, 1.0 eq.), 4-(pyrrolidine-1-yl)benzaldehyde (59 mg, 0.34 mmol, 1.2 eq.) and STAB-H (148 mg, 0.699 mmol, 2.5 eq.) were used. The crude product was purified by NP column chromatography (using a linear gradient of 30-70% (EtOAc:MeOH:TEA, 90:5:5, v:v:v%)/cyclohexane) to obtain the title compound as a white solid (68 mg, 50%). **LC/MS** (acidic mode) Rt 3.76 min, purity >98%, [M+H]⁺ calculated 481.24, found 481.30. **¹H** NMR (600 MHz, DMSO-d6) δ 8.86 (t, J = 6.0 Hz, 1H), 8.05 (ddd, J = 8.7, 8.7, 6.3 Hz, 1H), 7.57 (ddd, J = 11.5, 9.2, 2.5 Hz, 1H), 7.32 (ddd, J = 8.4, 8.4, 2.5 Hz, 1H), 7.14 (d, J = 2.9 Hz, 1H), 7.06 - 7.03 (m, 2H), 6.50 - 6.41 (m, 2H), 3.29 (s, 2H), 3.21 - 3.16 (m, 4H), 3.15 (dd, J = 6.4, 6.4 Hz, 2H), 2.80 - 2.74 (m, 2H), 1.95 - 1.90 (m, 4H), 1.86 -1.79 (m, 2H), 1.72 -1.58 (m, 2H), 1.64 - 1.47 (m, 1H), 1.19 - 1.10 (m, 2H). **¹³C NMR** (151 MHz, DMSO-d6) δ 164.0 (d, J = 2.7 Hz), 163.6 (dd, J = 252.0, 12.4 Hz), 159.6, 159.0 (dd, J = 254.8, 13.2 Hz), 158.2, 146.8, 129.8, 129.6 (dd, J = 10.5, 3.3 Hz), 124.6, 113.0 (dd, J = 22.0, 3.3 Hz), 111.5 (dd, J = 12.4, 3.8 Hz), 111.2, 105.5 (dd, J = 26.0, 26.0 Hz), 102.5 (d, J = 8.3 Hz), 62.2, 52.7, 47.3, 44.5, 35.6, 29.7, 24.9. **HRMS** m/z [M+H-*p*-(pyrrolidine-1-yl)Bn]⁺ C₁₆H₁₈F₂N₃O₂⁺ calculated 322.1362, found 322.1379.

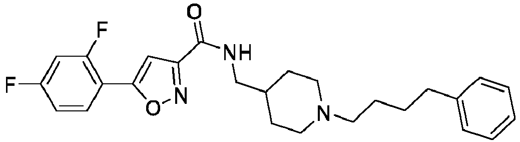

### 5-(2,4-difluorophenyl)-N-((1-(4-phenylbutyl)piperidin-4-yl)methyl)isoxazole-3-carboxamide (45)

This compound was synthesised according to general procedure C. 5-(2,4-difluorophenyl)-N-(piperidin-4-ylmethyl)isoxazole-3-carboxamide hydrochloride (100 mg, 0.279 mmol, 1.0 eq.), 1-chloro-4-phenylbutane (51 µL, 0.31 mmol, 1.1 eq.) and K₂CO₃ (77 mg, 0.56 mmol, 2.0 eq.) were used. The reaction mixture was stirred overnight at RT. Additional 1-chloro-4-phenylbutane was added (1.5 eq.) and the mixture was stirred for another 24 h. Subsequently, the reaction mixture was heated to 50°C and stirred at that temperature overnight. The volatiles were evaporated *in vacuo* and the residue was suspended in an aqueous solution of NaOH (2M). The formed suspension was extracted with DCM (three times). The combined organic layers were dried over anhydrous Na₂SO₄, filtered and concentrated *in vacuo.* The crude product was purified by NP column chromatography (using a linear gradient of 10-50% (EtOAc:MeOH:TEA, 90:5:5, v:v:v%)/cyclohexane) to obtain the title compound as an off-white solid (40 mg, 32%). **LC/MS** (acidic mode) Rt 3.73 min, purity 97%, [M+H]⁺ calculated 454.23, found 454.20. **¹H NMR** (500 MHz, DMSO-d6) δ 8.87 (t, J = 6.0 Hz, 1H), 8.05 (ddd, J = 8.7, 8.7, 6.2 Hz, 1H), 7.57 (ddd, J = 11.5, 9.2, 2.6 Hz, 1H), 7.32 (ddd, J = 8.5, 8.5, 2.6 Hz, 1H), 7.29 - 7.23 (m, 2H), 7.19 - 7.13 (m, 4H), 3.14 (dd, J = 6.4, 6.4 Hz, 2H), 2.83 - 2.75 (m, 2H), 2.60 - 2.52 (m, 2H), 2.27 - 2.19 (m, 2H), 1.82 - 1.74 (m, 2H), 1.65 - 1.58 (m, 2H), 1.58 - 1.50 (m, 3H), 1.44 - 1.36 (m, 2H), 1.19 - 1.08 (m, 2H). **¹³C NMR** (126 MHz, DMSO-d6) δ 164.0, 163.6 (dd, J = 252.1, 12.4 Hz), 159.6, 159.0 (dd, J = 255.4, 12.9 Hz), 158.2, 142.3, 129.6 (dd, J = 10.3, 3.4 Hz), 128.2, 128.2, 125.6, 113.0 (dd, J = 22.1, 3.5 Hz), 111.9 - 110.9 (m), 105.5 (dd, J = 26.0, 26.0 Hz), 102.4 (d, J = 8.4 Hz), 58.0, 53.1, 44.5, 35.7, 35.0, 29.8, 28.9, 26.1. **HRMS** m/z [M+H]⁺ C₂₆H₃₀F₂N₃O₂⁺ calculated 454.2301, found 454.2307.

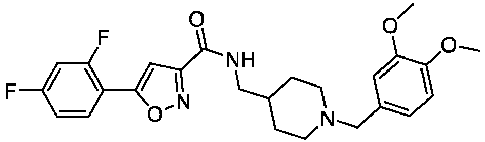

### 5-(2,4-difluorophenyl)-N-((1-(3,4-dimethoxybenzyl)piperidin-4-yl)methyl)isoxazole-3-carboxamide (46)

This compound was synthesised according to general procedure D. 5-(2,4-difluorophenyl)-N-(piperidin-4-ylmethyl)isoxazole-3-carboxamide hydrochloride (100 mg, 0.279 mmol, 1.0 eq.), TEA (39 µL, 0.28 mmol, 1.0 eq.), 3,4-dimethoxybenzaldehyde (56 mg, 0.34 mmol, 1.2 eq.) and STAB-H (148 mg, 0.699 mmol, 2.5 eq.) were used. The crude product was purified by NP column chromatography (using a linear gradient of 10-50% (EtOAc:MeOH:TEA, 90:5:5, v:v:v%)/cyclohexane) to obtain the title compound as a white solid (100 mg, 76%). **LC/MS** (acidic mode) Rt 3.41 min, purity 98%, [M+H]⁺ calculated 472.20, found 472.20. **¹H NMR** (500 MHz, DMSO-d6) δ 8.88 (t, J = 5.9 Hz, 1H), 8.05 (ddd, J = 8.7, 8.7, 6.4 Hz, 1H), 7.58 (ddd, J = 11.6, 9.2, 2.5 Hz, 1H), 7.35 - 7.29 (m, 1H), 7.15 (d, J = 2.9 Hz, 1H), 6.88 - 6.84 (m, 2H), 6.79 - 6.75 (m, 1H), 3.73 (s, 3H), 3.72 (s, 3H), 3.35 (s, 2H), 3.16 (dd, J = 6.4, 6.4 Hz, 2H), 2.81 - 2.76 (m, 2H), 1.89 - 1.81 (m, 2H), 1.66 - 1.60 (m, 2H), 1.60 - 1.51 (m, 1H), 1.23 - 1.12 (m, 2H). **¹³C NMR** (126 MHz, DMSO-d6) δ 164.0, 163.6 (dd, J = 252.1, 12.6 Hz), 159.6, 159.0 (dd, J = 255.2, 12.8 Hz), 158.2, 148.5, 147.7, 131.1, 129.6 (dd, J = 10.4, 3.3 Hz), 120.7, 113.0 (dd, J = 22.0, 3.5 Hz), 112.3, 111.5, 111.5, 105.5 (dd, J = 26.0, 26.0 Hz), 102.5 (d, J = 8.3 Hz), 62.2, 55.5, 55.4, 52.8, 44.4, 35.6, 29.7. **HRMS** m/z [M+H]⁺ C₂₅H₂₈F₂N₃O₄⁺ calculated 472.2042, found 472.2055.

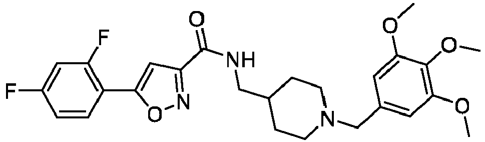

### 5-(2,4-difluorophenyl)-N-((1-(3,4,5-trimethoxybenzyl)piperidin-4-yl)methyl)isoxazole-3-carboxamide(47)

This compound was synthesised according to general procedure D. 5-(2,4-difluorophenyl)-N-(piperidin-4-ylmethyl)isoxazole-3-carboxamide hydrochloride (100 mg, 0.279 mmol, 1.0 eq.), TEA (39 µL, 0.28 mmol, 1.0 eq.), 3,4,5-trimethoxybenzaldehyde (66 mg, 0.34 mmol, 1.2 eq.) and STAB-H (148 mg, 0.699 mmol, 2.5 eq.) were used. The crude product was purified by NP column chromatography (using a linear gradient of 10-50% (EtOAc:MeOH:TEA, 90:5:5, v:v:v%)/cyclohexane) to obtain the title compound as a white solid (110 mg, 79%). **LC/MS** (acidic mode) Rt 3.51 min, purity >98%, [M+H]⁺ calculated 502.21, found 502.20. **¹H NMR** (500 MHz, DMSO-d6) δ 8.89 (t, J = 5.9 Hz, 1H), 8.05 (ddd, J = 8.7, 8.7, 6.3 Hz, 1H), 7.58 (ddd, J = 11.6, 9.2, 2.5 Hz, 1H), 7.35 - 7.30 (m, 1H), 7.15 (d, J = 3.0 Hz, 1H), 6.58 (s, 2H), 3.75 (s, 6H), 3.63 (s, 3H), 3.36 (s, 2H), 3.17 (dd, J = 6.4, 6.4 Hz, 2H), 2.83 - 2.78 (m, 2H), 1.91 - 1.83 (m, 2H), 1.68 - 1.60 (m, 2H), 1.60 - 1.51 (m, 1H), 1.25 - 1.13 (m, 2H). **¹³C NMR** (126 MHz, DMSO-d6) δ 164.0, 163.6 (dd, J = 252.0, 12.1 Hz), 159.6, 159.0 (dd, J = 255.3, 12.8 Hz), 158.2, 152.7, 136.2, 134.5, 129.6 (dd, J = 10.4, 3.4 Hz), 113.0 (dd, J = 22.0, 3.5 Hz), 112.9 - 110.3 (m), 105.5, 105.5 (dd, J = 26.0, 26.0 Hz), 102.5 (d, J = 8.4 Hz), 62.5, 59.9, 55.8, 52.9, 44.4, 35.6, 29.7. **HRMS** m/z [M+H]⁺ C₂₆H₃₀F₂N₃O₅⁺ calculated 502.2148, found 502.2166.

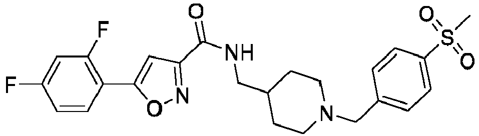

### 5-(2,4-difluorophenyl)-N-((1-(4-(methylsulfonyl)benzyl)piperidin-4-yl)methyl)isoxazole-3-carboxamide (48)

5-(2,4-difluorophenyl)-N-(piperidin-4-ylmethyl)isoxazole-3-carboxamide hydrochloride (100 mg, 0.279 mmol, 1.0 eq.) was suspended in DMF (2.8 mL). K₂CO₃ (77 mg, 0.56 mmol, 2.0 eq.) and 1-(chloromethyl)-4-(methylsulfonyl)benzene (63 mg, 0.31 mmol, 1.1 eq.) were added. The reaction mixture was stirred overnight at RT and then concentrated *in vacuo.* The resulting crude product was purified by NP column chromatography (using a linear gradient of 10-50% (EtOAc:MeOH:TEA, 90:5:5, v:v:v%)/cyclohexane) to obtain the title compound as a white solid (67 mg, 49%). **LC/MS** (acidic mode) Rt 3.22 min, purity >99%, [M+H]⁺ calculated 490.16, found 490.15. **¹H NMR** (500 MHz, DMSO-d6) δ 8.89 (t, J = 6.0 Hz, 1H), 8.05 (ddd, J = 8.7, 8.7, 6.4 Hz, 1H), 7.90 - 7.83 (m, 2H), 7.61

- 7.52 (m, 3H), 7.37 - 7.29 (m, 1H), 7.15 (d, J = 2.9 Hz, 1H), 3.55 (s, 2H), 3.20 (s, 3H), 3.17 (dd, J = 6.4, 6.4 Hz, 2H), 2.82 - 2.74 (m, 2H), 1.98 - 1.90 (m, 2H), 1.68 - 1.62 (m, 2H), 1.62 - 1.53 (m, 1H), 1.26 - 1.15 (m, 2H). **¹³C NMR** (126 MHz, DMSO-d6) δ 164.0, 163.6 (dd, J = 252.0, 12.5 Hz), 159.6, 159.0 (dd, J = 255.3, 12.8 Hz), 158.2, 145.2, 139.3, 129.6 (dd, J = 10.4, 3.3 Hz), 129.3, 126.9, 113.0 (dd, J = 21.9, 3.5 Hz), 111.5 (dd, J = 12.5, 3.8 Hz), 105.5 (dd, J = 25.9, 25.9 Hz), 102.5 (d, J = 8.5 Hz), 61.6, 52.9, 44.4, 43.6, 35.4, 29.7. **HRMS** m/z [M+H]⁺ C₂₄H₂₆F₂N₃O₄S⁺ calculated 490.1607, found 490.1618.

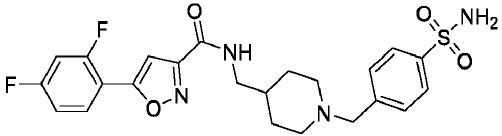

### 5-(2,4-difluorophenyl)-N-((1-(4-sulfamoylbenzyl)piperidin-4-yl)methyl)isoxazole-3-carboxamide (49)

5-(2,4-difluorophenyl)-N-(piperidin-4-ylmethyl)isoxazole-3-carboxamide hydrochloride (200 mg, 0.559 mmol, 1.0 eq.) was suspended in DMF (2.8 mL) and DIPEA (195 mg, 1.12 mmol, 2.0 eq.) and 4-(bromomethyl)benzenesulfonamide (70 mg, 0.61 mmol, 1.1 eq.) were added. The reaction mixture was stirred overnight at RT. The reaction mixture was poured into an ice-cold aqueous solution of NaOH (2M) and extracted with DCM (3 times). The combined organic layers were dried over anhydrous Na₂SO₄, filtered and concentrated *in vacuo.* The crude product was purified by RP column chromatography (using a linear gradient of 10-70% ACN/H₂O with 0.1% FA) to obtain the title compound as a white solid (127 mg, 46%). **LC/MS** (acidic mode) Rt 3.08 min, purity >97%, [M+H]⁺ calculated 491.16, found 491.25. **¹H NMR** (600 MHz, DMSO-d6) δ 8.87 (t, J = 5.9 Hz, 1H), 8.05 (ddd, J = 8.7, 8.6, 6.3 Hz, 1H), 7.79 - 7.74 (m, 2H), 7.57 (ddd, J = 11.5, 9.2, 2.6 Hz, 1H), 7.47 (d, J = 8.1 Hz, 2H), 7.35 - 7.26 (m, 3H), 7.15 (d, J = 2.9 Hz, 1H), 3.51 (s, 2H), 3.17 (dd, J = 6.4, 6.4 Hz, 2H), 2.83 - 2.72 (m, 2H), 1.97 - 1.89 (m, 2H), 1.69 - 1.61 (m, 2H), 1.61 - 1.53 (m, 1H), 1.24 - 1.15 (m, 2H). **¹³C NMR** (151 MHz, DMSO-d6) δ 164.0 (d, J = 2.7 Hz), 163.6 (dd, J = 252.0, 12.3 Hz), 159.6, 159.0 (dd, J = 255.3, 12.8 Hz), 158.2, 143.1, 142.6, 129.6 (dd, J = 10.2, 3.2 Hz), 128.9, 125.6, 113.0 (dd, J = 22.2, 3.5 Hz), 111.5 (dd, J = 12.4, 3.8 Hz), 105.5 (dd, J = 26.0, 26.0 Hz), 102.5 (d, J = 8.5 Hz), 61.7, 52.9, 44.4, 35.4, 29.7. **HRMS** m/z [M+H]⁺ C₂₃H₂₅F₂N₄O₄S⁺ calculated 491.1559, found 491.1578.

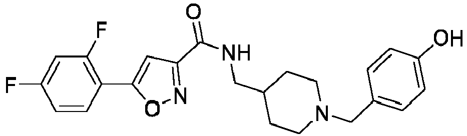

### 5-(2,4-difluorophenyl)-N-((1-(4-hydroxybenzyl)piperidin-4-yl)methyl)isoxazole-3-carboxamide (50)

5-(2,4-difluorophenyl)-N-(piperidin-4-ylmethyl)isoxazole-3-carboxamide hydrochloride (100 mg, 0.279 mmol, 1.0 eq.) was suspended in THF (2.8 mL) and TEA (39 µL, 0.28 mmol, 1.0 eq.) and 4-hydroxybenzaldehyde (41 mg, 0.34 mmol, 1.2 eq.) were added. The mixture was stirred at RT for 10 min. Subsequently, STAB-H (148 mg, 0.699 mmol, 2.5 eq.) was added and the reaction mixture was stirred overnight at RT. Additional 4-hydroxybenzaldehyde (20 mg, 0.17 mmol, 1.2 eq.) and STAB-H (71 mg, 0.34 mmol, 1.2 eq.) were added to drive the reaction to completion. MeOH was added to terminate the reaction and the volatiles were removed *in vacuo.* The crude product was purified by NP column chromatography (using a linear gradient of 10-50% (EtOAc:MeOH:TEA, 90:5:5, v:v:v%)/cyclohexane). The combined product fractions were triturated with MeOH to obtain the title compound as a white solid (67 mg, 49%). **LC/MS** (acidic mode) Rt 3.21 min, purity 98%, [M+H]⁺ calculated 428.18, found 428.20. **¹H NMR** (500 MHz, DMSO-d6) δ 9.29 - 9.17 (m, 1H), 8.94 - 8.78 (m, 1H), 8.11 - 7.99 (m, 1H), 7.64 - 7.51 (m, 1H), 7.38 - 7.27 (m, 1H), 7.14 (d, J = 3.2 Hz, 1H), 7.09 - 7.01 (m, 2H), 6.74 - 6.62 (m, 2H), 3.29 (s, 2H), 3.21 - 3.10 (m, 2H), 2.81 - 2.72 (m, 2H), 1.82 (t, J = 11.5 Hz, 2H), 1.66 - 1.58 (m, 2H), 1.58 - 1.50 (m, 1H), 1.22 - 1.08 (m, 2H). **¹³C NMR** (126 MHz, DMSO-d6) δ 164.0, 163.6 (dd, J = 251.7, 12.2 Hz), 159.6, 159.0 (dd, J = 255.2, 12.7 Hz), 158.2, 156.2, 129.9, 129.6 (dd, J = 10.4, 2.8 Hz), 128.6, 114.8, 113.0 (dd, J = 22.3, 2.6 Hz), 111.5 (dd, J = 12.4, 3.6 Hz), 105.5 (dd, J = 26.0, 26.0 Hz), 102.5 (d, J = 8.5 Hz), 62.0, 52.7, 44.4, 35.6, 29.7. **HRMS** m/z [M+H]⁺ C₂₃H₂₄F₂N₃O₃⁺ calculated 428.1780, found 428.1784.

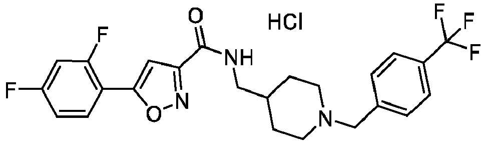

### 5-(2,4-difluorophenyl)-N-((1-(4-(trifluoromethyl)benzyl)piperidin-4-yl)methyl)isoxazole-3-carboxamide hydrochloride(51)

5-(2,4-difluorophenyl)-N-(piperidin-4-ylmethyl)isoxazole-3-carboxamide hydrochloride (200 mg, 0.559 mmol, 1.0 eq.) was suspended in THF (2.8 mL) and TEA (78 µL, 0.56 mmol, 1.0 eq.) and 4-(trifluoromethyl)benzaldehyde (117 mg, 0.671 mmol, 1.2 eq.) were added. The mixture was stirred at RT for 10 min. Subsequently, STAB-H (296 mg, 1.40 mmol, 2.5 eq.) was added and the reaction mixture was stirred overnight at RT. MeOH was added to terminate the reaction and the volatiles were removed *in vacuo.* The resulting solid were suspended in an ice-cold aqueous solution of NaOH (2M). The solids were collected by vacuum filtration and dissolved in MeOH. An excess of 4M HCI in dioxane was added. The volatiles were removed *in vacuo* to obtain the HCI salt. The resulting salt was triturated with acetone to obtain the title compound as a yellow solid (100 mg, 35%). **LC/MS** (acidic mode) Rt 3.63 min, purity >99%, [M+H]⁺ calculated 480.17, found 480.20. **¹H NMR** (500 MHz, DMSO-d6) δ 11.17 - 10.89 (m, 1H), 9.03 (t, J = 6.0 Hz, 1H), 8.05 (ddd, J = 8.7, 8.6, 6.3 Hz, 1H), 7.90 - 7.80 (m, 4H), 7.58 (ddd, J = 11.5, 9.2, 2.5 Hz, 1H), 7.38 - 7.29 (m, 1H), 7.20 - 7.14 (m, 1H), 4.42 (d, J = 5.5 Hz, 2H), 3.34 - 3.28 (m, 2H), 3.17 (dd, J = 6.2, 6.2 Hz, 2H), 2.96 - 2.84 (m, 2H), 1.88 - 1.77 (m, 3H), 1.66 - 1.53 (m, 2H). Cis-trans mixture observed. **¹³C NMR** (126 MHz, DMSO-d6) δ 164.6, 164.1 (dd, J = 252.2, 12.5 Hz), 160.0, 161.3 - 157.5 (m), 158.8, 135.0, 132.8, 130.3, 130.1 (dd, J = 10.3, 3.5 Hz), 126.0 (d, J = 4.2 Hz), 124.5 (q, J = 272.3 Hz), 113.5 (dd, J = 22.1, 3.4 Hz), 111.9 (dd, J = 12.4, 3.8 Hz), 106.0 (dd, J = 26.0, 26.0 Hz), 103.0 (d, J = 8.2 Hz), 58.6, 51.8, 44.1, 34.0, 27.0. **HRMS** m/z [M+H]⁺ C₂₄H₂₃F₅N₃O₂⁺ calculated 480.1705, found 480.1710.

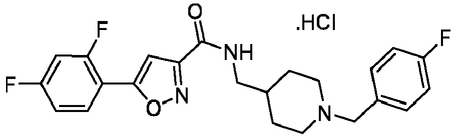

### 5-(2,4-difluorophenyl)-N-((1-(4-fluorobenzyl)piperidin-4-yl)methyl)isoxazole-3-carboxamide hydrochloride (52)

5-(2,4-difluorophenyl)-N-(piperidin-4-ylmethyl)isoxazole-3-carboxamide hydrochloride (200 mg, 0.559 mmol, 1.0 eq.) was suspended in THF (2.8 mL) and TEA (78 µL, 0.56 mmol, 1.0 eq.) and 4-fluorobenzaldehyde (83 mg, 0.67 mmol, 1.2 eq.) were added. The mixture was stirred at RT for 10 min. Subsequently, STAB-H (296 mg, 1.40 mmol, 2.5 eq.) was added and the reaction mixture was stirred overnight at RT. MeOH was added to terminate the reaction and the volatiles were removed *in vacuo.* The resulting solid were suspended in an ice-cold aqueous solution of NaOH (2M). The solids were collected by vacuum filtration and dissolved in MeOH. An excess of 4M HCI in dioxane was added. The volatiles were removed *in vacuo* to obtain the HCI salt. The resulting salt was triturated with acetone to obtain the title compound as a yellow solid (170 mg, 65%). **LC/MS** (acidic mode) Rt 3.44 min, purity 95%, [M+H]⁺ calculated 430.17, found 430.15. **¹H NMR** (500 MHz, DMSO-d6) δ 10.83 - 10.53 (m, 1H), 9.09 - 8.97 (m, 1H), 8.05 (ddd, J = 8.7, 8.6, 6.3 Hz, 1H), 7.73 - 7.62 (m, 2H), 7.58 (ddd, J = 11.5, 9.2, 2.5 Hz, 1H), 7.36 - 7.25 (m, 3H), 7.20 - 7.13 (m, 1H), 4.24 (d, J = 5.5 Hz, 2H), 3.33 - 3.26 (m, 2H), 3.17 (dd, J = 6.1, 6.1 Hz, 2H), 2.92 - 2.78 (m, 2H), 1.93 - 1.77 (m, 3H), 1.63 - 1.49 (m, 2H). Cis-trans mixture observed. **¹³C NMR** (126 MHz, DMSO-d6) δ 164.1, 163.7 (dd, J = 252.0, 12.3 Hz), 162.7 (d, J = 246.3 Hz), 159.5, 159.0 (dd, J = 255.4, 12.8 Hz), 158.3, 133.8 (d, J = 8.6 Hz), 129.6 (dd, J = 10.4, 3.3 Hz), 126.2 (d, J = 3.2 Hz), 115.6 (d, J = 21.7 Hz), 113.0 (dd, J = 22.2, 3.6 Hz), 111.4 (dd, J = 12.5, 3.7 Hz), 105.5 (dd, J = 25.9, 25.9 Hz), 102.5 (d, J = 8.4 Hz), 58.0, 51.0, 43.6, 33.5, 26.5. **HRMS** m/z [M+H]⁺ C₂₃H₂₃F₃N₃O₂⁺ calculated 430.1737, found 430.1744.

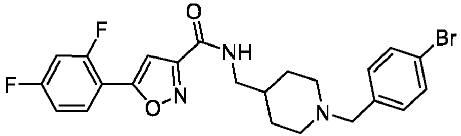

### N-((1-(4-bromobenzyl)piperidin-4-yl)methyl)-5-(2,4-difluorophenyl)isoxazole-3-carboxamide (53)

This compound was synthesised according to general procedure C. 5-(2,4-difluorophenyl)-N-(piperidin-4-ylmethyl)isoxazole-3-carboxamide hydrochloride (200 mg, 0.559 mmol, 1.0 eq.), K₂CO₃ (155 mg, 1.12 mmol, 2.0 eq.) and 4-bromobenzyl bromide (154 mg, 0.615 mmol, 1.1 eq.) were used. The crude product was purified by NP column chromatography (using a linear gradient of 10-50% (EtOAc:MeOH:TEA, 90:5:5, v:v:v%)/cyclohexane) to obtain the title compound as a white solid (100 mg, 56%). **LC/MS** (acidic mode) Rt 3.57 min, purity 99%, [M+H]⁺ calculated 490.09; 492.09, found 490.10; 492.15. **¹H NMR** (500 MHz, DMSO-d6) δ 8.88 (t, J = 6.0 Hz, 1H), 8.05 (dd, J = 8.7, 8.7, 6.4 Hz, 1H), 7.57 (ddd, J = 11.5, 9.3, 2.5 Hz, 1H), 7.52 - 7.47 (m, 2H), 7.33 (ddd, J = 8.6, 8.5, 2.5 Hz, 1H), 7.27 - 7.22 (m, 2H), 7.15 (d, J = 2.9 Hz, 1H), 3.40 (s, 2H), 3.16 (dd, J = 6.4, 6.4 Hz, 2H), 2.80 - 2.72 (m, 2H), 1.93 - 1.84 (m, 2H), 1.66 - 1.60 (m, 2H), 1.60 - 1.52 (m, 1H), 1.24 - 1.12 (m, 2H). **¹³C NMR** (126 MHz, DMSO-d6) δ 164.0, 163.6 (dd, J = 252.1, 12.2 Hz), 159.6, 159.0 (dd, J = 255.3, 13.0 Hz), 158.2, 138.3, 131.0, 130.8, 129.6 (dd, J = 10.3, 3.3 Hz), 119.7, 113.0 (dd, J = 22.2, 3.5 Hz), 111.8 - 111.4 (m), 105.5 (dd, J = 25.8, 25.8 Hz), 102.5 (d, J = 8.4 Hz), 61.5, 52.8, 44.4, 35.5, 29.7. **HRMS** m/z [M+H]⁺ C₂₃H₂₃BrF₂N₃O₂⁺ calculated 490.0936; 492.0916, found 490.0957; 492.0948.

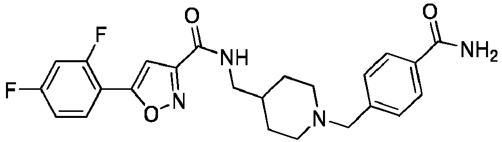

### N-((1-(4-carbamoylbenzyl)piperidin-4-yl)methyl)-5-(2,4-difluorophenyl)isoxazole-3-carboxamide (54)

5-(2,4-difluorophenyl)-N-(piperidin-4-ylmethyl)isoxazole-3-carboxamide hydrochloride (200 mg, 0.559 mmol, 1.0 eq.) was suspended in DMF (5.6 mL) and DIPEA (195 µL, 1.12 mmol, 2.0 eq.) and 4-(chloromethyl)benzamide (104 mg, 0.615 mmol, 1.1 eq.) were added. The reaction mixture was stirred overnight at RT. Subsequently, the reaction mixture was poured into an ice-cold aqueous solution of NaOH (2M). The resulting aqueous phase was extracted thrice with DCM. The DCM was dried over anhydrous Na₂SO₄, filtered and concentrated *in vacuo* to obtain the crude product. The crude product was purified by RP column chromatography (using a linear gradient of 10-70% ACN/H₂O with 0.1% FA). The product fractions were combined, basified with an aqueous solution of NaOH (2M) and extracted three times with DCM. The combined DCM layers were dried over anhydrous Na₂SO₄, filtered and concentrated *in vacuo* to obtain the title compound as a white solid (130 mg, 51%). **LC/MS** (acidic mode) Rt 2.96 min, purity >96%, [M+H]⁺ calculated 455.19, found 455.25. **¹H NMR** (600 MHz, DMSO-d6) δ 10.68 (s, 1H), 9.01 (t, J = 6.0 Hz, 1H), 8.08 - 8.01 (m, 2H), 7.93 (d, J = 7.8 Hz, 2H), 7.68 (d, J = 8.3 Hz, 2H), 7.57 (ddd, J = 11.5, 9.2, 2.5 Hz, 1H), 7.46 (s, 1H), 7.33 (ddd, J = 8.5, 8.5, 2.6 Hz, 1H), 7.16 (d, J = 2.9 Hz, 1H), 4.29 (d, J = 5.2 Hz, 2H), 3.34 - 3.27 (m, 2H), 3.17 (dd, J = 6.2, 6.2 Hz, 2H), 2.95 - 2.83 (m, 2H), 1.94 - 1.68 (m, 3H), 1.65 - 1.50 (m, 2H). Cis-trans mixture observed **¹³C NMR** (151 MHz, DMSO-d6) δ 167.3, 164.1 (d, J = 2.8 Hz), 163.7 (dd, J = 252.3, 12.4 Hz), 159.5, 159.0 (dd, J = 255.3, 12.9 Hz), 158.4, 135.0, 132.9, 131.3, 129.6 (dd, J = 10.4, 3.3 Hz), 127.8, 113.0 (dd, J = 22.2, 3.4 Hz), 111.4 (dd, J = 12.7, 3.9 Hz), 105.5 (dd, J = 26.0, 26.0 Hz), 102.5 (d, J = 8.2 Hz), 58.5, 51.3, 43.6, 33.5, 26.6. **HRMS** m/z [M+H]⁺ C₂₄H₂₅F₂N₄O₃⁺ calculated 455.1889, found 455.1910.

### Appendix 1 Pharmacological data

Rating *p*Kᵢ < 5 = + *p*Kᵢ 5-6.0 = ++, *p*Kᵢ 6.0-7.0 = +++, *p*Kᵢ≥7.0 = ++++

| **Molecule #** | **Affinity** |
|---|---|
| 1 | ++++ |
| 2 | + |
| 7 | + |
| 8 | + |
| 9 | + |
| 12 | ++ |
| 14 | + |
| 15 | +++ |
| 16 | +++ |
| 17 | +++ |
| 18 | +++ |
| 19 | +++ |
| 20 | +++ |
| 22 | ++ |
| 23 | +++ |
| 24 | ++++ |
| 25 | ++++ |
| 26 | ++++ |
| 27 | ++++ |
| 28 | +++ |
| 29 | +++ |
| 30 | ++ |
| 31 | ++ |
| 32 | + |
| 33 | ++++ |
| 34 | ++++ |
| 35 | + |
| 36 | ++++ |
| 37 | ++++ |
| 38 | + |
| 39 | ++++ |
| 40 | ++ |
| 41 | ++ |
| 42 | ++++ |
| 43 | + |
| 44 | ++++ |
| 45 | ++++ |
| 46 | ++++ |
| 47 | +++ |
| 48 | ++ |
| 49 | +++ |
| 50 | ++++ |
| 51 | + |
| 52 | +++ |
| 53 | +++ |

## Claims

1. A compound of formula (I): 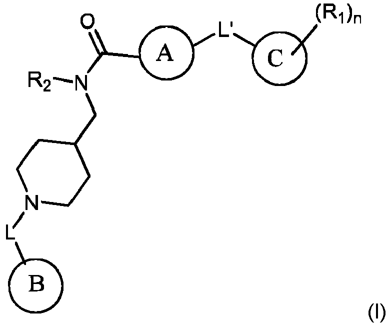 Wherein
Each R₁ is independently selected from halogen;
n is an integer between 0 and 5, preferably between 1 and 5;
Ring A is an heteroaryl having 5 ring atoms;
L' is a bond or a divalent linker selected from the group consisting of C₁-C₆ alkylene, -O-, and C₁-C₆ heteroalkylene;
Ring C is a ring selected from the group consisting of C₃-C₁₂ cycloalkyl, heterocyclyl having 5 to 10 ring atoms, aryl having 6 to 10 ring atoms, and heteroaryl having 5 to 10 ring atoms;
R₂ is selected from H, C₁-C₆ alkyl, C₃-C₆ cycloalkyl, heterocyclyl having 5 to 10 ring atoms, aryl having 6 to 10 ring atoms, and heteroaryl having 5 to 10 ring atoms; said alkyl, cycloalkyl, heterocyclyl, aryl and heteroaryl being optionally substituted with one or more substituents independently selected from oxo, halogen, -OH, -NO₂, -CN, and C₁-C₆ alkyl, preferably R2 is H;
L is a bond or a divalent linker selected from the group consisting of -(CR₃R₄)ₚ- p being an integer between 1 and 12, C₁-C₁₂ heteroalkylene, C₃-C₆ cycloalkylene, C₂-C₁₂ alkenylene, C₂-C₁₂ alkynylene, -O-, -NR'-, -S-, -SO-, and -SO2-, said heteroalkylene, alkenylene, and alkynylene being optionally interspersed with one or more groups selected from -(C₃-C₆ cycloalkyl)-, and said alkylene, heteroalkylene, cycloalkylene, alkenylene, and alkynylene being optionally substituted with one or more substituents independently selected from oxo, halogen, -OH, -NO₂, -CN, C₁-C₆ alkyl, C₃-C₆ cycloalkyl, heterocyclyl having 5 to 10 ring atoms, aryl having 6 to 10 ring atoms, heteroaryl having 5 to 10 ring atoms, C₁-C₆ alkoxy, C₁-C₆ haloalkyl, C₁-C₆ haloalkoxy, -(CO)-R', -O-(CO)-R', -(CO)-OR', -(CO)-NR"R‴, -NR"-(CO)-R', and -NR"R‴;
Ring B is a ring selected from the group consisting of C₃-C₁₂ cycloalkyl, heterocyclyl having 5 to 10 ring atoms, aryl having 6 to 10 ring atoms, and heteroaryl having 5 to 10 ring atoms, said cycloalkyl, heterocyclyl, aryl and heteroaryl being optionally substituted with one or more substituents independently selected from oxo, halogen, -OH, -NO₂, -CN, C₁-C₆ alkyl, C₃-C₆ cycloalkyl, heterocyclyl having 5 to 10 ring atoms, aryl having 6 to 10 ring atoms, heteroaryl having 5 to 10 ring atoms, C₁-C₆ alkoxy, C₁-C₆ haloalkyl, C₁-C₆ haloalkoxy, -(CO)-R', -O-(CO)-R', -(CO)-O-R', -(CO)-NR"R‴, -NR"-(CO)-R', -NR"R‴, -S(O)₂-R', and S(O)₂-NR"R‴;
R₃ and R₄ are independently selected from H, C₁-C₆ alkyl, C₃-C₆ cycloalkyl, heterocyclyl having 5 to 10 ring atoms, aryl having 6 to 10 ring atoms, heteroaryl having 5 to 10 ring atoms; said alkyl, cycloalkyl, heterocyclyl, aryl and heteroaryl being optionally substituted with one or more substituents independently selected from oxo, halogen, -OH, -NO₂, -CN, and C₁-C₆ alkyl;
Or R₃ and R₄, together with the C atom to which they are bonded, form a C₃-C₁₂ cycloalkyl or a heterocyclyl having 5 to 10 ring atoms;
R' is selected from H, C₁-C₆ alkyl, C₃-C₆ cycloalkyl, heterocyclyl having 5 to 10 ring atoms, aryl having 6 to 10 ring atoms, and heteroaryl having 5 to 10 ring atoms;
R" and R'" are independently selected from H, C₁-C₆ alkyl, C₃-C₆ cycloalkyl, heterocyclyl having 5 to 10 ring atoms, aryl having 6 to 10 ring atoms, heteroaryl having 5 to 10 ring atoms; said alkyl, cycloalkyl, heterocyclyl, aryl and heteroaryl being optionally substituted with one or more substituents independently selected from oxo, halogen, -OH, -NO₂, -CN, and C₁-C₆ alkyl;
Or R" and R‴, together with the N atom to which they are linked, form a heterocycle having 5 to 10 ring atoms, or a heteroaryl having 5 to 10 ring atoms, said heterocyclyl, and heteroaryl being optionally substituted with one or more substituents independently selected from oxo, halogen, -OH, -NO₂, -CN, and C₁-C₆ alkyl;
and pharmaceutically acceptable salts, solvates and esters thereof.

2. Compound according to claim 1, wherein said compound is a modulator of ACKR3.

3. Compound according to claim 1 or 2, wherein said compound is binding to ACKR3 as measured with a displacement assay using a fluorescently labelled chemokine CXCL12.

4. Compound according to any of claims 1 to 3, wherein ring A is selected from the group consisting of 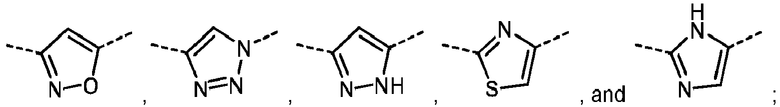 preferably ring A is

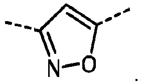

5. Compound according to any of claims 1 to 4, wherein L is a divalent linker selected from C₃-C₁₂ alkylene, C₃-C₆ cycloalkylene, C₃-C₁₂ alkenylene, and C₃-C₁₂ alkynylene.

6. Compound according to any of claims 1 to 5, wherein ring B is an aryl having 6 to 10 ring atoms, said aryl being optionally substituted with one or more substituents independently selected from halogen, -OH, C₁-C₆ alkyl, C₁-C₆ alkoxy, -(CO)-O-R', -NR"-(CO)-R', and - NR"R‴; and
R', R", and R'" are as defined in claim 1.

7. Compound according to any of claims 1 to 6, wherein the compound is a compound of formula (II)

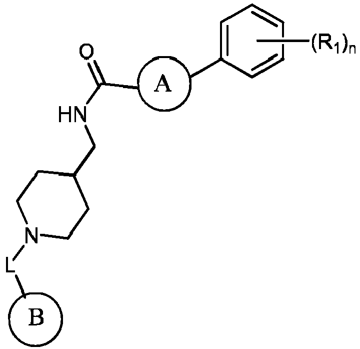

8. Compound according to any of claims 1 to 7, wherein the compound is a compound of formula (III) 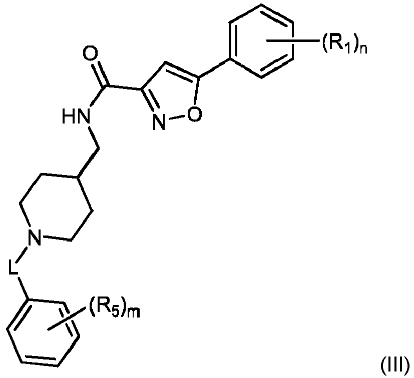 Wherein
Each R₁ is independently selected from halogen, provided that at least one R₁ is -F;
n is an integer between 1 and 5,
m is an integer from 0 to 5; and
each R5 is selected from halogen, -OH, -NO₂, -CN, C₁-C₆ alkyl, C₃-C₆ cycloalkyl, heterocyclyl having 5 to 10 ring atoms, aryl having 6 to 10 ring atoms, heteroaryl having 5 to 10 ring atoms, C₁-C₆ alkoxy, C₁-C₆ haloalkyl, C₁-C₆ haloalkoxy, -(CO)-R', -O-(CO)-R', -(CO)-O-R', -(CO)-NR"R‴, -NR"-(CO)-R', - NR"R‴, -S(O)₂-R', and S(O)₂-NR"R‴;
n, L, R', R", and R"are as defined in claim 1.

9. Compound according to any of claims 1 to 7, wherein the compound is selected from 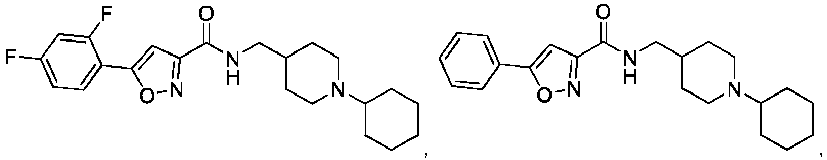 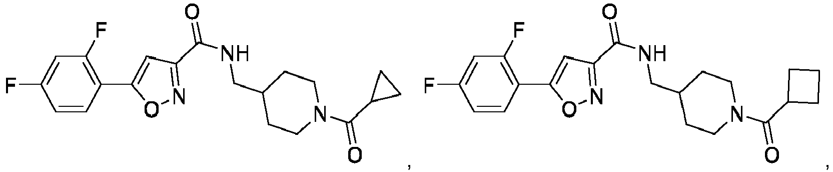 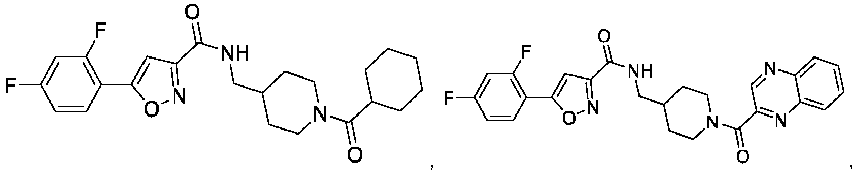 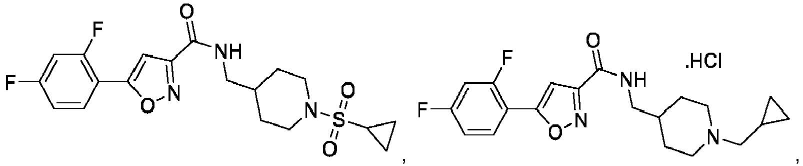 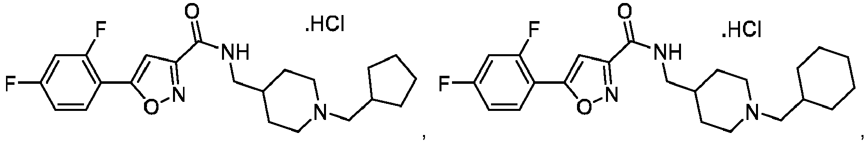 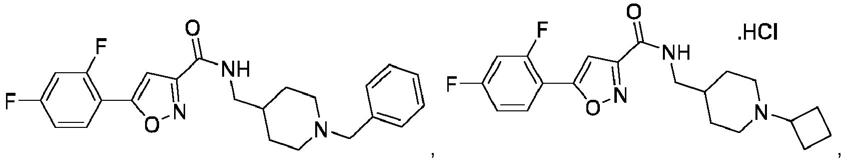 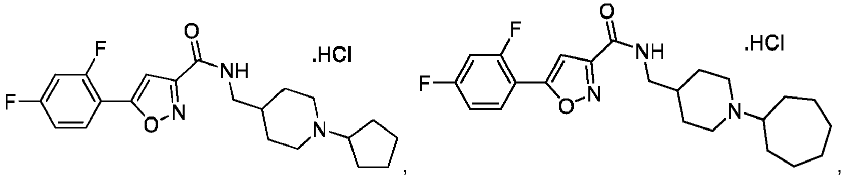 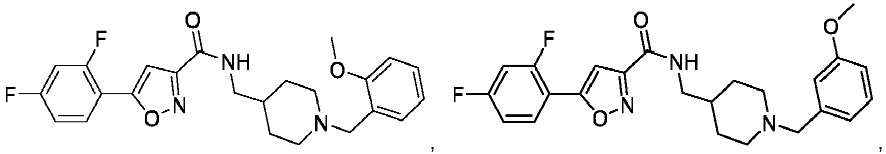 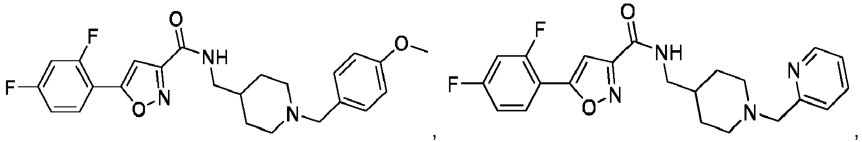 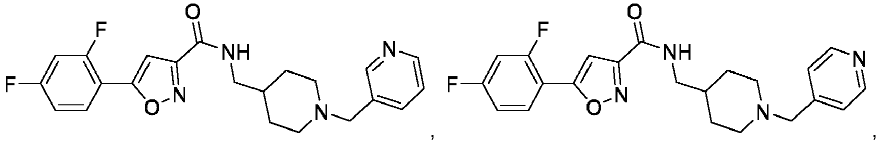 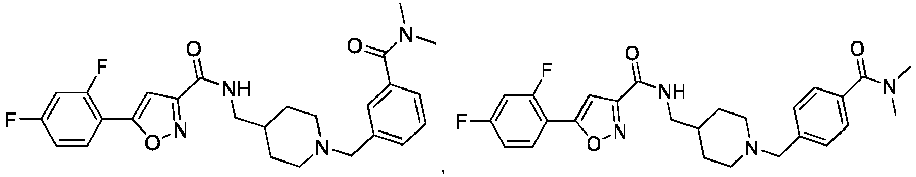 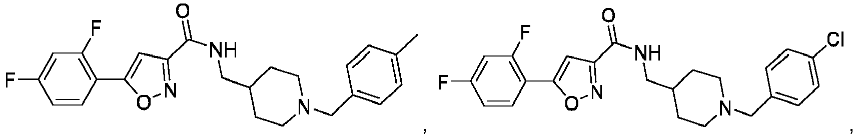 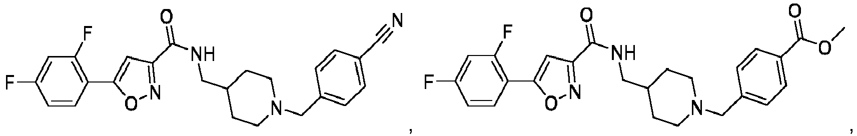 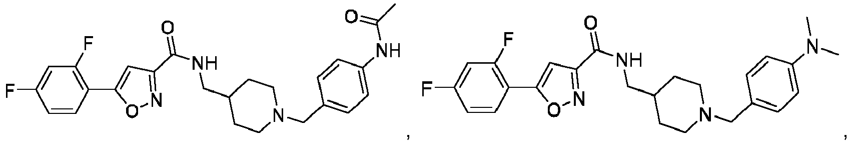 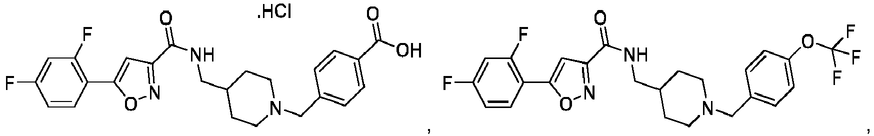 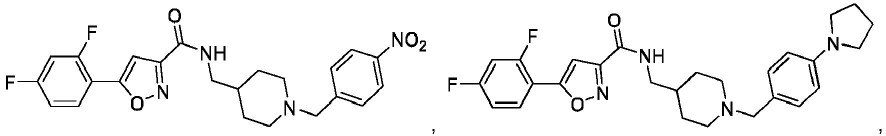 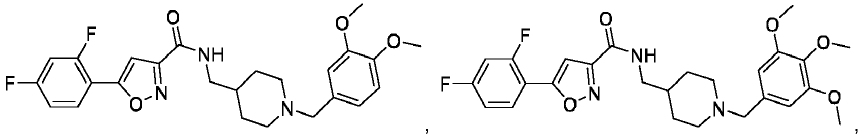 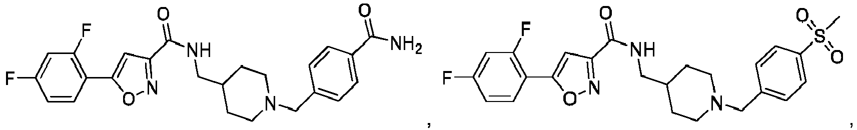 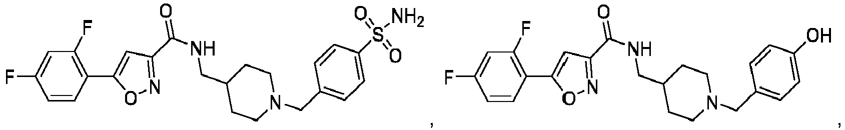 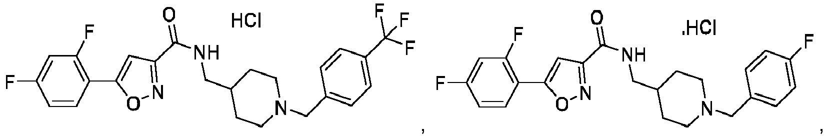 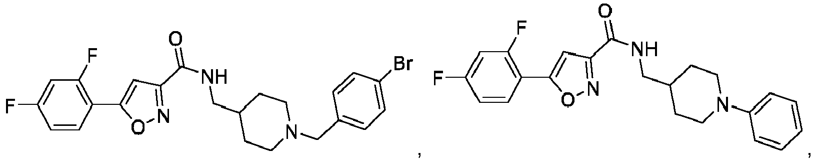 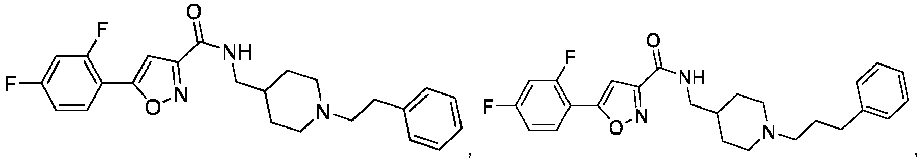 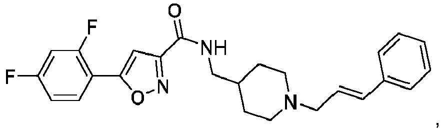 and 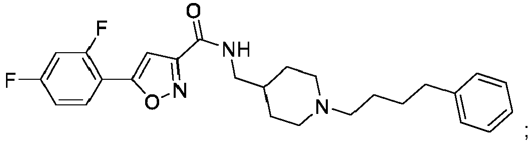 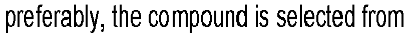 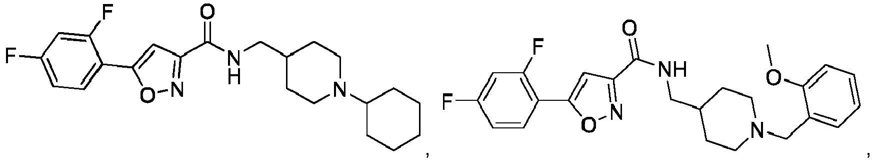 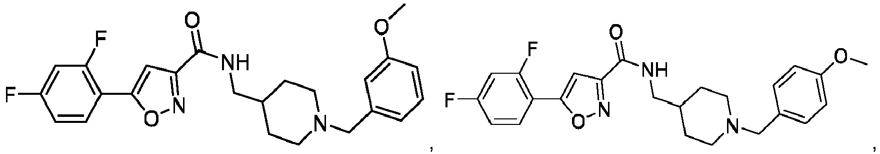 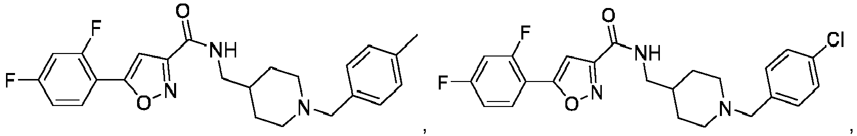 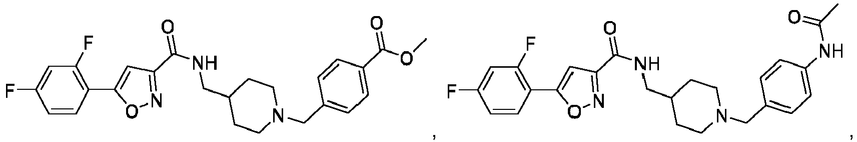 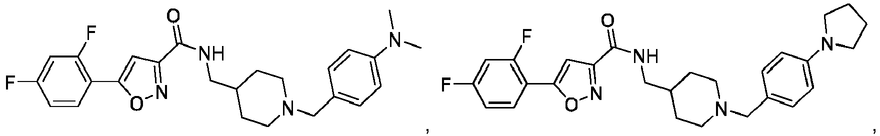 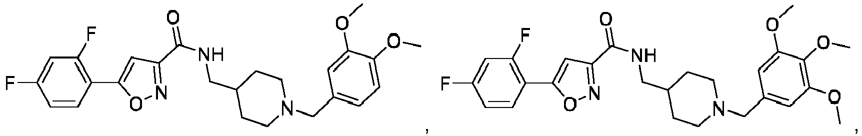 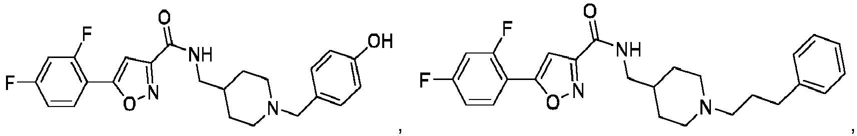 and

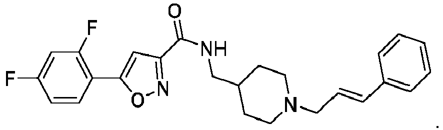

10. Pharmaceutical composition comprising a compound according to any of claims 1 to 9 and a pharmaceutically acceptable carrier.

11. Compound according to any of claims 1 to 9, for use as a drug.

12. Compound according to any of claims 1 to 9, for use in the treatment of disorders relating to the ACKR3 receptor.

13. Compound according to any of claims 1 to 9, for use in the treatment of cancer, autoimmune disorders, inflammatory diseases, transplant rejection, fibrosis or pain.
